# EUROPEAN PATENT APPLICATION

(11) **EP 3 733 869 A1**
(43) Date of publication of application: **04.11.2020**
(21) Application number: 19185338.1
(22) Date of filing: 09.07.2019
(51) Int. Cl.: C12Q 1/6806, B01D 61/18

(54) **AUTOMATED METHOD AND SYSTEM FOR SPLIT POOL BASED BARCODING OF CELLULAR MOLECULES**

(30) Priority: 02.05.2019 EP 19172427; 02.05.2019 EP 19172428
(71) Applicant: QIAGEN GmbH, 40724 Hilden (DE)
(72) Inventor: DAMMANN, Christian, 40724 Hilden (DE); HERSCHEL, Floran, 40724 Hilden (DE); NARZ, Frank, 40724 Hilden (DE)
(74) Representative: Roth, Carla

(57) **Abstract**

Provided are methods, systems and modules useful in split and pool workflow.

## Description

### BACKGROUND OF THE INVENTION

In the recent years, the biochemical analysis techniques have shifted from analyzing a large population of cells (cell pools) towards single-cells. This advancement is essential in order to gain deeper insights about the functions of specific cells and cell types and understand their complex interaction with other cells. By acquiring information of the biomolecular profiles of single cells, these can be categorized in respect to their cellular function, regional specificity, stage of differentiation, etc. This categorization allows to recapitulate multicellular systems, e.g. for diagnosis or treatment of diseases.

Transcriptomic profiling has emerged as a powerful tool of biomolecular analysis. Thereby, expression patterns can be exposed to define cellular states or to identify genes with similar expression patterns. The transcriptome comprises the full range of messenger ribonucleic acid (RNA) molecule expressed by an organism. Hence, a transcriptome represents the genetic code that is transcribed into RNA molecules. The transcriptome offers valuable information on the significant biological processes behind the maintenance of the functionality of the cell and multicellular systems (Casamassimi, A. et al. "Transcriptome Profiling in Human Diseases New Advances and Perspectives." Int. J. Mol. Sci. 18(8) (2017): 1652; Rani, B. et al. "Transcriptome profiling methods and applications- A review". Agricultural Reviews, 38(4) (2017): 271-281). Transcriptomic profiling is performed by polymerizing the RNA by reverse transcription into cDNA which is then processed to be sequenced. This process is conventionally limited to the analysis of cell population, wherein the single-cells of the population may have substantially different transcriptomes.

A recent technique to acquire the transcriptomic profile of single cells is based on providing a barcode to the single nucleic acids (like mRNAs or their cDNAs; Rosenberg, A. B., et al. "Single-cell profiling of the developing mouse brain and spinal cord with split-pool barcoding." Science 360.6385 (2018): 176-182). Thereby, on the one hand, an individual RNA-molecule can be processed and identified. On the other hand, the information is preserved from which single cell the respective RNA-molecule originates. Moreover, barcoding has allowed transcriptomic profiling of single cells in a high throughput manner. The prior art has shown that this goal is technically feasible, however by means of tedious manual workflows (which have duration of two or more days). At the center of these barcoding techniques is a so-called "split-and-pool" process, in which a large number of cells are divided among a large number of vessels ("wells") ("split" process) in order to attach part of a barcode. By subsequent reunification of the cells ("pool" process), mixing and possibly re-dividing in the split state, the above-mentioned barcoding goal can be achieved.

Splitting of cell suspensions has for instance been achieved by microfluidically entrapping cells in nanoliter droplets. For barcoding the split cells, single oligonucleotide-attached beads were co-entrapped with the single cells inside the droplets (WO 2016/145409 A1; Macosko, E. Z. et al. "Highly Parallel Genome-wide Expression Profiling of Individual Cells Using Nanoliter Droplets." Cell 161(5) (2015): 1202-1214; Klein, A. M. et al. "Droplet barcoding for single-cell transcriptomics applied to embryonic stem cells." Cell 161(5) (2015): 1187-1201). To access the mRNA the cells were then lysed (by adding lysis reagents to the droplets). The oligonucleotide attached to the bead comprised a barcode sequence and an mRNA hybridizing sequence (27 T-repeat). After hybridizing the mRNA, cDNA was transcribed to generate single-cell transcriptomes attached to the beads. These can then function as templates for PCR reactions und subsequent synthesis. The barcode allows for sorting the mRNA to the respective single cell.

In order to facilitate scalable profiling of single cells and to barcode the cellular transcriptome, Rosenberg *et al.* developed an in cell mRNA barcoding protocol called "SPLiT-seq" ("Split Pool Ligation-based Transcriptome sequencing"), herein incorporated by reference. The method enables transcriptional profiling of hundreds or thousands of cells or nuclei in a single experiment. It does not require partitioning single cells into individual external compartments such as droplets or wells, but instead uses the cells or nuclei themselves as compartments. The barcode-labeling can be performed directly in fixed and permeabilized cells or nuclei - i.e. cells are not lysed for barcoding. The cells thus represent a biological compartment, in which the RNA is entrapped avoiding release and mixing with other cell's RNA. The transcriptomes of the cells or nuclei are labelled with split-pool barcoding. In each split-pool round performed, fixed cells or nuclei are randomly distributed into wells and transcripts are labeled with well-specific barcodes. E.g. barcoded RT (reverse transcription) primers can be used in the first-round and second and third-round barcodes are appended to cDNA through ligation. As each cell has taken through the labelling process a unique path through the wells (containers), it is labelled with a unique combination of three barcodes. Thus, every RNA originating from the same biological compartment (cell, nucleus) is labelled with the same barcode combination. A fourth barcode may be added to cDNA molecules by PCR during sequencing library preparation. The obtained barcoded molecules can then be sequenced. After sequencing, each transcriptome can be assembled by combining reads containing the same barcode combination. An according *in situ* method for combinatorial labeling of cellular molecules such as RNA is also described in US 2016/0138086 and WO2019/060771, both herein incorporated by reference.

The barcoding method described in Rosenberg et al and WO2019/060771 allows to barcode thousands of cells and their RNA can be grouped based on the introduced barcode by cell of origin. However, the described methods require a manual, complex workflow and necessitate numerous different devices, such as centrifuges, thermocyclers, microscopes in order to perform the workflow. The complexity of the SPLiT-seq protocol necessitates performance by a skilled user and is in view of the multiple, time-consuming manual steps prone to handling errors. E.g., the manual workflow requires the exchange of processing solution. To achieve this, the cells are collected from one processing solution as pellet by centrifugation and resuspended in a different solution. This step requires special skills, as a large number of cells can be e.g. easily lost when washing a cell pellet, which is critical for these high-throughput techniques. Thus, only an experienced experimenter can successfully carry out the workflow. There is thus a great need for further split-and-pool-based workflows that also enable an inexperienced person to perform such workflow while avoiding handling errors. Moreover, a large number of cells are obtained from a large number of wells in this workflow. The cells have the tendency to remain in the well due to adhesion to vessel walls and fluid residues. Against this background, it is important how the cell suspension is taken up from a well with which mixing and pipetting scheme and how adhered cells can be released if necessary. Such a process typically shows variations from experimenter to experimenter which is disadvantageous for the reproducibility of the obtained results.

The present invention aims at avoiding drawbacks of the prior art. In particular, it is an object to provide a solution allowing to automate one or more, preferably all, steps of split and pool workflows which require handling of biological compartments such as cells. Furthermore, it is an object to provide implementable modules that allow or facilitate performing one or more steps of a split and pool workflow in an automated manner. These modules may be implemented into automated systems or prior art systems. It is furthermore an object to provide an automated system for split-and-pool workflows.

It is also an object to provide modules that can be implemented into an automated system. There is a need to provide modules that can be automated and allow to replace a centrifugation process, allow the singularization of cells, enable automatic cell counting and/or allow a dynamic protocol adaptation during the workflow and further steps. It is furthermore an object to provide an automated system comprising such modules that is capable of performing split-and-pool workflows.

### SUMMARY OF THE INVENTION

The present disclosure describes advantageous methods as well as individual technical modules that can be advantageously used in combination to provide an automated system allowing to perform an automated split-and-pool process.

According to a first aspect, a method for labeling target molecules within a plurality of biological compartments with a combination of barcode labels is provided, the method comprising:
(a) separating a plurality of biological compartments into at least two aliquots;
(b) contacting barcode labels comprising a barcode sequence with the at least two aliquots comprising biological compartments,
   - wherein the barcode sequence of the barcode labels contacted with a given aliquot is the same, and
   - wherein a different barcode sequence is used for different aliquots;
(c) combining aliquots comprising biological compartments into a pool; and
(d) repeating steps (a), (b) and (c) using the combined pool.

In one embodiment, a method for labeling target molecules within a plurality of biological compartments with a combination of barcode labels is provided, the method comprising:
(a) separating a plurality of biological compartments into a number (n) of aliquots, wherein n is at least 2;
(b) contacting barcode labels comprising a barcode sequence with each of the n aliquots,
   - wherein the barcode sequence of the barcode labels contacted with a given aliquot is the same, and
   - wherein a different barcode sequence is used for different aliquots, preferably for each of the n different aliquots;
(c) combining the n aliquots into a pool; and
(d) repeating steps (a), (b) and (c) using the combined pool.

It will be appreciated that several independent aspects according to the present invention such as e.g. individual modules, methods and systems that can be used in the context of the method according to the present invention and therefore are described in such context provide independent aspects and are disclosed and also claimed as such independent aspects according to the present invention.

Other objects, features, advantages and aspects of the present disclosure are described in the subsequent disclosure and will become apparent to those skilled in the art from the following description and appended claims. It should be understood, however, that the following description, appended claims, and specific examples, while indicating preferred embodiments of the application, are given by way of illustration only.

### DETAILED DESCRIPTION OF THE INVENTION

The inventions provided by the present disclosure are described in further detail in the following.

### THE METHOD ACCORDING TO THE FIRST ASPECT

According to a first aspect, a method for labeling target molecules within a plurality of biological compartments with a combination of barcode labels is provided, the method comprising:
(a) separating a plurality of biological compartments into at least two aliquots;
(b) contacting barcode labels comprising a barcode sequence with the at least two aliquots comprising biological compartments,
   - wherein the barcode sequence of the barcode labels contacted with a given aliquot is the same, and
   - wherein a different barcode sequence is used for different aliquots;
(c) combining aliquots comprising biological compartments into a pool; and
(d) repeating steps (a), (b) and (c) using the combined pool.

In one embodiment thereof, a method for labeling target molecules within a plurality of biological compartments with a combination of barcode labels is provided, the method comprising:
(a) separating a plurality of biological compartments into a number (n) of aliquots, wherein n is at least 2;
(b) contacting barcode labels comprising a barcode sequence with each of the n aliquots,
   - wherein the barcode sequence of the barcode labels contacted with a given aliquot is the same, and
   - wherein a different barcode sequence is used for different aliquots, preferably for each of the n different aliquots;
(c) combining the n aliquots into a pool; and
(d) repeating steps (a), (b) and (c) using the combined pool.

The number (n) aliquots may correspond, e.g. to the number of wells of a well-plate into which the aliquots may be transferred.

Steps (a) to (d) describe basic protocol steps of the "split" and "pool" process that can be used to label target molecules within biological compartments such as cells or cell nuclei. These steps and advantageous embodiments thereof are known in the prior art and are e.g. described in Rosenberg et al, the supplementary materials to this paper (published March 15, 2018 on Science First Release DOI: 10.1126/science.aam8999); US2016/0138086 and WO2019/060771 as well as the SPLiT-seq Protocol, Version 3.0 (publically available from https://sites.google.com/uw.edu/splitseq/protocol), herein incorporated by reference. These steps known in the art are therefore not described in detail herein. The present disclosure provides advantageous improvements of such methods which allow to automate one or more and in advantageous embodiments all steps of the method. This is described in further detail below.

Step (d) may be repeated a number of times sufficient to generate a unique combination of barcode sequences for the target molecules in a single cell. In various embodiments, step (d) (i.e., steps (a), (b), and (c)) may be repeated a number of times sufficient to generate a unique combination and thus series of barcode sequences for the target molecules, e.g. cDNAs in a first biological compartment such as a cell. Stated another way, step (d) may be repeated a number of times such that the target molecules, e.g. cDNAs in the first biological compartment (e.g. cell) may have a first unique series of barcode sequences, the cDNAs in a second biological compartment (e.g. cell) may have a second unique series of barcode sequences, the cDNAs in a third biological compartment (e.g. cell) may have a third unique series of barcode sequences, and so on.

The methods of the present disclosure may provide for the labeling of cDNA sequences from single biological compartments such as cells with unique barcodes, wherein the unique barcodes may identify or aid in identifying the biological compartment (e.g. cell or cell nucleus) from which the cDNA originated. In other words, a portion, a majority, or substantially all of the cDNA from a single biological compartment may have the same barcode, and that barcode may not be repeated in cDNA originating from one or more other biological compartments in a sample (e.g., from a second cell, a third cell, a fourth cell, etc.).

In step (a) an aliquot or group of biological compartments such as cells can be separated into different containments, e.g. containers such as reaction vessels, in particular wells of a well plate. After contacting the aliquots with the barcode labels, aliquots may then be pooled, mixed, and separated again and further barcode labels can be added, whereby another barcode label is coupled to the barcode label that was coupled to the target molecule in the previous round of barcoding. Details are described in the prior art cited above. In various embodiments, the same barcode label may be added to more than one aliquot of biological compartments in a single or given round of labeling. However, after repeated rounds of splitting, tagging and repooling, the target molecules (e.g. cDNAs) of each biological compartment may be bound to a unique combination or series of introduced barcode sequences that form a combined barcode. In some embodiments, biological compartments, such as cells in a single sample, may be separated into a number of different reaction vessels. For example, the number of reaction vessels may include four 1.5 ml microcentrifuge tubes, a plurality of wells of a 96-well plate, or another suitable number and type of reaction vessels or well plates.

As discussed herein, the plurality of biological compartments can be repooled and the method can be repeated any number of times, adding more barcode labels to the target molecules (e.g. cDNAs) creating a set of barcode labels that can act as a combined barcode. As more and more rounds are added, the number of paths that a biological compartment can take increases and consequently the number of possible combined barcodes that can be created also increases. Given enough rounds and divisions, the number of possible combined barcodes will be much higher than the number of biological compartments, resulting in each biological compartment likely having a unique combined barcode that is created from the serially attached barcode labels. For example, if the division of the plurality of biological compartments in aliquots took place in a 96-well plate, after 4 divisions there would be 96⁴=84,934,656 possible barcodes.

### Reverse transcription

In one embodiment, the method is used for barcoding nucleic acids within a biological compartment. The method may comprise generating cDNAs within the biological compartments by reverse transcribing RNAs into cDNA, preferably using a reverse transcription primer comprising a 5' overhang sequence.

In one embodiment, reverse transcription is performed prior to step (a). Reverse transcription may be performed using a reverse transcription primer. Embodiments are described in the prior art, see e.g. US2016/0138086 and WO2019/060771 and the other publically available prior art documents referred to above and may be used in conjunction with the present invention.

Reverse transcription can be performed using the plurality of the biological compartments (e.g. cells or cell nuclei) in form of a pool. In one embodiment, a 5' overhang of the reverse transcription primer provides an adapter sequence for the barcode label that is used in step (a).

The reverse transcription step may furthermore be used to introduce a first barcode label during reverse transcription. In this case, the method may comprise (i) separating a plurality of biological compartments into at least two aliquots; and (ii) contacting each of the aliquots with a reverse transcription primer comprising a barcode sequence,
∘ wherein the barcode sequence of the reverse transcription primer is the same for a given aliquot, and
∘ wherein a different barcode sequence is used for different aliquots.

After reverse transcription, the biological compartments of the aliquots comprising cDNAs carrying the first barcode label are combined thereby providing a plurality of compartments that can be subjected to step (a) of the method for attaching a further barcode label. Such methods are described in the prior art referenced above and may be used in the context of the present invention. As described herein, the biological compartments are preferably mixed during and/or after the pooling process. For this purpose, the mixing method described herein may be used.

### Ligation

The method preferably comprises performing a ligation reaction. Such reaction may be performed for ligating at least two of the barcode labels that are bound to the target molecules, wherein preferably the ligation is performed within the plurality of cells. Ligation ensures e.g. that barcode labels are firmly connected to provide the combined barcode.

A ligation reaction may be performed after attaching all barcode labels providing the combined barcode, i.e. a combined ligation can be performed after adding the desired barcodes (see e.g. WO2019/060771).

In embodiments, a ligation reaction is performed during or following each round of barcode attachment in step (c). This ensures a secure attachment of each barcode label that is added during each round of barcode attachment to the target molecule.

The ligation reaction can be advantageously performed inside the plurality of biological compartments.

For ligation, a composition (e.g. a ligation-ready-mix) may be added to the plurality of biological compartments. In embodiments also described in the prior art, the components required for ligation are mixed with the plurality of biological compartments prior to splitting the plurality of biological compartments into at least two aliquots in step (a). Each aliquot is then transferred to a containment (e.g. well of a well plate). The containment may already comprise the barcode labels or the barcode labels may be added after transferring the aliquots into the containments. Alternatively, the ligation reagents may be subsequently added to the aliquots after transfer into the containments, e.g. the wells of a well-plate or other device providing reservoirs, preferably arranged in an array format.

The ligation mix may comprise a ligase buffer (e.g. NEB) and a ligase enzyme (e.g. T4 DNA ligase). Further compounds may be one or more of the following: one or more RNase inhibitors and one or more adhesion reducing compounds (e.g. Pluronic F127, Triton-X100, as is described in further detail below).

In a preferred embodiment, the plurality of biological compartments is contacted with one or more ligase compounds (e.g. a ligase or a ligase reaction master mix) after preforming a reverse transcription and prior to splitting the plurality of biological compartments into portions/aliquots in step (a). To ensure removal of the reaction medium used for reverse transcription and in particular the reverse transcription primers, said reaction medium containing the plurality of biological compartments may be removed using the advantageous filter module of the present invention. The biological compartments can thereby be contacted e.g. with a solution comprising one or more ligase reagents (e.g. a ligase or a ligase reaction mix), forming again a suspension. This filter module and advantageous embodiments thereof that allow to separate biological compartments from their surrounding solutions and enables a solution exchange in an automated manner is described elsewhere herein and it is referred to the present disclosure. Such reaction medium exchange step using the filter module of the present invention may e.g. occur, between a reverse transcription step and a ligation step.

For ligation e.g. during step (c), the containment (e.g. well) comprising the aliquot of biological compartments may be heated. According to an advantageous embodiment, the containment may be located on a heating module, such as an automatically lockable heating module or cycling module described elsewhere herein. Thereby, the one or more containments comprising the ligation reaction can be heated to a desired temperature, while the flexible mat allows to prevent significant evaporation of the liquids. A ligation reaction may be performed by the heating module such as the automatically lockable heating module or cycling module, allowing for heating to elevated temperatures, suitable for ligation.

According to one embodiment, the ligation reaction ligates and thus connects barcode labels. Details are described in the prior art referred to herein and it is referred to this disclosure which also applies here.

According to one embodiment, blocking agents may be applied after ligation. Blocking agents may be selected from blocking oligonucleotides. Blocking ensures that unbound DNA barcodes cannot mislabel the reaction product in future barcoding rounds. The use of such blocking agents is described in the prior art and may be used in the context of the present invention.

### Use of a filter module

The method may furthermore comprise using a filter module for separating biological compartments from a liquid and/or for concentrating biological compartments within a liquid.

During the workflow of the method, it may be preferred or even necessary to exchange one or more liquids surrounding the plurality of biological compartments. The exchange of liquid may facilitate the performance of subsequent method steps.

In particular, according to a preferred embodiment, a reverse transcription is carried out as describe herein to provide cDNA from the RNA within the biological compartments (such as cells). The compounds present after performing the reverse transcription reaction are preferably removed. Moreover, according to a preferred embodiment, one or more ligation reactions are performed in sequential steps during barcode attachment, necessitating an exchange of the liquid surrounding the plurality of biological compartments. According to the prior art, this liquid exchanging step is performed with a centrifuge (e.g., by centrifuging the biological compartments, removing the liquid and resuspending the biological compartments in a fresh buffer; see Rosenberg *et al*.). In this context, the use of a centrifuge has at least two disadvantages: a centrifuge would be a larger maintenance-relevant component in scope of an automated system. Furthermore, it would be difficult to wash the cell pellet loss-free or substantially loss-free, as loss is very likely after each centrifugation. The present disclosure allows to avoid the drawbacks of the prior art by exchanging the liquid surrounding the plurality of biological compartments using a filter module. The filter module advantageously allows for exchanging a liquid surrounding the plurality of biological compartments in an automated manner - without the need for a centrifuge. The filter module according to the present disclosure may be advantageously controlled automatically.

The method may thus further comprise separating biological compartments from a reaction medium using a filter module. Separation of the biological compartments using the filter module may fulfill one of more of the following characteristics:
- it is performed in an automated manner; and/or
- it is performed after performing a reverse transcription reaction and/or after attachment of a barcode label.

The liquid surrounding the plurality of biological compartments may thus be automatically exchanged in the present method by using a filter module.

The filter module allows to perform the required washing of the plurality of biological compartments in the present workflow in an automated manner. Moreover, the filter module allows to provide the plurality of biological compartments in a liquid suitable for performing subsequent reactions (e.g. a ligation reaction).

According to one embodiment, the plurality of biological compartments and the liquid is transferred into a portion of a filter module, e.g., the portion is a feed portion of the filter module. The portion preferably comprises a surface filter element, preferably a membrane, separating the portion from a further portion, e.g., the further portion being an effluent portion. The filter element can be provided at the bottom section of the feed portion. The filter element is preferably a surface filter, such that the biological compartments can be collected at the surface of the filter element, without the biological compartments entering the filter element. The portion, the filter element (preferably a membrane) and the further portion may be referred to as a "well". The filter element, preferably a membrane or filter, is configured to retain substantially the plurality of biological compartments. Therefore, when generating a pressure differential across the filter element, liquid surrounding the plurality of biological compartments moves through the filter element, while the biological compartments do not move through the filter element. Preferably, a portion of the liquid, preferably a small portion, does not pass the filter element but is retained as residue that comprises the biological compartments. Afterwards, another solution may be added into which the plurality of biological compartments can be resuspended.

The removal of liquid surrounding the plurality of biological compartments and the resuspension may be repeated. Repetition may be desired in order to remove remainders of the liquid (respectively the compounds provided therein) the plurality of biological compartments was suspended in before. For instance, it may be desirable to remove the remains of the compounds present during the reverse transcription (e.g. primer, reverse transcriptase, particular buffer compounds, etc.). Therefore, multiple separation and resuspension steps may be performed.

According to the present disclosure, also a filter module is provided for exchanging a liquid surrounding biological compartments (here referred to as a "filter module") that can be used in conjunction with the method according to the first aspect. Details of this filter module and methods/uses that can be performed using this filter module are described below. As noted above, these aspects also form independent aspects of the present invention.

Also disclosed in the context of the present disclosure is thus a filter module for exchanging a liquid surrounding a plurality of objects, preferably biological compartments, or for separating a liquid from a plurality of objects, preferably biological compartments, the filter module comprising:
- a feed portion (top part), an effluent portion (bottom part) and a filter element, preferably a membrane, wherein the filter element is provided between the feed portion and the effluent portion;
- wherein the feed portion or the effluent portion is configured to be connected to a device capable of generating a pressure differential; and
- wherein the feed portion is configured such that liquid and/or liquid comprising a plurality of biological compartments can be fed into the feed portion.

Also disclosed in the context of the present disclosure is a method for removing and preferably exchanging a liquid surrounding a plurality of objects, preferably biological compartments, or for separating a liquid from a plurality of objects, preferably biological compartments, the method comprising the steps:
- feeding the liquid comprising the plurality of objects, preferably biological compartments, into a feed portion, which is separated from an effluent portion by a filter element, preferably a membrane,
- wherein a pressure differential is generated by applying an overpressure to the feed portion and/or applying an negative pressure to the effluent portion.

The present disclosure is also directed to the use of a filter element, preferably a membrane, for exchanging a liquid surrounding a plurality of objects, preferably biological compartments, or for separating a liquid from a plurality of objects, preferably biological compartments, wherein a filter element, preferably a membrane, is used to filter the objects, wherein a device is used which applies a pressure differential acting across the a filter element, which preferably is a membrane.

Also provided is a method of exchanging a liquid surrounding a plurality of biological compartments using a filter module, the method comprising the steps of:
(a) optionally, separating at least a part of the liquid surrounding the plurality of biological compartments by generating a pressure differential;
(b) contacting and mixing the plurality of biological compartments with another liquid;
(c) optionally, repeating steps (a) and (b).

Further features of the filter module and the corresponding aspects disclosed above are summarized in the following. It may have one or more of the following characteristics, respectively one or more of the following characteristics may be fulfilled:
According to one embodiment, the feed portion of the filter module has an elongated body portion. The feed portion may have a longitudinal axis that intersects the filter element (preferably a membrane). An opening of the feed portion may be spaced apart from the filter element (which preferably is a membrane). This opening is referred to in the figures as second opening.

According to one embodiment, the effluent portion of the filter module has an elongated body portion. The effluent portion may have a longitudinal axis that intersects the filter element (preferably a membrane). An opening of the effluent portion may be spaced apart from the filter element (preferably a membrane). This opening is referred to in the figures as first opening.

According to one embodiment, the feed portion and the effluent portion meeting in the filter element (preferably a membrane) confine an angle equal to substantially 90°, such as an angle selected between 85° to 95°, such as 86°, 87°, 88°, 89°, 90°, 91°, 92°, 93°, 94°, or usually 90°. According to one embodiment, the feed portion and the effluent portion meeting in the filter element (preferably a membrane) confine an angle different than 90°. The surface of the filter element, preferably a membrane, may at least partially confining an angle with the longitudinal axis of the feed portion which is smaller than 90°. The effluent portion may comprise a flow path comprising a redirection of fluid by 90°.

In one embodiment, the feed portion is a component being connectable or being connected to the effluent portion securing the filter element (preferably a membrane) between feed portion and effluent portion. In one embodiment, the feed portion (top part) and the effluent portion (bottom part) are configured to be connected by a frictional fit, form fit and/or adhesive bonding, e.g. screwing, clamping or snap-fit assembly.

The filter element, preferably a membrane, is secured between the feed portion and the effluent portion by using at least one sealing means or at least one filter element holder between the filter element and the feed portion and/or the filter element and the effluent portion. Hence, the filter module may comprise an elongated body comprising a feed portion/top part and an effluent portion/bottom part, configured to be connected and secure a filter element, preferably a membrane, between feed portion/top part and effluent portion/bottom part. Securing may include one or more sealing means/filter element holders.

A first sealing means/filter element holder may be provided between the filter element and the feed portion, e.g. the first sealing means/filter element holder is in contact with a first surface of the filter element, the contact being preferably a closed shape, e.g. circular, elliptical or polygonal shape, and a second sealing means/filter element holder is in contact with the second surface of the filter element, preferably a membrane, being the surface opposite the first surface, the contact being preferably a closed shape, e.g. circular, elliptical or polygonal shape. The sealing means/filter element holder may comprise a structural element, the structural element being adapted to interact with the filter element, especially for holding or positioning the filter element with regard to the sealing means/filter element holder. Especially the structural element can be a reception for the filter element, which preferably is a membrane, such that the sealing means/filter element holder at least partially surrounds the filter element. According to one embodiment, the feed portion and one of the sealing means/filter element holders each comprise a structural element, the structural elements being adapted to interact with each other, especially for holding or positioning the sealing means/filter element holder by or to the feed portion. The effluent portion and one of the sealing means/filter element holders may each comprise a structural element, the structural elements being adapted to interact with each other, especially for holding or positioning the sealing means/filter element holder by or to the effluent portion.

According to one embodiment, the effluent portion/bottom part is configured to be connected to a tubing, wherein preferably the first opening of the effluent portion/bottom part is configured to be connected to the device capable of generating a pressure differential. In one embodiment, the effluent portion/bottom part is connected via a tubing to the device, preferably the opening of the effluent portion/bottom part, is connected via a tubing to the device.

According to one embodiment, the filter element, which preferably is a membrane, has a mean pore size, which is smaller than the size of the biological compartments to be filtered, wherein the filter element has one or more of the following characteristics:
- it has a mean pore size ranging from 0.01 µm to 30 µm, preferably less than 15 µm, less than 10 µm, less than 8 µm, more preferably less than 3 µm or approximately 0.1 µm;
- it is provided by a membrane, wherein the membrane comprises or consists of a film or foil having pores or holes of a defined mean pore size;
- wherein the membrane is a track etched membrane, preferably having a pore size of less than 8 µm, less than 3 µm, more preferably approximately 0.1 µm or less.

The filter module described herein allows to achieve good biological compartment (e.g. cells) recovery rates, while being capable to be operated in an automated manner. The cell recovery rate is in embodiments greater than 30%, preferably greater than 50%, more preferably greater than 70%.

According to one embodiment, the filter module is controlled in an automated manner, wherein in particular the pressure differential is controlled in an automated manner. A control unit may be provided which controls the application of the pressure differential. The control unit is in embodiments adapted to apply a pressure differential, especially the control unit being in functional connection with the device capable of generating a pressure differential. The control unit can be part of the device capable of generating the pressure differential. The control unit may be a unit which is functionally connected to or part of a control unit for controlling feed of the liquid and/or biological compartments, which may be comprised in a liquid. According to one embodiment, the control unit and/or the device capable of generating the pressure differential is in functional connection with a pressure sensor. The device capable of generating a pressure differential may be adapted to apply a differential pressure by providing or applying a determined volume of gas/fluid or sucking a determined volume of gas/fluid, preferably a fluid. The device is controlled to achieve that a defined volume of liquid is sucked through the filter module. The device capable of generating a pressure differential is preferably a syringe pump.

In one embodiment, the control unit is adapted to allow a liquid, e.g. a supernatant, in the feed portion, e.g. after filtering. The liquid remaining above the filter element may comprise the biological compartments (e.g. cells) in form of a concentrated suspension. Controlling the pressure device in such manner is advantageous, in particular when processing a liquid that comprises biological compartments. It is gentle to the biological compartments while allowing to remove the majority of the original fed liquid. Thereby, the biological compartments (e.g. cells) are effectively concentrated. Moreover, the concentrated biological compartments can be easily collected in form of a concentrated suspension.

In a preferred embodiment, the filter module is capable of being implemented into an automated robotic platform. Also provided is an automated platform comprising a filter module as described herein.

Performing the filtering function may be controlled by an algorithm, computer program or sequence of operation instructions in any suitable form, e.g. any suitable programming language.

Further suitable and preferred embodiments are described in the following:
According to one embodiment, a filter module is provided that comprises openings in a feed portion (may be referred to as "top part") and an effluent portion (may be referred to as "bottom part"). Especially the feed portion is at the top and the effluent portion at the bottom of the filter module. Furthermore, a filter element is provided which preferably is a membrane. The filter element may be permeable. Subsequently, the filter element is predominantly referred to with respect to the preferred embodiment wherein the filter element is a membrane. However, also other filter elements may be used. For example, a woven cell strainer mesh with fine mesh width may also be used as filter element.

For instance, by means of an external screw thread on the top part of the filter module, a membrane (filter element) can be clamped in the bottom part of the filter module. The filter module is only permeable through the pores of the filter element (preferably a membrane). This can be ensured by means of one or more sealing means/filter element holders (e.g., sealing/membrane holders). Below the membrane (filter element) is the effluent portion/second part of the filter module which is configured to be connected to a device capable of generating a pressure differential. For instance, the bottom part may be connected to a syringe pump. This arrangement allows fluid to be removed from or added to a portion of the filter module (e.g. containment above the membrane, respectively filter element) by means of a syringe pump (or other means of moving a fluid, e.g. via compressed air, the use of syringe pump being preferred though).

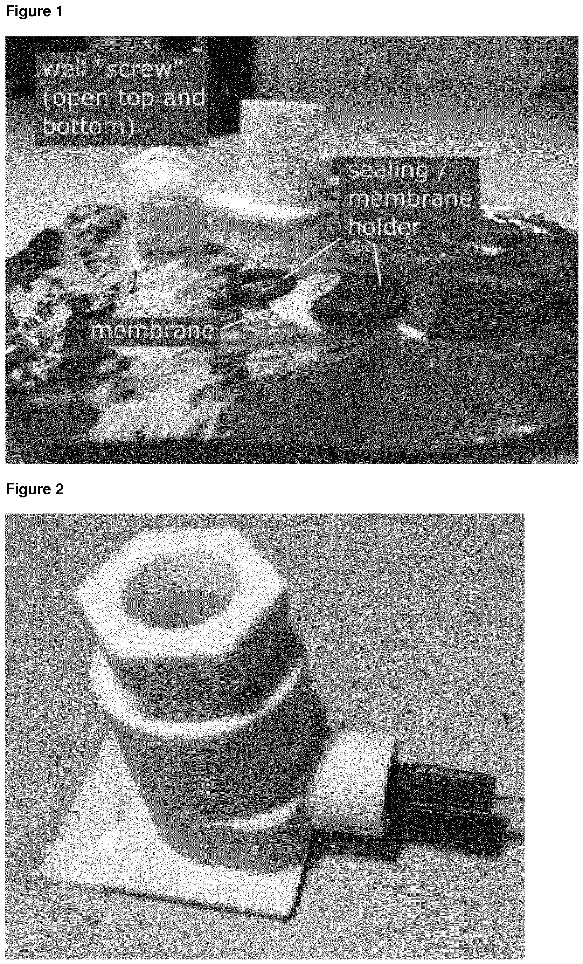

An exemplary setup for this technical solution is described in **Fig. 1****.** In Fig. 1, a photograph of exemplary filter module components is depicted. Therein, the two sealing means /membrane holders are shown which can be used to hold a membrane (preferred filter element) in between. For instance, the one sealing means/membrane holder may be provided at the effluent portion/bottom part, whereupon the membrane (filter element) is put. Afterwards, the second sealing means/membrane holder may be put on top. Therefore, advantageously the membrane (filter element) is stably held by the two sealing means/filter element holders. At the same time, the sealing means/membrane holders are configured to let fluid get into contact with the filter element, preferably a membrane, by providing one or more central openings. The held membrane may be added to the photographed effluent portion/bottom part, which contains an inner contact surface. One sealing means/membrane holder may be inserted into the bottom part and is arrested on the contact surface. The photographed top part may be assembled with the bottom part in order to clamp the filter element (preferably a membrane), respectively the sealing means/membrane holders which hold the membrane inbetween. For instance, the top part may be screwed together with the bottom part. Thereby, a well "screw" is provided.

In **Fig. 2****,** a photograph of an exemplary assembled filter module is depicted. An assembled filter module is shown, which comprises a feed portion/top part and an effluent portion/bottom part in the connected state. Accordingly, the connection may be achieved by screwing the effluent portion/bottom part and feed portion/top part together. The bottom part is configured to be connected to a device capable of generating a pressure differential. Therefore, the bottom part comprises a first opening, which may be configured to be connected to a device capable of generating a pressure differential. The connection may be established by plugging a tube into or onto the first opening. According to one embodiment, the first opening comprises an inner threaded surface. For instance, the tube may comprise components at the end having at least partially a threaded surface. The threaded surface of the tube and the effluent portion/bottom part can then form a screw fit connection. Other modes of connection and the required components may be readily available to the skilled person and can be applied in scope of the present disclosure.

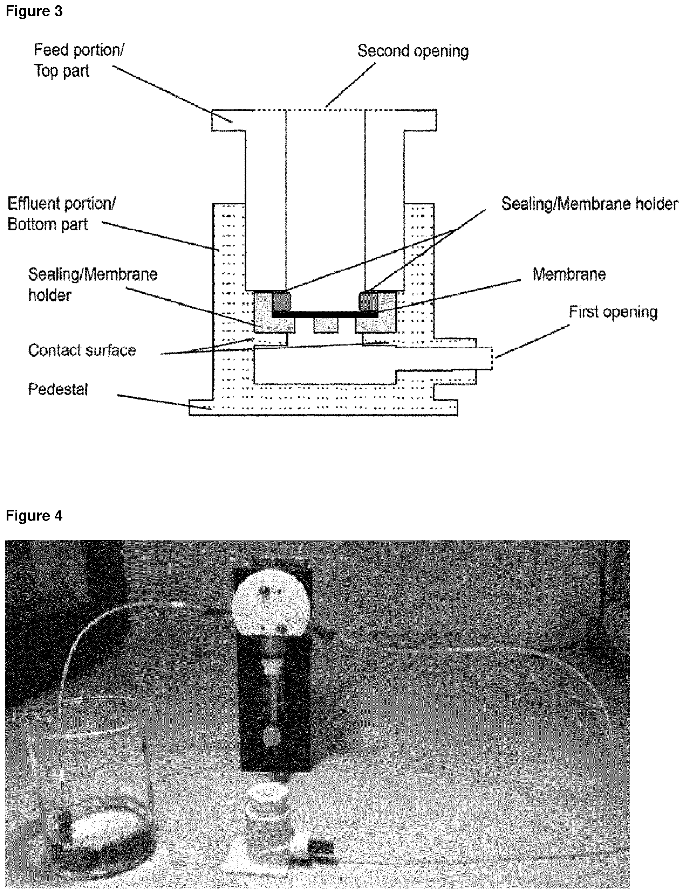

In **Fig. 3** an illustration of the cross-section of the filter module is depicted. The illustration shows a preferred embodiment of the filter module in the assembled state. Accordingly, the sealing means/filter element holders (e.g., sealing/membrane holders) hold the membrane (preferred filter element) and are placed on top of a contact surface. In the shown embodiment, two sealing means/filter element holders are provided wherein the filter element is assembled in-between. In the shown embodiment, the first sealing means/filter element holder that contacts the contact surface of the bottom part is larger than the filter element and the second sealing means/membrane holder and receives both the filter element and the second sealing means/filter element holder in a provided reception. The contact surface may be provided by the bottom part, e.g. in form of a protrusion. The protrusion may extend into an inner cavity of the bottom part, e.g. in form of a ring structure. Thereby, a contact surface is provided that is suitable for securely holding the sealing means/filter element holders and the filter element that is located in-between. The feed portion/top part may then clamp the sealing means/filter element holders (e.g., suitable sealing means/membrane holders) from the other side in the assembled state. The region above the membrane (filter element) advantageously forms a sealed well, in which the material can be kept unless the material is permeable through the filter element, which preferably is a membrane. E.g., when applying a pressure differential, liquid surrounding the plurality of biological compartments may pass the membrane. Thereby, the liquid is removed through the filter element while retaining the biological compartments in the "well" that is provided above the filter element. The sealed well may be filled via the second opening with a plurality of biological compartments. Moreover, the first opening provided in the bottom part may be connected to a device for generating a pressure differential. In case a pressure differential is applied, the liquid surrounding the plurality of biological compartments may be removed, i.e. passes the filter element, preferably membrane. Moreover, the liquid surrounding the plurality of biological compartments may then pass the first opening. It is within the scope of the present disclosure to exchange only a part of the liquid surrounding the plurality of biological compartments, if desired (e.g., to concentrate the plurality of biological compartments). As disclosed herein, a small volume of the liquid may remain to provide a concentrated suspension of the biological compartments.

In **Fig. 4****,** a photograph of an exemplary assembled filter module is depicted connected to a syringe pump, a device which is preferably used for creating the pressure differential in a controlled manner. This embodiment is well-suitable for the intended automation within the method according to the invention. Therefore, the effluent portion/bottom part forms a connection to a syringe pump, wherein preferably the connection is a tube. The particular type of syringe pump may not be limiting according to the present disclosure.

### Body of filter module

According to an embodiment, the filter module comprises an elongated, hollow body comprising a first and a second opening and a filter element, preferably a membrane, wherein the filter element, preferably the membrane, is provided between the two openings. According to one embodiment, the hollow, elongated body can have various forms or geometries. The hollow, elongated body may have a size ranging from a millimeter up to dozens of centimeters, e.g. in a range of 0.5cm to 40cm, such as 1cm to 35cm, 1.5cm to 30cm and 2cm to 25cm. According to a particular embodiment, the hollow, elongated body may have a size of multiple centimeters (e.g., 1, 2, 3, 4, 5, 6, 7, 8, 9, 10, 11, 12, 13, 14, 15, 16, 17, 18, 19, or 20 cm). According to one embodiment, the hollow, elongated body has a cylindrical shape. The inner dimensions of the hollow, elongated body may not be limited, as long as a liquid can generally pass the hollow elongated body. The liquid passes the filter element that is provided in the elongated, hollow body of the filter module.

According to one embodiment, the hollow elongated body comprises a filter element, preferably a membrane, wherein the filter element preferably covers a cross-section of the hollow elongated body in order to separate the hollow, elongated body into two parts. The separation of the hollow, elongated body is characterized by the properties of the filter element, which preferably is a surface filter, more preferably a membrane. For instance, if a preferred permeable membrane is applied, the separation comprises also permeation.

Embodiments of the hollow elongated body of the filter module were already described above. The body may be provided by one piece or preferably by two or more components, such as a bottom part and a top part that can be connected to each other (e.g. screwed) as described above. In one embodiment, the filter element is assembled so that it can be exchanged. This simplifies the assembly of different filter elements (e.g. depending on the biological compartments or interest) as well as the exchange of expanded filter elements.

### Filter element, preferably a membrane

The filter element applied in the filter module is capable of at least partially separating the plurality of biological compartments form the liquid surrounding the biological compartments. Therefore, the filter element may be selected according to the filtration characteristics, e.g. the suitability of separate the biological compartments from the surrounding liquid. Different types and materials are applicable in scope of the present disclosure. Characteristics of the filter element were already described above.

The filter element is preferably a membrane. According to a particularly preferred embodiment, the membrane has a mean pore size which is small enough to efficiently retain the biological compartments. According to one embodiment, the membrane has a pore size, which is smaller than the size of the biological compartments (e.g. smaller than a cell or cell nucleus). Moreover, it is also preferred to provide a membrane comprising pores which are large enough so that the fluid resistance is not too high. Thereby, the liquid surrounding the plurality of biological compartments can be effectively removed.

According to a preferred embodiment, the membrane allows for a highly efficient retention of cells. Moreover, the membrane advantageously allows to recover the cells at high recovery rates. For instance, the cell recovery rate may be at least 30% at least 50%, or at least 70%. This can be adjusted based on the choice of the filtering element by the skilled artisan.

### Membrane pore size

According to a preferred embodiment, the mean pore size ranges from 0.01 µm to 30 µm, preferably less than 15 µm, less than 10 µm, less than 8 µm, more preferably less than 3 µm or approximately 0.1 µm or less. According to one embodiment, the pore size is selected from the group comprising of 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 4, 5, 6, 7, 8, 9, 10, 11, 12, 13, 14 and 15 µm. Preferably the pore size is selected from the range of 0.01 to 8 µm, e.g. 0.01 to 5µm, preferably from 0.01 to 3 µm, more preferably from 0.05 to 3 µm. According to a particular embodiment, the membrane comprises a film, wherein the film has a defined mean pore size. For instance, the mean pore size distribution may have a particularly low standard deviation. For instance, a standard deviation of less than 50%, less than 40%, less than 30%, less than 20%, or even less than 10% may be applied. According to a particular embodiment, the membrane is a track etched membrane, preferably having a pore size of less than 8 µm, 5 µm or less, less than 3 µm, 2 µm or less, preferably 1 µm or less. In embodiments, the pore size is 0.5 µm or less, more preferably approximately 0.1 µm or less.

According to one embodiment, the membrane comprises a smooth surface. Therefore, the membrane may have been originally a film, which is then processed to a track etched membrane. The smooth surface of the film in comparison to other filter types can be advantageous in order to achieve high retention rates of the biological compartments. Other filter structures may also be applied in scope of the present disclosure, including polyethersulfone (PES) membranes (e.g. provided as Supor® PES). It is preferred according to the present disclosure, that the membranes are not selected from conventional depth filters. Depth filters may disadvantageously trap cells inside the membrane and thereby reduce the efficiency to retain cells.

### Track etched membrane

According to a particular embodiment, the membrane of the present disclosure is a track etched membrane. According membranes are described e.g. in US 6,103,119 A, herein incorporated by reference. Accordingly a track etched membrane may be formed from a plastic film. The plastic may be a polymeric material, i.e., a polymerization product incorporating repeating chemical units. Polymeric materials include but are not limited to polyesters, polystyrenes, aromatic polyesters, polycarbonates, polyolefins, including polyethylene, polyethylene terephthalate, polypropylene, vinyl plastics such as polyvinyl difluoride (PVDF), and cellulose esters such as cellulose nitrate, cellulose butyrate and cellulose acetate. In one embodiment of the disclosure, the track etched membrane is formed from polyester. However, although an illustrative list of materials has been disclosed herein, it should be understood that the present disclosure is not limited to a membrane formed from any specific material, and that any material capable of being track etched can be used to form the track etched membrane used in the filter module of the present disclosure. The filter module may be a cartridge.

### Device capable of generating a pressure differential

The filter module according to the present disclosure is configured to be connected to a device capable of generating a pressure differential. This has been explained above. Therefore, preferably the first opening of the hollow, elongated body is connected to the device. The connection may be established by any suitable means, preferably via a tube. Therefore, the first opening is configured to be connected to the device via a connection such as a tube, e.g. by providing a cylindrical geometry which can be connected with or to the tube as an example of suitable connection means. Preferably, the connection should results in a sealed connection, wherein leakage is reduced or prevented. Further means to prevent leakage (e.g. hose clamp, etc.) may additionally be applied in scope of the present disclosure. According to a particular embodiment, the first opening may be threaded, preferably at the inner surface. Moreover, a connecting element may be provided that comprises a threaded surface, preferably an outer threaded surface. Therefore, the connecting element and the first opening may form a screw fit connection. Other modes of connection and the required components may be readily available to the skilled person and can be applied in scope of the present disclosure.

According to one embodiment, the filter module comprises additionally a device capable of generating a pressure differential. Such device may be connected to the filter module. Any device capable of generating a pressure differential may be applied in scope of the present disclosure. In particular devices are preferred, which can be controlled in an automated manner, e.g. by providing an interface to a processing unit. It is preferred to control the pressure differential in an automated manner. Thereby, the filter module can be controlled in an automated manner. This allows to control the filter operation and performance of the filter module.

The filter module according to the present disclosure advantageously comprises a first opening, which is connected to the device. The device can then advantageously create a low pressure atmosphere inside a portion of the hollow, elongated body (e.g., below the filter element, which preferably is a membrane), in order to generate a pressure differential. Thereby, material (e.g. a liquid) above the filter element, preferably the membrane, may be induced or supported to pass the filter element. The liquid may be sucked through the filter element. For instance, the generation of a low pressure may induce liquid to permeate through the membrane. In view of a plurality of biological compartments surrounded by a liquid, the liquid may be induced to pass the permeable membrane (filter element), leading to separation of the liquid and the plurality of biological compartments. The biological compartments that cannot pass the filter element are retained above the filter element. This allows to concentrate the biological compartments in an easy and automatable manner. This is a considerable advantage of the filter module according to the invention. The concentrated biological compartments may then be contacted again with a liquid, e.g. to further process, e.g. wash, the filtered and thereby concentrated biological compartments. Preferably, a syringe pump is used to generate a low pressure that sucks a defined volume of liquid from the feed portion through the filter element and into the effluent portion.

### Syringe pump as preferred embodiment

According to the present disclosure, it may be advantageous to apply a defined pressure differential. Therefore, according to one embodiment, a device is applied, capable of generating a pressure differential that is capable of controlling the pressure differential in a defined manner. As a result, a defined amount of material (e.g., liquid) may pass the filter element, which preferably is a membrane. Therefore, preferably a device capable of generating a pressure differential is applied that can generate a defined pressure differential. This allows to suck a defined volume of liquid from the feed portion through the filter element to the effluent portion. For instance, using a syringe pump allows to let a defined volume of liquid pass the permeable filter element, which preferably is a membrane. Thus, according to a preferred embodiment, a syringe pump is applied to generate a pressure differential. Other devices to create a low pressure may also be applied. For instance, a pressure control unit may measure the pressure constantly and thereby adjust the pressure differential. As a result, similar to the syringe pump, a defined volume may be removable. However, the use of a syringe pump has the advantage that a defined volume can be removed. The volume can advantageously be controlled in a defined manner.

### Overpressure

According to another embodiment, an overpressure may be generated in order to induce or support material to pass the filter element, which preferably is a membrane. For instance, after adding the sample, the second opening may be connected to a device capable of generating a pressure differential, wherein an overpressure is generated. The overpressure preferentially is generated above the filter element, preferably the membrane such that the liquid surrounding the plurality of biological compartments is induced or supported to pass the filter element, preferably the membrane. For instance, a device such as a syringe pump may be connected to the first opening and a defined overpressure is generated above the filter element, preferably the membrane, to induce or support a defined liquid surrounding the plurality of biological compartments to pass the filter element, preferably the membrane.

### Multiple filter modules

In embodiments, multiple filter modules are applied, preferably as part of an automated system. In case multiple filter modules are applied, it is within the scope of the present disclosure to provide one or multiple devices for generating a pressure differential. For instance, in case one filter module is applied, one device capable of generating a pressure differential such as a syringe pump may be applied for generating a pressure differential. In case two filter modules are applied, two devices such as syringe pumps may be applied. Alternatively, one device, e.g. a syringe pump, may be applied for the two filters modules but a valve is connected in between to shift the pressure operations (e.g. to apply a pressure differential to the desired filter module). Other configurations and methods to apply a defined pressure using a syringe pump can be applied in scope of the present disclosure.

Moreover, parallel exchange of the liquid surrounding the plurality of biological compartments may be performed. For instance, it may be desirable to process larger sample volumes. Therefore, it may be desirable to parallel add portions of the plurality of biological compartments into multiple filter units (e.g., 2, 3, 4, or 5 filter units) in order to parallel remove the liquid surrounding the plurality of biological compartments. Therefore, one or more devices capable of generating a pressure differential may be applied.

### The second opening for inserting the sample

According to the present disclosure, the filter module, preferably the hollow, elongated body comprises a second opening, which is configured such that a fluid, preferably comprising a plurality of biological compartments such as cells can be added through the second opening, e.g. into a portion of the elongated hollow body. Therefore, the second opening may have a diameter which is sufficient to enable a fluid flow or preferably a pipette tip to pass and eject the fluid. For instance, a pipette comprising a tip capable of holding a fluid in the range of 1 µL to 10 mL may at least partially enter through the second opening. Afterwards, the pipette may eject all or some of the fluid, which is then present inside the hollow, elongated body. As noted above, a well may be formed by the comprised filter element (e.g. corresponding to the bottom of the well) and the walls are provided by the walls of the hollow elongated body. The second opening may preferably be open but can also be closable and may thus be closed if desired.

### Top and bottom part of filter module

According to one embodiment, the filter module is provided by two or more components. In one embodiment, it comprises a top part and a bottom part. The top part may serve as feed portion, the bottom part as effluent portion. The top and the bottom part are configured to be connected, for instance by screwing, clamping or any other means for assembling two parts. Details were already described above. In the assembled state, the top and bottom part provide each at least one opening. Thereby, advantageously a simple configuration is provided, which supports the prevention of undesired pressure reduction by potential leaks. The assembled top part and bottom part may form the hollow, elongated body. A filter element may be comprised in the hollow elongated body.

### Clamping the filter element

In the connected state, the bottom and top part can advantageously clamp the filter element, which preferably is a membrane. For instance, the membrane can be clamped directly using the top and bottom part. Preferably, clamping includes one or more sealing means/filter element holders, also referred to as sealing/membrane holders. For instance, the sealing means/filter element holder may be pre-assembled to hold the filter element, which preferably is a membrane, and at the same time is capable of being held by the assembled top and bottom parts. According to one particular embodiment, the filter element, preferably the membrane, is held by two sealing means/filter element holders. In case two sealing/membrane holders hold the membrane, a first sealing mean/filter element holder may surround the filter element (preferably a membrane) circumferentially and partially from one side, and a second sealing mean/filter element holder holds the filter element (preferably a membrane) partially from the other side. Thereby the filter element such as a membrane is advantageously held in a stable position, while the plurality of biological compartments which may be surrounded by respectively comprised in a liquid can contact the filter element, such as a membrane. Therefore, by having a contact to the filter element an applied pressure differential may directly affect the biological compartments comprised in the liquid (e.g. by sucking the liquid through the filter element such as a permeable membrane but retaining the plurality of biological compartments at the filter element).

According to one embodiment, the filter module is configured to hold the membrane (as exemplary filter element) such that the membrane separates at least a portion of the top part and at least a portion of the bottom part of the filter module, preferably the membrane separates at least a portion of the top part of the elongated, hollow body and at least a portion of the bottom part of the elongated, hollow body. The portion above the filter element (preferably a membrane) may be referred to as a "well". The well may correspond to the region wherein the plurality of biological compartments is initially added, e.g. for the exchange of the liquid surrounding the plurality of biological compartments. The region below the filter element, preferably the membrane, may be the region, at which the low pressure is applied.

### Separation

The permeability of the filter element, preferably the membrane, for a fluid allows to separate a fluid that is filled into a portion of the filter module (e.g., a containment above the membrane/filter element, which may correspond to a "well"). It allows to separate at least a portion of the fluid, such as a liquid, from comprised biological compartments. For instance, a plurality of biological compartments, preferably in form of a suspension, may be provided into the well. This may be done in an automated manner using a pipetting module. According to one embodiment, in connection with the filter element the feed portion or top part forms a containment, into which a plurality of biological compartments can be filled. As disclosed herein, the biological compartments are usually comprised in a liquid. The containment ("well") may be dimensioned to receive a volume of 100mL or less, 50mL or less or 30mL or less. In embodiments, the receivable volume is 25mL or less, 20mL or less, 15mL or less or 10mL or less. In further embodiments, the receivable volume is 7mL or less, 5mL or less or 3mL or less.

The filter element, preferably the membrane, may be configured such, that a portion of the suspension can pass the filter element, preferably the membrane, while the remaining portion of the cell suspension cannot pass the filter element, preferably the membrane.

According to a preferred embodiment, the liquid that surrounds respectively comprises the biological compartments in suspension can pass the filter element (preferably a membrane), while the biological compartments cannot pass the filter element (membrane). According to one embodiment, the passing of the portion of the biological compartments suspension, such as the liquid, may be induced or supported by applying a pressure differential, e.g. using a syringe pump. Therefore, a device may be connected to the bottom of the well below the membrane.

According to one embodiment, the pump mechanism is based on a syringe pump due to the fact that smaller flow rates can be adjusted compared to a pressure control - even with a low fluid resistance in the system. According to the present disclosure, different fluidic pump protocols and variations for pumps and individual membrane clamping devices are applicable. Potential recovery rates are usually above 50% and typically in the 70-90% range. A high measured efficiency of such a process was 94% recovery of the cells that were added to this filter module for washing.

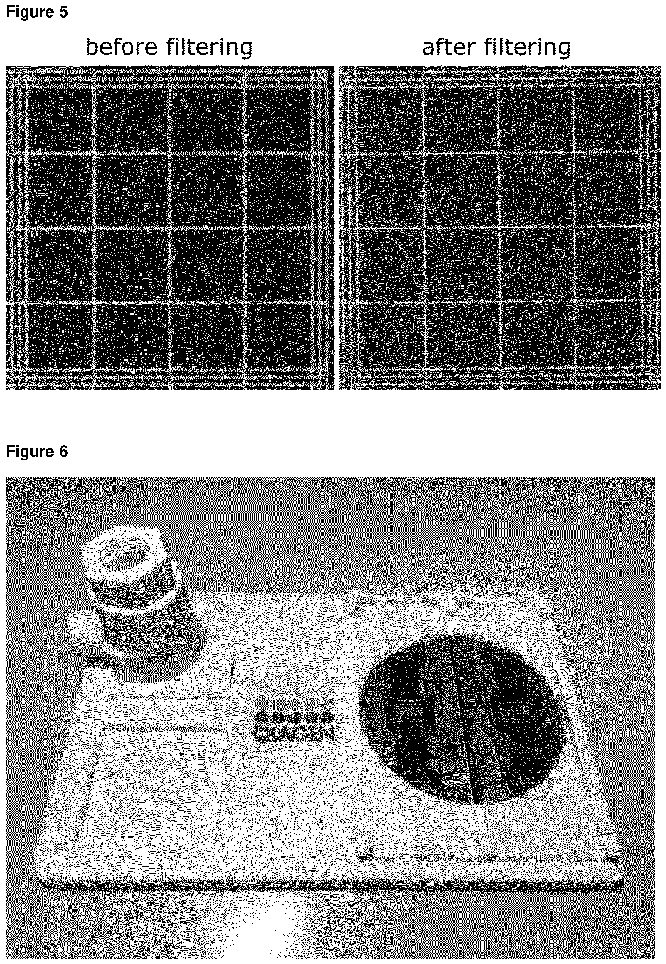

According to the following exemplary embodiment illustrated in Fig. 5, cells were recovered at a rate of approximately 80%. The microscopic images show that the washing process in the filter module does not negatively affect the cells, as the cells remain undamaged. According to this exemplary embodiment, the pore size of the track-etched membranes of 0.1 µm fits for this filter function to conventional cells, which have a typical size of 5 to 20 µm in suspension. The track-etched membranes are also available with smaller pore sizes for the same material. Therefore, the disclosed filter principle can be transferred directly to isolated cell nuclei, which typically have a dimension of about 1 µm (e.g., for the buffer change).

### Contact surface holding membrane

According to one embodiment, the bottom part of the hollow, elongated body comprises a contact surface capable of holding a sealing means/filter element holder, e.g. a first sealing/membrane holder. Alternatively, the filter element, which preferably is a membrane, may be directly held by the contact surface. The contact surface may provide one or more contact surfaces with which the sealing means/filter element holder (e.g. sealing/membrane holder) or the filter element (e.g. membrane) gets in contact. Therefore, the sealing means/filter element holder or filter element (e.g. membrane) is held at the position of the contact surface and is not moved due to an application of a pressure differential. This advantageously avoids the filter element to become loose and being sucked towards the first opening. The contact surface may comprise a circumferential surface present at the inner surface of the bottom part providing the effluent portion. The contact surface may be one complete contact surface or multiple contact points/areas. Embodiments were also described above.

According to one embodiment, the bottom part is configured to be connected to the device capable of generating a pressure differential, which preferably is a syringe pump. Therefore, the bottom part may comprise the first opening disclosed above. Moreover, the bottom part may comprise a connecting part, by which it can be connected to the top part. Therefore, for instance a screw surface may be provided at the inner surface of the bottom part. A complementary screw surface may be provided at the outer surface of the top part (or vice versa). The top part may provide the feed portion and the bottom part the effluent portion.

According to one embodiment, the filter module, e.g. the bottom part, may comprise a stand, e.g. a recess onto which the filter module can stably stand. Alternatively, any kind of clamping or screwing mechanism may be provided e.g. to the bottom part in order to stably hold the filter module during the pipetting operations. This is particularly advantageous when using the filter module within an automated system.

According to one embodiment, the filter module comprising the different disclosed parts are provided separately or partially assembled. This has the advantage, that the user can flexibly change the components of the filter module, e.g. for separating the filter element (e.g. membrane) or sealing means/filter element holder(s). According to another embodiment, the filter module is provided as one part, e.g. in form of a consumable, wherein the user can preferably easily exchange the filter module (e.g., by re-attaching the device capable of generating a pressure differential).

According to a preferred embodiment, the filter module is capable of being implemented into an automated robotic platform. Therefore, the filter module may have an electronic interface by which the filter module can be connected to a processing unit. Moreover, the device may be controlled by an algorithm for automating a method. Also disclosed is an automated system comprising an according filter module.

Moreover, according to one embodiment, the mixing method described herein can be advantageously performed for resuspension. The mixing method can be used in order to resuspend biological compartments that have been filtered with the filter module according to the present invention. After filtering, the biological compartments are comprised in the feed portion. As noted above, the may be comprised in a small amount of liquid that has not been removed by the filtering action, thereby providing the biological compartments in concentrated form. The biological compartments may then be contacted again with a liquid, e.g. a buffer solution, in order to resuspend the biological compartments therein. The resuspension process can be performed using the mixing process described herein. For instance, the mixing may be performed after adding a solution (e.g. ligation buffer). Finally, the plurality of biological compartments is resuspended in a liquid, preferably a buffer suitable in scope of a ligation reaction. The resuspended biological compartments may then be transferred into a collection containment. Buffer may be added to the portion of the filter module (e.g. the "well") and mixing according to the present disclosure may be performed in order to e.g. release adhered biological compartments. This may be followed by transferring the remaining plurality of biological compartments resuspended in the buffer into a collection containment. The steps of releasing adhered biological compartments transfer to a collection containment may be repeated one, two, three or more times. In one embodiment, the filter module is comprised in an automated system and the resuspended biological compartments are transferred from the filter module (e.g. from the "well" formed in the filter module) to a collection containment located in the automated system, e.g. a well of a well plate that is comprised in the automated system. Transfer may be achieved by a pipetting module of the automated system.

### Exemplary embodiment of filter method

According to an exemplary embodiment, one or more of the following steps may be performed in the disclosed filter method, that may be performed using the filter module of the present disclosure:
- Pipette 1 mL wash buffer onto the membrane (filter element) of the filter module and then aspirate 1 mL via a device capable of generating a pressure differential (e.g. a syringe pump).
- Transfer 500 µL of the plurality of biological compartments (e.g., cells from the cell pool) to be washed into a portion of the filter module (e.g. the feed portion) and aspirate 400 µL using a syringe pump.
- Transfer the remaining approx. 500 µL of the plurality of cells (from the cell pool) to be washed into the portion of the filter module and aspirate 500 µL using a syringe pump. Thereby, the cells from both transferred aliquots (500µl each) have been filtered and are comprised in the feed portion in concentrated form.
- Transfer 500 µL of the wash buffer into the portion of the filter module using a pipette.
- Suction of 500 µL by syringe pump from the portion of the filter module.
- Add 500 µL of the wash buffer into the portion of the first type filter module using a pipette.
- Suction of 500 µL by syringe pump from the portion of the filter module.
- Resuspend the cells of the portion of the filter module well by adding 500 µL wash buffer with a pipette and perform mixing. The tip of the pipette is guided close to the membrane (filter element) to remove any adhesive cells. Pipette the resuspended cells into a collection containment.
- Perform the last step preferably more than once, e.g. for a total of four times.
- The cell suspension is thus present in 2000 µL of the wash buffer.

As will be appreciated by the skilled person, this method may be modified e.g. by processing or transferring e.g. different volumes.

### Further uses of the filter module

The filter module described herein may be used in order to concentrate objects comprised in a fluid. The objects to be concentrated are retained by the filter element, while the surrounding fluid may pass, thereby allowing to achieve a concentration of the comprised objects in a controllable manner. Advantageously, this concentration step may be automated. In the context of the method according to the first aspect, it may be used for concentrating a plurality of biological compartments comprised in a fluid, e.g. after performing a counting step as described herein. This allows to concentrate a cell suspension prior to performing step (a) ("split"), e.g. in case counting of the biological compartments reveals that the concentration of the biological compartments within the suspension is too low to transfer a suitable or desired amount of cells during aliquoting. The concentrating is subsequently predominantly described for concentrating biological compartments comprised in a fluid. The same disclosure, however, applies to other objects comprised in a fluid that may be concentrated analogously. The pore size/openings of the filter element are chosen such that the objects to be concentrated are retained.

This concentrating step using the filter module can again be performed in an automated manner as described herein. E.g. a certain amount of liquid may be withdrawn with the aid of the filter module (preferably using a syringe pump for applying the pressure differential), leaving behind a more concentrated cell suspension in the feed portion. In this embodiment, it is not necessarily required to exchange the medium, as the removal of a certain amount of liquid (as required to achieve the desired concentration effect) is sufficient to achieve the desired concentration effect. The concentrated liquid comprising the plurality of the biological compartments may then be removed from the filter module and further processed, e.g. subjected to step (a). This is advantageous, because the system can automatically perform such concentrating step, without requiring interference by the user.

### Fixing and permeabilization of the biological compartments

According to on embodiment, the method further comprises fixing and permeabilizing the plurality of biological compartments prior to step (a).

### Fixing of biological compartments

According to a preferred embodiment, the biological compartments are fixed. Hence, the method may comprise fixing biological compartments. The fixing step may also be performed on an automatic system such as the robotic system disclosed herein.

The biological compartments may be fixed prior to performing step (a). Preferably, the biological compartments are fixed prior to any modification or processing of molecules comprised within the biological compartments, such as e.g. prior to reverse transcription of comprised RNA and in any case prior to adding the first barcode label.

Fixation of the biological compartments, e.g. by formalin, is preferably performed at the beginning of the process. However, fixing biological compartments oftentimes requires handling of hazardous chemicals (e.g. formaldehyde or paraformaldehyde) and the fixation efficiency is dependent on the experimenter. In particular, fixing of biological compartments according to the prior art protocols necessitates centrifugation and manual resuspension steps. These steps are error-prone and can result in a significant loss of biological compartments. Moreover, the transcriptomic profile of the biological compartments may be altered due to the handling of the biological compartments (e.g. if not processed fast enough). The present disclosure can avoid these drawbacks by automating the fixing step. Therefore, the fixing of the plurality of biological compartments can be improved in regard to reproducibility and allows for yielding high numbers of biological compartments. Moreover, the costs for personnel are reduced. Furthermore, pipetting errors by the user can be avoided.

Advantageously, fixing a plurality of biological compartments can be performed in an automated manner. For this purpose, the filter module described herein may be applied. The present disclosure advantageously allows to automate the fixing step using the filter module described herein which allows to separate biological compartments from a liquid surrounding. The filter module solves the challenge of exchanging a liquid surrounding a plurality of biological compartments, preferably in an automated manner. In essence, the suspending fluid is sucked off via the filter while the biological compartments are retained. The biological compartments can then be completely resuspended by adding a new buffer. Various embodiments of this principle are within the scope of the present invention. The filter module can be implemented into the system of the present disclosure or into existing automated platforms and is therefore, highly versatile.

Fixing of a plurality of cells may be performed by contacting the plurality of biological compartments with a fixing compound. The fixing compound may be provided in solution (e.g. by dissolving or diluting the fixing compound). Suitable fixing compounds and fixing solutions are well-known in the art. Fixing of a plurality of biological compartments may be e.g. performed by adding a solution comprising formalin or paraformaldehyde. Fixation advantageously can reduce perturbations to endogenous gene expression (i.e. allows to arrest the state of the transcriptome) during handling of the biological compartments and allows to store biological compartments for future experiments (see also Rosenberg *et al*.).

The plurality of biological compartments may be e.g. fixed using formaldehyde in phosphate buffered saline (PBS). The plurality of biological compartments may be fixed, for example, in about 1-4% formaldehyde in PBS. In various embodiments, the plurality of biological compartments may be fixed using methanol (e.g. 100% methanol) at about -20 °C or at about 25 °C. In various other embodiments, the plurality of biological compartments may be fixed using methanol (e.g., 100% methanol), at between about -20 °C and about 25 °C. In yet various other embodiments, the plurality of biological compartments may be fixed using ethanol (e.g., about 70-100% ethanol) at about -20 °C or at room temperature. In yet various other embodiments, the plurality of biological compartments may be fixed using ethanol (e.g., about 70-100% ethanol) at between about -20 °C and room temperature. In still various other embodiments, the plurality of biological compartments may be fixed using acetic acid, for example, at about -20 °C. In still various other embodiments, the plurality of biological compartments may be fixed using acetone, for example, at about -20 °C. Other suitable methods of fixing the plurality of biological compartments are also within the scope of this disclosure.

The fixing of a plurality of biological compartments typically requires biological compartments to be separated from the surrounding fluid. "Separating" the surrounding fluid and the biological compartments should be understood as separating the predominant volume of the surrounding fluid. Separation may lead to the generation of a wet pellet of biological compartments. After having separated the biological compartments, a fixing compound (which may be present in a solution) may be added to the biological compartments. Alternatively, the fixing compound may be directly added to the plurality of cells without a prior separation step. Therefore, it may be desirable to add the fixing compound at a high concentration, achieving a concentration sufficient to fix the cells in the resulting mixture.

After having added the one or more fixing compounds, the resulting mixture may be incubated. Incubation may be performed at defined conditions (e.g. adjusted time and temperature). For example, incubation may be conducted for some minutes (e.g. 10 minutes) at room temperature. The particular incubation conditions may not be limiting according to the method of the present disclosure.

Afterwards, the fixed biological compartments may be again separated from the surrounding fluid. This step can advantageously stop the fixing compound from further affecting the biological compartments. Separation can be performed by using the filter module according to the present disclosure. Finally, a solution such as a buffered solution may be added in which the fixed biological compartments are resuspended. In order to remove impurities (e.g. from the fixing solution), the biological compartments may be separated from the surrounding fluid and washed, e.g. resuspended in a solution one, two or more times. The removal of liquid may be performed using the filter module according to the present disclosure. Alternatively, methods known in the field may be applied (e.g. centrifuging and liquid removal). The prior art protocols require manual addition of reagents by pipetting and washing and by centrifugation. According to the present disclosure, a filter module is provided that allows to automate the process of fixing cells, e.g. using conventional formalin or formaldehyde-based protocols. The present disclosure provides the technical solution for automating the cell fixing step by making use of a filter module described herein.

After fixing the biological compartments, the plurality of biological compartments may comprise aggregates of biological compartments (e.g. multiple cells or nuclei which are associated). Preferably, the method comprises removing cell aggregates from the plurality of biological compartments after fixation, preferably by passing the plurality of fixed biological compartments through a sieving module. Removal of aggregates is advantageous as it improves the efficiency of the subsequent barcode-labeling. Otherwise, there is a risk that the target molecules of the biological compartments in the aggregate become labeled by the same barcode, so that a single biological compartment within the aggregate cannot be differentiated from the other biological compartments of the aggregate. Aggregate removal may be performed manually, e.g. using prior art methods, which typically require use of a sieve. However, manual procedures for removing cell aggregates even though feasible, have drawbacks as they tend to cause spillage and loss of biological compartments.

The present disclosure allows to avoids the drawbacks of a manual method by providing an advantageous sieving module. This sieving module advantageously allows to remove aggregates of biological compartments in an automated manner. The sieving module can be advantageously implemented into an automated system, such as the automated system according to the present disclosure or into prior art automated platforms. The sieving module is described elsewhere herein and it is referred to the respective disclosure.

### Permeabilization

In certain embodiments, the method comprises permeabilizing the plurality of biological compartments. Permeabilization of the biological compartments facilitates the performance of *in situ* reactions within the cells. E.g. permeabilization of the cells facilitates the introduction of reaction compounds, such as primers and/or barcode labels. Permeabilization is preferably performed prior to step (a).

Any suitable method suitable for permeabilization of the biological compartments, such as e.g. cells or cell nuclei, may be used. Such methods are well known in the art, including the prior art cited herein and therefore, do not need any detailed description. For example, holes or openings may be formed in outer membranes of the plurality of biological compartments. TRITON™ X-100 may be added to the plurality of biological compartments, followed by the optional addition of HCI to form the one or more holes. About 0.2% TRITON™ X-100 may be added to the plurality of biological compartments, for example, followed by the addition of HCI. In certain other embodiments, the plurality of biological compartments may be permeabilized using ethanol (e.g., about 70% ethanol), methanol (e.g., about 100% methanol), Tween 20 (e.g., about 0.2% Tween 20), and/or NP-40 (e.g., about 0.1 % NP-40).

### Cell counting

For split-and-pool protocols it is advantageous to know and/or adjust the concentration of biological compartments, as preferably a defined (approximate) number of biological compartments is divided into aliquots, e.g. by transfer to a plurality of containments (e.g. wells of a multi-well plate). Therefore, the concentration of the biological compartments (such as cells) is typically determined. This preferably involves a cell counting step.

In a preferred embodiment, the method thus further comprises performing a biological compartment counting step, such as a cell counting step. The cell counting step is preferably performed in an automated manner. For counting, a counting module may be used that can count the cells in an automated manner, preferably the novel counting module as described herein. In one embodiment, the number or concentration of the plurality of biological compartments is calculated based on the biological compartment count (e.g. cell count).

Afterwards, the biological compartment concentration may be adjusted manually or automatically. According to the state of the art usually manual procedures are performed. Yet, the manual procedures are prone to errors and oftentimes lack reproducibility. In particular, the counting of biological compartments is typically performed by microscopic analysis of a grid, upon which a cell suspension was spread. The identification of the biological compartments on the grid requires an experienced user. Moreover, subsequent calculations regularly lead to errors, which can negatively affect the efficiency of the barcode-labeling protocol. The present disclosure also provides counting modules that can be automated and which avoid prior art drawbacks.

According to a preferred embodiment, biological compartments of the plurality of biological compartments are counted prior to performing step (a). It is not required to count all biological compartments of the plurality. It is sufficient to use a portion of the plurality of biological compartments, which preferably are provided in a suspension, for counting, such as cell counting. In an embodiment, an aliquot of the biological compartments is subjected to the counting step. Advantageously, the biological compartments which are not counted are not affected by the counting step. For instance, biological compartments of the plurality of biological compartments may be further processed for counting (e.g. staining, etc.). The remaining plurality of biological compartments is not further processed and are therefore not affected by these processing means.

According to one embodiment, biological compartments of the plurality of biological compartments are transferred into another containment for counting. The containment can be a reaction containment. The reaction containment can be advantageously used in order to further process the biological compartments. Preferably, a portion of the plurality of biological compartments is transferred to one or more counting chambers. The counting chamber may be provided by a hemocytometer.

According to one embodiment, the further processing of the biological compartments for counting includes changing the optical properties of the biological compartments. According to one exemplary embodiment, the biological compartments are stained. Staining can change the optical properties of the biological compartments in order to improve a subsequent optical analysis. For instance, cells or nuclei may be stained with trypan blue (e.g., at a 1:1 ratio with 0.4% trypan blue). Other stains are readily available to the person skilled in the art. These prior art stains may be contacted with the cells in order to enhance the optical properties.

According to one embodiment, biological compartments of the plurality of biological compartments that may optionally be further processed (e.g. by staining) are transferred into one or more counting chambers, such as preferably a hemocytometer. A hemocytomer can be used for counting cells and other biological compartments as is known in the prior art.

The transfer may be performed manually or automatically. For automatic transfer, a pipetting module may be used, wherein the pipetting module may advantageously be automated. The transfer of the biological compartments may be performed by pipetting (manually or automatically) through a filling opening of the counting chamber. Therefore, the one or more counting chambers may be provided at a filling position (e.g. a position which can be filled by a pipetting module during an automated workflow).

According to one embodiment, more than one counting chamber is used. For instance, 2 or 3 or 4,5,6,7,8 or even more counting chambers may be used. Accordingly, each counting chamber may be filled by or through a different filling opening. According to one embodiment, the (optionally further processed) biological compartments may be pipetted through the filling opening at a different dilution ratio. For instance, a dilution series may be prepared before or during filling. A dilution series may include pipetting following samples through each filling opening of the respective counting chambers: e.g. undiluted sample, 1:10 diluted sample, 1:100 diluted sample and 1:1000 diluted sample. The particular dilution rate shall not be limiting. It is advantageous to provide the sample at different dilution rates as the biological compartment concentration is initially unknown.

After filling, the biological compartments are present in the one or more counting chambers. The counting chambers advantageously allow to count the biological compartments and based thereon to calculate the (approximate) number of the plurality of biological compartments (e.g. the number of cells/nuclei per mL). The calculated biological compartments number/concentration can then be used in subsequent steps, wherein portions of the plurality of biological compartments are transferred into individual containments ("split" procedure).

According to one embodiment, the one or more counting chambers can be manually counted. Therefore, the experimenter may count the biological compartments of the filled counting chambers using a microscope. Other techniques to count the biological compartments are readily available to the person skilled in the art.

According to one embodiment, the method is performed using an automated system, such as a robotic system, which comprises a counting module. Also disclosed is an automated system comprising such counting module. The counting module may comprise one or more counting chambers (e.g., hemocytometer, in this example a "c-chip in the Fuchs-Rosenthal scheme"). The one or more counting chambers can be held in position on the automated system (e.g. the deck of a robotic system) by a holding device. Due to this holding device, the filling opening(s) of the one or more counting chambers is given defined coordinates, so that the one or more chambers can be filled by pipetting using a pipetting module (e.g., of a pipetting robot). Therefore, the transfer of the portion of the plurality of biological compartments (such as an aliquot of the cell suspension) into the one or more counting chambers can be automated.

An example of such a holding device is shown in **Fig. 6****,** where two c-chips as an embodiment of a counting chamber (here a hemocytometer) are firmly clamped and thus brought into position of the deck of a robotic system. The pipetting robot can, for example, insert 1-4 samples with biological compartments (e.g. cells or nuclei) into one counting chamber each (e.g., volume 20 µL). In this way, a dilution series of a biological compartment suspension (e.g. cell or nuclei suspension) can be created, for example undiluted, 1:10, 1:100, 1:1000. This makes it possible to measure wide ranges of initially unknown biological compartment concentrations in the workflow.

According to one embodiment, the user removes the counting chambers (e.g., C-chips) from the holding device and evaluates the cells at the microscope according to conventional methods.

According to another embodiment, which can be advantageously applied for automation according to the present disclosure, an imaging device is directly provided on the deck of the disclosed system. The holding device holding the counting chamber(s) may engage with a sliding device that allows to move the holding device /holder and thus the counting chambers into a position that is accessible for the optical path of an imaging device, such as a microscope ("counting position"). The holding device is preferably axially movable in relation to the sliding device which provides guidance for the axial movement. To provide an automated system with such sliding or displacement function is advantageous to avoid a collision of imaging device parts and pipetting modules travel paths, which are close together. The sliding mechanism that can be used in the method according to the present disclosure advantageously solves this problem. In particular, it makes clever use of the available workspace within a robotic system.

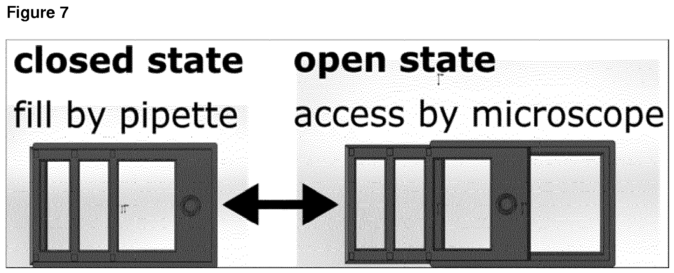

An embodiment is illustrated in **Fig. 7****.** Shown is the holding device for the counting chambers which engages with a sliding device, that enables axial movement of the holding device. In the shown embodiment, the sliding device is provided by a sliding frame, which receives the holding device. In the closed state shown on the left, the holding device is within the sliding frame and the counting chambers may be filled using a pipetting module. Advantageously, the openings of the counting chambers are accessible for the pipette tips. For transferring the loaded counting chamber to the imaging device, the holding device and thus the loaded counting chambers are axially moved so that the counting chambers are transferred to the counting position where they are accessible for the counting device, such as the optical path of an imaging device (see right side, open state). The sliding device guides the axial movement of the holding device, thereby enabling a precise operation.

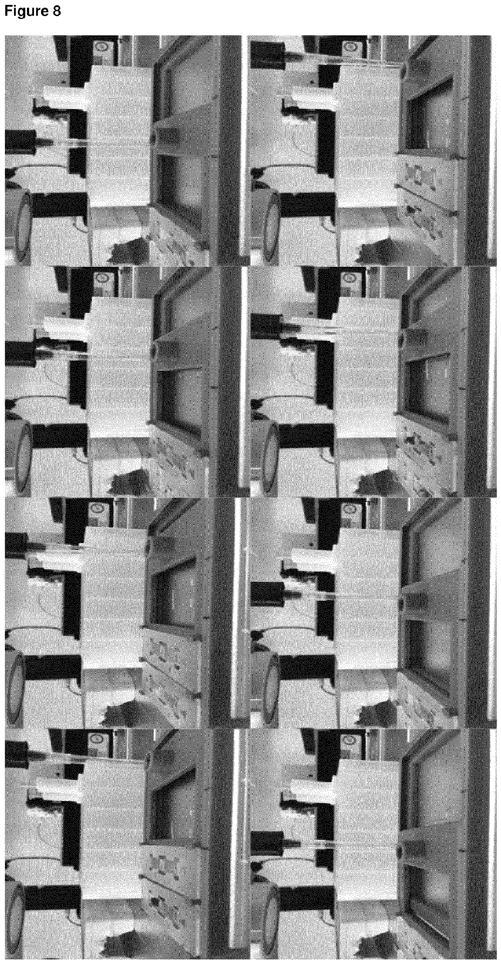

As disclosed herein, for axial movement of the holding device, the pipetting module may be used. In order to bring the counting chambers into the field of view of the microscope, the present disclosure teaches to use the pipetting module (in particular the pipetting robot head) for performing the axial movement. This is illustrated in **Fig. 8****.** As shown in this figure, the pipetting tip may engage with the holding device (e.g. by entering engagement means provided on the holding device, in the shown embodiment the engagement means are provided by a ring) and carries out a movement in the direction of displacement from this position. In the shown embodiment, the pipetting module carries out the movement from right to the left. The holding device is thereby slided to the left. The movement is guided by the provided sliding device which is here provided by a sliding frame. The holding device may then also be slided back to the original position.

The solution which is described in further detail below saves additional (electro-) mechanical device for moving the holding device. With this sliding or displacement function, the counting chambers enter the field of view of the camera.

In this way, images of the biological compartments in the counting chamber can be taken with the imaging device (e.g., microscope).

According to one embodiment, the pipetting robot instructions are implemented by an algorithmus. Preferably, such sliding mechanism is incorporated into the automated system that may be used to perform the method according to the first aspect.

### Automated counting

In an advantageous embodiment of the present method, the cells are counted in an automated manner. The present disclosure avoids the drawbacks of the prior art by providing a counting module that allows to count the biological compartments in an automated manner as already described above. This advantageous embodiment is described in further detail below.

The counting module may comprise a holding device configured to hold the one or more counting chambers. The mode of holding of the counting chambers may not be limited and *inter alia* includes clamping, screwing, adhesion or simple gravity.

The holding device preferably comprises or engages with a sliding device configured to axially direct the holding device/holder. The sliding device may be provided e.g. by a sliding frame which engages with the holding device as is also illustrated in the Figures. Alternatively, the sliding device may be provided by one or more tracks or rails that guide the movement of the holding device. As disclosed herein, the pipetting module of a robotic system may be advantageously used for moving the holding device.

According to a preferred embodiment, the holding device holds the one or more counting chambers at a position which allows filling by a pipetting module (may be referred to as "filling position"). After filling, the at least one counting chamber needs to be transferred to a position, wherein the biological compartments are counted (may be referred to as "counting position"). When using an automated system, transferring of the one or more counting chambers is typically required, as the counting usually requires an imaging device which may spatially interfere with the pipetting module. The present disclosure advantageously avoids any interference by axially moving the holding device and thereby the one or more counting chambers to one or more other ("counting") position(s).

The axial movement can be performed by various modes. For instance, mechanical or electromechanical devices may be integrated. According to a preferred embodiment of the present disclosure, the pipetting module is applied for axial movement of the holding device (also referred to as holder herein). In one embodiment, the holding device/holder can engage with the pipetting module. For instance, the holding device may comprise engagement means, such as an opening or ring structure, with which a part of a pipetting module is capable of engaging. Upon axial movement of the pipetting module the holding device/holder likewise performs an axial movement, and preferably performs an equivalent axial movement. In order to avoid any contact/interference between the pipetting module and an imaging device, a certain distance between the engagement position and the at least one counting chamber is advantageously provided.

In order to avoid any contact/interference between the pipetting module and an imaging device, a certain distance between the engagement position and the at least one counting chamber is advantageously provided. According to one embodiment, the distance between the engaging position and the counting chamber may be chosen such that the pipetting module and the imaging device do not come in contact with each other. Typically, distances between 1 to 10 cm can be selected. However, the particular distance may not be limiting as long as the pipetting module and the imaging device to not come into contact with each other.

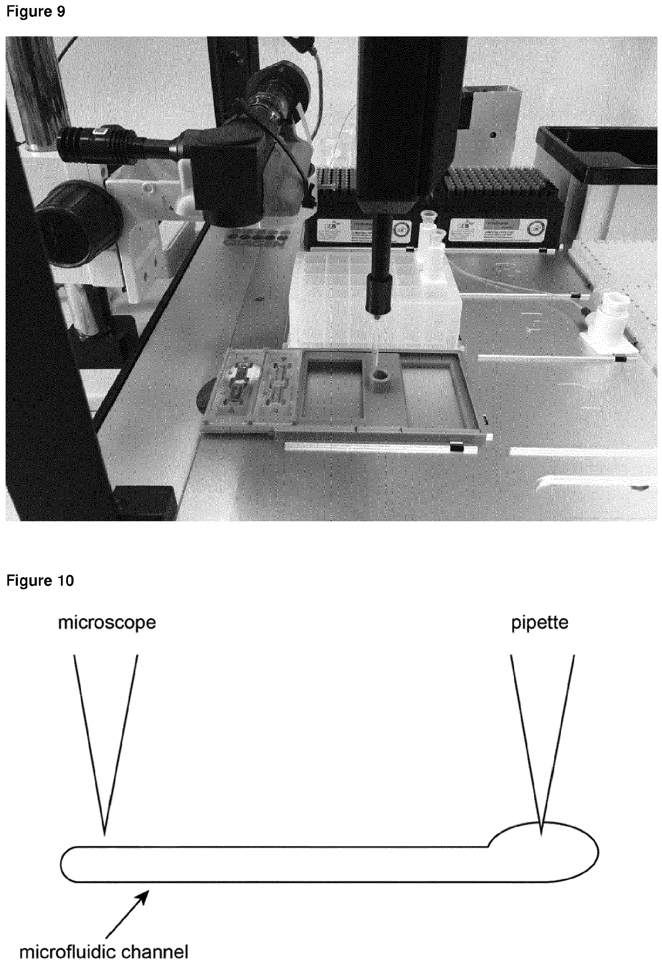

According to one embodiment, the distance between the pipetting module and the counting chamber may be increased by providing a channel between the filling opening and the counting chambers. The biological compartments (preferably a suspension of cells or nuclei) may be added via the pipetting module and the biological compartments move through the channel into the counting chambers. An exemplary illustration is depicted in **Fig. 10****,** wherein the channel may transfer the biological compartments by capillary forces.

**Fig. 10** illustrates a microfluidic channel, which can facilitate transport of biological compartments that are filled using a pipette. The channel may transport the biological compartments towards a counting position (e.g. part of a counting chamber). At the counting position, an imaging device (here microscope) may acquire images of the biological compartments for counting of the biological compartments.

Hence, in an advantageous embodiment, the holding device of the counting module comprises engaging means. In the automated system, the engaging means (also referred to herein as engagement means) can interact with the pipetting module of the automated system, e.g. a pipetting tip attached to the pipetting module. The engaging means may comprise a contact surface which can be used to exert a force on the holding device in axial direction. According to one embodiment, the engaging means comprise a three dimensional geometry, wherein the geometry is selected from the group comprising cube, tetrahedron, pyramid, prism, octahedron, cylinder, cone, sphere, torus and combinations thereof. According to one embodiment, the engaging means comprises an opening configured to be engaged with a part of the pipetting module, preferably a pipet tip. According to a particular embodiment, the engaging means comprise a cylindrical geometry with an opening capable of engaging with part of the pipetting module. Suitable embodiments are also shown in the figures.

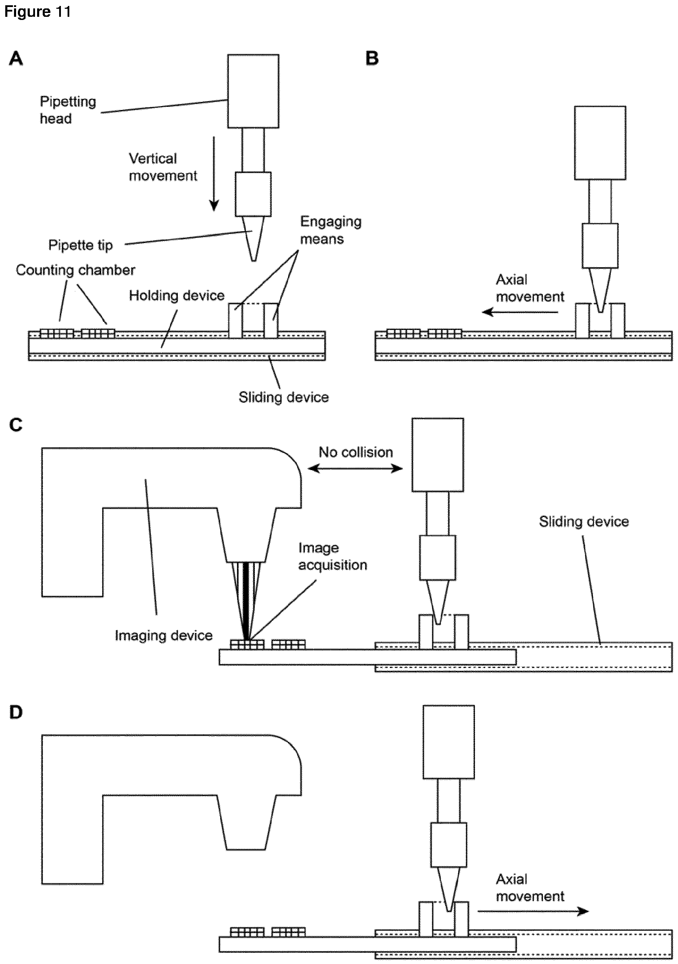

An embodiment of the counting module that allows counting biological compartments such as cells in an automated manner is illustrated in **Fig. 11****.** According to **Fig. 11** **A,** a pipetting module performs a vertical movement towards the engaging means of the holding device. E.g. the pipette tip may contact the holding device by interacting with the engaging means, in the shown embodiment the pipetting tip enters the engagement means that are formed by a ring or cylinder structure present at the surface of the holding device. The holding device engages with a sliding device, which is provided by a sliding frame in the shown embodiment. Engagement of the pipetting module with the holding device via the engagement means is followed by axial movement (see **Fig. 11** **B**). The movement is guided by the sliding device, thereby ensuring a precise operation and movement towards the counting position. The imaging device is located at the counting position. Upon movement of the holding device and accordingly the counting chamber to the counting position, the imaging device can acquire images, which may be used for identifying the biological compartments, followed by counting of the identified biological compartments **(****Fig. 11 C)****.** Then, the pipetting module may perform an axial movement in the opposite direction as in **Fig. 11** **B** to bring the holding device back into the initial position.

At a counting position, biological compartments can be counted which are present in the one or more counting chambers. Counting can be advantageously conducted automatically as described herein. Therefore, the automated system preferably comprises an imaging device, which can acquire microscopic images. For instance, the imaging device may be a bright field microscope capable of imaging the biological compartments present in the one or more cell counting chambers. The particular mode of imaging may not be limited. For instance, imaging may be performed by reflection or by transmittal of light signals to the bright field microscope. Here, it may be advantageous to image biological compartments, which have been changed in their optical properties (e.g. by staining).

In further embodiments, cell counting may be performed using fluorescence microscopy or phase contrast microscopy.

According to a further embodiment, the imaging device or parts of the imaging device may be manually or automatically movable. Moveable or partially moveable imaging devices may be advantageous in order to image a wider area. For instance, when providing counting chamber in at different planar positions of the holding device/holder of the counting module, it may be advantageous to image in X and Y direction. Accordingly, the axial movement of the counting module may facilitate imaging in axial direction (e.g., X axis), while the imaging device is capable of moving along the Y-axis. Therefore, a high number of counting procedures can be performed (e.g., a high number of counting chambers can be imaged). Therefore, the throughput may be further improved.

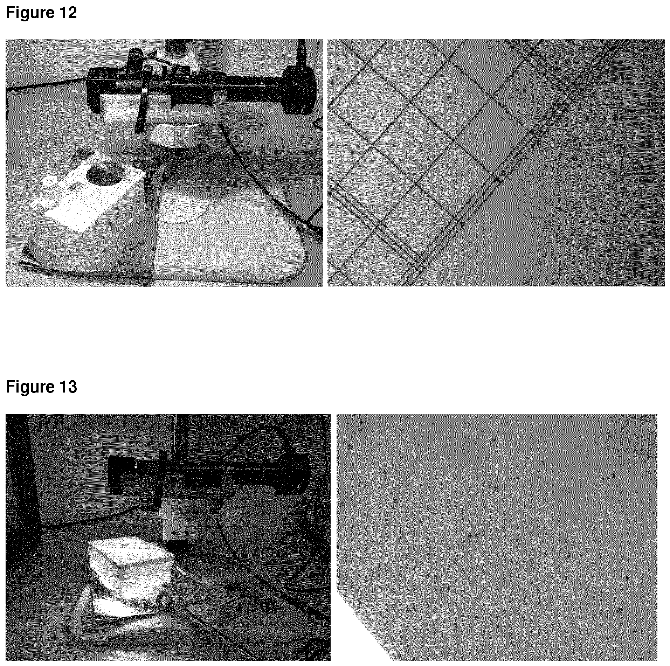

Different variants of the microscope structure are possible to image the biological compartments. According to one embodiment, a bright field microscope with coupled illumination is applied, which images the cells in the counting chamber by reflection on a reflecting surface (e.g. a single crystal silicon wafer can be used). This is illustrated in **Fig. 12****.** The microscope image on the left shows formalin-fixed K562 cells that have been imaged with the structure.

As a further embodiment, the biological compartments (e.g. cells) are photographed in a similar structure, but with a transmitted light source homogenized by a material that makes the light diffuse (e.g. white plastic). This is illustrated in **Fig. 13****.**

Normally, biological compartments are difficult to record in simple brightfield microscopy because the biological compartment interior (e.g., of cells or nuclei) typically provides little imaging contrast. For this reason, according to an exemplary embodiment, formalin-fixed cells were stained 1:1 with 0.4% trypan blue, so that this staining provides sufficient contrast to identify the cells. As described herein, one may only stain the small part of the biological compartments that also enters the counting chambers. Trypan Blue is an application example for this method, but all staining methods giving absorption contrast are conceivable.

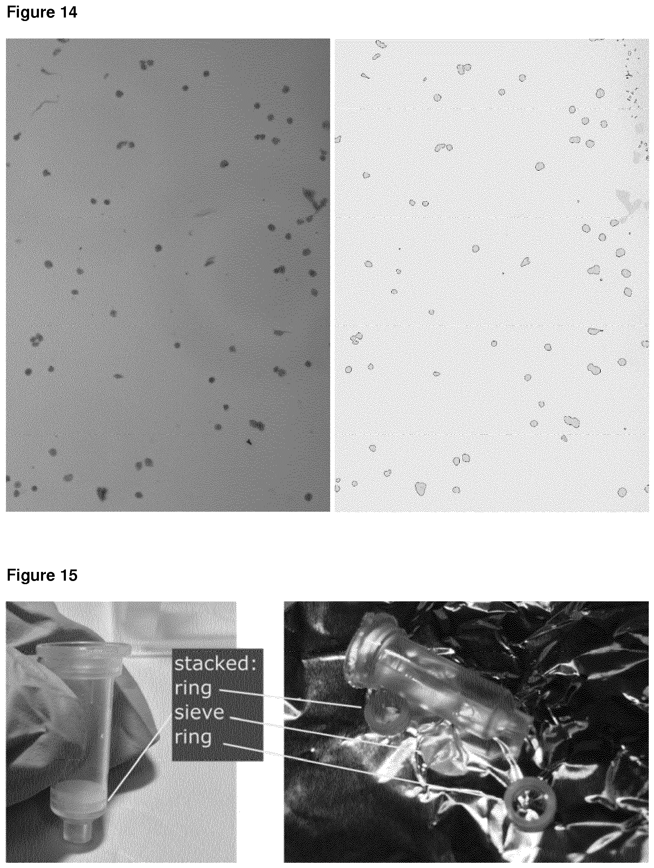

An algorithm for automatic data evaluation may also be advantageously used. This algorithm counts the biological compartments in the images (e.g., microscope images). **Fig. 14** shows a microscopic image of Trypan blue stained cells whose contour was analyzed with the algorithm to finally count the cells. The drawn cell outlines on the right picture show exemplary that automatic cell counting with the described method provides realistic results.

The cell counting step, which is preferably performed in an automated manner, allows to count biological compartments such as cells in a suspension. This provides data regarding the cell number, which can be subsequently used in order to dynamically adjust the workflow that is performed by the automated system. E.g. after cell counting, the cell concentration within a suspension can be adjusted by dilution to provide a cell concentration that lies within a desired range. This is described in further detail below.

Optionally, a cell counting step is performed prior to fixing the biological compartments. This allows to adjust the concentration prior to fixing. However, preferably and importantly, such counting step may be performed after fixing and/or permeabilizing the biological compartments and prior to further processing, e.g. prior to performing step (a) or prior to performing a reverse transcription reaction in case a reverse transcription reaction is performed as first step in order to reverse transcribe RNA into cDNA. As disclosed herein, a first barcode label may be attached during this reverse transcription reaction, as it is also described in the prior art protocols relating to the SPLiT-seq protocol referred to herein.

In a further embodiment, an according counting step is performed after performing repeated rounds of splitting and pooling, e.g. prior to lysing the cells. This would allow to further process only a certain number of biological compartments for subsequent sequencing.

The present disclosure also provides as further aspect of the invention a counting module comprising
- a holding device configured to hold one or more counting chambers; and
- a sliding device configured to axially direct the holding device.

Characteristics of said counting module were already described above. A particular advantage of axially moving the holding device/holder (respectively, the counting chambers if mounted to the holding device/holder) lies in that the available work space can be advantageously maximized. This is of particular advantage when providing an automated system. The counting module may be advantageously used in the methods of the present disclosure to count the biological compartments.

The counting module may comprise one or more counting chambers configured to receive biological compartments through a filling opening. The one or more counting chambers may be mounted to the holding device. The counting device may furthermore comprise an imaging device such as a microscope.

Further optional and preferred features of the counting module that may also be provided in combination are described below. The counting module may be used in the methods of the present disclosure for counting cells. Hence, the counting module may have one or more of the following characteristics:
The holding device may be or is moved axially, wherein moving axially preferably comprises moving the holding device/holder between a filling position and one or more counting positions.

The holding device may be configured so that it can engage with a pipetting module. In one embodiment, the holding device is configured to be moved axially by the pipetting module upon engagement.

In one embodiment, more than one counting chambers are provided, configured to receive biological compartments from the plurality of biological compartments (e.g. cells), which preferably differ in their concentration by providing different dilutions.

The holding device may comprise means for engaging with a part of a pipetting module, such as preferably an attached pipetting tip. The engaging means may have one or more of the following characteristics:
- it comprises a contact surface which can be used to exert a force on the holding device in axial direction;
- it comprises a three dimensional geometry, wherein the geometry is selected from the group comprising cube, tetrahedron, pyramid, prism, octahedron, cylinder, cone, sphere, torus, and combinations thereof;
- it comprises an opening configured to be engaged with a part of the pipetting module, preferably a pipet tip; and/or
- it comprises a cylindrical geometry with an opening capable of engaging with part of the pipetting module.

In one embodiment, the imaging device comprises a microscope. E.g. the imaging device may comprises a bright field microscope coupled with illumination. In one embodiment, the imaging device comprises a microscope configured to move automatically.

A sliding device for axially directing an object using a pipetting module is provided as further aspect of the invention, wherein the object is configured to engage with the pipetting module. Details of the sliding device are also described elsewhere herein.

A method for counting biological compartments and calculating the number of a plurality of biological compartments by an automatic counting module is provided as further aspect of the invention, the method comprising:
(a) filling one or more counting chambers with biological compartments of the plurality of biological compartments, wherein the counting chambers are mounted to a holding device;
(b) moving the holding device axially;
(c) imaging biological compartments present in the one or more counting chambers using an imaging device; and
(d) optionally automatically counting the biological compartments and calculating the number of the plurality of biological compartments.

A system for counting biological compartments and calculating the number of a plurality of biological compartments is provided as further aspect of the invention, the system comprising:
- a pipetting module;
- one or more counting chambers configured to receive biological compartments of the plurality of biological compartments through a filling opening;
- a holding device configured to hold the one or more counting chambers;
- a sliding device configured to axially direct the holding device/holder using a pipetting module; and
- optionally an imaging device.

Details of the counting chamber, the holding device and the sliding device are also described elsewhere herein and it is referred to this disclosure. Further features are described in the following. One or more of the following characteristics may be fulfilled:
The system may further comprise a processing unit and a program for automatically adjusting the biological compartment concentration. The automatic adjustment may comprise one or more of the following features:
- wherein liquid is added to the plurality of biological compartments;
- wherein the liquid surrounding the plurality of biological compartments is exchanged using a filter module, wherein preferably the exchanged liquid comprises a lower volume than the initial liquid surrounding the biological compartments;
- wherein transferring aliquots of the plurality of biological compartments comprises transferring less aliquots;
- wherein the plurality of cells are concentrated using a filter module, preferably the filter module as described herein.

Details of the filter module according to the present invention are disclosed elsewhere herein and it is referred to this disclosure.

### Sliding device

According to the present disclosure, as further aspects of the invention, a sliding device and method is provided for axially directing an object using a pipetting module, wherein the object is configured to engage with the pipetting module. The object may be any object that can be axially directed by the pipetting module. Therefore, the object preferably comprises engaging means. Engaging means are described in the present disclosure and it is here referred thereto. As described herein, such sliding device may be used in order to axially move the holding device for a counting chamber and/or may be used in order to facilitate the sealing of containments comprising biological compartments (e.g. wells of a well-plate) in an automated manner, e.g. by precisely placing the sealing means over the containments for closing.

This sliding mechanism may therefore be preferably implemented into an automated system of any kind that comprises a pipetting module. Many applications are feasible and the disclosure is not limited to the herein described specific embodiments. E.g. this mechanism may be used to axially direct objects such as sample holders or containments, e.g. well plates and other consumables, lids or other objects in a precise manner. As disclosed herein, the sliding mechanism is advantageous, as it allows to make efficient use of the available working space in a robotic system and allows to use existing modules, such as a pipetting module for directing, objects in an axial direction. Details of the sliding device and possible engagements with other objects such as devices that are guided by the sliding device, e.g. a holding system for an object, are described herein. Also provided as further aspect is a system comprising the sliding device and the engaged object that is axially directed by the sliding device.

### Adjustment of the concentration of the biological compartments

According to one embodiment, the method according to the first aspect comprises adjusting the concentration of biological compartments prior to step (a) or adjusting the number of biological compartments within the provided aliquots by choosing the number of aliquots (n) that are generated from the plurality of cells.

As described herein, the plurality of biological compartments may be separated into aliquots by transferring portions of the plurality of biological compartments, such as cells, into individual containments, such as e.g. wells of a well plate, such as a 96 well plate of 384 well plate. In scope of the SPLiT-seq and other split-and-pool workflows providing an adequate cell number per containment is important, since the limited combinatorial space for barcoding in the SPLiT-seq workflow means that not as many cells as desired may pass through the workflow in order to avoid barcode collisions. Advantageously, the knowledge of the cell number/concentration obtained in the cell counting step, if performed, can be used to ensure that in relation to the used barcode labels, an acceptable number of biological compartments are provided in the aliquots. The interaction of the cell counting module and the other modules (e.g., of the disclosed system) provides the possibility of counting biological compartments such as cells in a suspension fully automatically. This provides data on the cell count, which can influence the further protocol during the running protocol on the robotic system, e.g. by bringing the biological compartments to a certain desired concentration after determining the concentration. This module thus enables a "dynamic protocol" for the entire workflow.

Adjustment of the cell concentration prior to step (a) is only required, in case the cell concentration is not suitable for providing the aliquots, e.g. by transferring portions of the plurality of biological compartments into different containments (e.g. wells), whereby different aliquots can be provided. Accordingly, it is advantageously to provide an approximate equal and defined cell number per aliquot throughout the "split" process. This may be achieved by transferring an adjusted volume of the plurality of biological compartments (e.g., the cell suspension) into the containments. In case the volume of the cell suspension is too low or should be kept constant at a particular volume, the adjustment of the cell number/concentration may include diluting the plurality of cells. Such dilution is preferably performed in case the number of cells is high. In case the number of biological compartments is too low to fill the complete/desired number of containments with aliquots, the adjustment of the number and/or concentration of the biological compartments may include dividing the plurality of biological compartments into fewer aliquots (n) in step (a). The adjustment of the cell number/concentration, if necessary in view of the results of the cell counting steps, can be performed manually or automatically according to the present disclosure.

Hence, in one embodiment, the method comprises counting biological compartments and adjusting the concentration of biological compartments prior to step (a). The biological compartments are preferably provided in form of a suspension. E.g. the measured cell count may exceed the maximum number of cells that can pass through the workflow (e.g. due to a limited barcoding space). The cell suspension is then preferably diluted to process only the maximum number of cells that can be processed while ensuring unique labeling of the single cells.

Hence, according to one embodiment, the method comprises adjusting the biological compartment concentration prior to step (a). Adjusting may comprise adding a dilution solution to the plurality of biological compartments. This allows to lower the concentration of biological compartments in the suspension. The dilution solution may be an aqueous solution and may optionally corresponds to the liquid surrounding the plurality of biological compartments. Advantageously, the present method allows to automatically adjust the concentration of biological compartments.

According to one embodiment, the concentration adjustment is performed to lower the concentration of biological compartments by dilution to achieve that the concentration of biological compartments is within an acceptable range. The acceptable range is preferably predetermined, e.g. depending on the number of different barcode sequences used and/or depending on the number of split and pool cycles/rounds performed.

According to one embodiment, adjustment occurs after performing a cell counting step, preferably after performing the automated cell counting step described in detail above. It is referred to the above disclosure.

The adjustment of the concentration of the biological compartments can be performed in an automated manner. Preferably, the cell counting step and the concentration adjustment step are both performed in an automated manner. This is advantageous, because it reduces handling errors. In a preferred embodiment, adjustment of the concentration is performed in a dynamic manner by an automated system using the results of the cell counting step, which preferably, is also performed in an automated manner as described herein. In one embodiment, the automated system compares the concentration determined as a result of a cell counting step with a predetermined reference value or reference range. If the determined concentration is higher than the reference value or reference range, the automated system adds the required volume of a dilution solution to the plurality of biological compartments to dilute the plurality of biological compartments which are preferably provided in form, of a suspension. This allows to achieve that the plurality of biological compartments are provided in a concentration that is within the predetermined reference value or reference range. If the determined cell density is within the reference value of reference range, no adjustment is performed by the automated system.

According to a further embodiment, the number (n) of aliquots provided in step (a) are chosen using the results of the cell counting step, wherein preferably, the cell counting step is performed as described above. In on embodiment, the number (n) of aliquots is determined by an automated system using the results of the cell counting step. The automated system may compare the concentration determined as a result of a cell counting step with a predetermined reference value or reference range, wherein if the determined concentration is lower than the reference value or reference range, the automated system separates the plurality of biological compartments into fewer aliquots compared to as when the determined concentration meets the reference value of reference range.

Adjustment, such as a dilution, may be performed in an automated manner using an algorithm. This algorithm dilutes the provided cell suspension when it is measured during the protocol that the cell suspension provided exceeds a maximum value. In this case, the cell suspension is diluted before further processing.

Further options include a limitation to fewer containments (e.g. wells) by reducing the number of aliquots e.g. during reverse transcription and also ligations if the cell suspension has a low cell density. This will reduce process time, save reagents and possibly increase the cell recovery rate as the total surface area wetted by the cell suspension is reduced.

Furthermore, as described herein, fewer cells may be transferred for further processing, such as lysis if e.g. it is desired to sequence a limited number of cells in depth.

According to a preferred embodiment, the cell counting module and/or the concentration adjustment module can be implemented into an automated robotic platform. The modules may have an electronic interface by which it can be connected to a processing unit. Moreover, the device may be controlled by an algorithm for automating the method steps. The module(s) may be controlled by an algorithm, computer program or sequence of operation instructions in any suitable form, e.g. any suitable programming language.

### Removing at least a portion of aggregates by passing the plurality of biological compartments through a sieving module

According to one embodiment, the method according to the first aspect comprises removing aggregates of biological compartments, preferably by passing a suspension of biological compartments through a sieving module.

The removal of the aggregates fulfills one of more of the following characteristics:
- it is performed in an automated manner;
- it is performed after pooling step (c) and/or after fixing the biological compartments.

Suitable and advantageous embodiments are described in further detail.

There are several instances, wherein aggregates of biological compartments may be formed, e.g. after performing fixation and permeabilization steps. Furthermore, such aggregates may be present after the pooling steps. The plurality of biological compartments (e.g. present in a pool) may comprise aggregates of biological compartments (e.g. multiple cells or nuclei which are associated). Removal of aggregates improves the efficiency of the subsequent barcode-labeling step. Otherwise, the RNA of the biological compartments in the aggregate would be labeled by the same barcode. Thus, the single biological compartments (e.g., cells/nuclei) within the aggregate cannot be differentiated. According to the prior art, aggregates are removed by manual procedures, which typically require use of a cell sieve. These procedures tend to cause spillage and loss of sample. Details are disclosed in the prior art documents referred to herein.

The present disclosure allows to avoid the drawbacks of the manual prior art methods. Therefore, the present disclosure also provides a novel sieving module. The sieving module provides a further aspect of the present invention. The sieving module can be advantageously implemented into the automated system according to the present disclosure or into prior art automated platforms, preferably without the need for additional specialized devices.

### The sieving module

According to the present disclosure a sieving module and sieving systems suitable for removing aggregates from a plurality of biological compartments are provided. The sieving module disclosed herein has the advantage, that the plurality of biological compartments, which pass the sieving module are laterally directed in their path towards a lateral outlet. The sieve (also referred to as sieving element) comprised in the sieving module is chosen such that it is capable of removing aggregates of the biological compartments of interest (e.g. cells or cell nuclei). The sieving module may be loaded by using any means, preferably by using a pipette or a pipetting module when using an automated system. To improve the passage of the fluid comprising the biological compartments through the sieving element, the pipette tip may be tightly pushed or pressed against the sieving element, thereby however, avoiding damage of the sieving element. Upon dispension from the pipette tip, the fluid comprising the biological compartments are directly pushed against the mesh and flowing through of the biological compartments is supported. Thus, in one embodiment, the sieving element is accessible for a pipette tip, e.g. the pipette tip of a pipetting module that is used in an automated system.

The sieving module allows to direct the sieved suspension comprising singularized biological compartments to a lateral position, e.g. into an adjacent reservoir (e.g. well of a well plate). This has the advantage that the sieving module does not need to be removed in order to access the sieved suspension comprising the biological compartments (which typically has to be done according to prior art cell sieves). Moreover, this has the advantage that no specialized equipment is required to remove the sieve. The sieving module allows to sieve the plurality of biological compartments and direct the sieved suspension to a lateral position. The lateral position is then advantageously accessible by a pipetting module. Therefore, the pipetting module can access and retrieve the sieved suspension from the adjacent well. Therefore, the sieving module is a device, which does not require specialized equipment to be implemented into an automated pipetting platform. Furthermore, the sieving module allows to further automate the method according to the present disclosure in a straight-forward manner by including the sieving module for automated removal of aggregates of biological compartments.

The mechanism underlying the sieving module described herein may furthermore be advantageously applied for other liquid handling operations as the advantages are compelling for automated systems.

To be able to use existing consumables, a holder is herein disclosed for holding a receptacle, said holder being configured to laterally direct a fluid, e.g. comprising a plurality of biological compartments, passing the receptacle. This has the advantage that a receptacle can be held, wherein material can be inserted (e.g. a cell suspension), processed and then directed towards a lateral position, such as an adjacent well. As a result the pipetting robot can directly access the lateral position and perform pipetting operations - without the need for further devices. The holder may comprise a receptable for receiving the receptacle.

### Single part/ multiple components of sieving module

According to one embodiment, the sieving module is provided as a single part, i.e. a one-piece element. Alternatively the sieving module may be provided by two or more parts. It may comprise or be assembled from a number of parts which can be provided in an assembled state or can be assembled by the user.

According to one embodiment, the receptacle comprising the sieve and optionally further components here disclosed components may be provided as a single part (e.g., as a consumable). According to one embodiment, the sieving module comprising the different disclosed components are provided separately or partially assembled. This has the advantage, that the user can flexibly change the components of the sieving module, e.g. for separating the sieve or sealing. According to another embodiment, the sieving module is provided as one part, e.g. in form of a consumable, wherein the user can easily exchange the sieve module. According to one embodiment, the sieving module may partially comprise a consumable. For instance, the receptacle comprising the sieve and optionally the sealing/sieve holder(s) may be a consumable, while the holder is reusable.

Moreover, the sieving module may be provided with further components in form of a system. For instance, the disclosed sieving module may be provided in a system with one or more containments, preferably an array of containments. In a particular embodiment, the sieving module is provided with a well plate, wherein a well may hold the sieving module while a neighboring well may provide a lateral position, wherein the plurality of biological compartments can be directed. As noted above it is advantageous that the sieved fluid can be directed by the sieving module into a neighboring well as it simplifies the access to the sieved fluid. This is particularly advantageous for use in automated systems. Further details of the sieving module are also described below.

The fluid path can be a path for fluid having at least two portions which extend substantially in the same direction such that especially the two portions can be accessed by one robotic pipetting module without transfer or movement of the sieving module. Therefore, the fluid path comprises a connection portion which extends laterally to the two portions.

The sieving module may comprise a receptacle comprising two openings and a sieve, wherein the sieve is provided between the two openings (or at the lateral outlet opening), wherein the openings are configured such that the fluid, e.g. comprising the plurality of biological compartments in singularized form, can pass the openings. Undesired aggregates are retained by the sieve. Furthermore, the sieving module may comprise a holder configured to laterally direct the plurality of biological compartments passing the receptacle outlet.

According to a preferred embodiment, the sieving module can be implemented into an automated platform. Advantageously, the sieving module does not require specialized robotic equipment in scope of an automated platform. In particular, a pipetting module of an automated platform is sufficient to remove aggregates using a sieving module disclosed herein. Therefore, sieving of biological compartments can be advantageously automated.

### Receptacle of sieving module

According to one embodiment, a receptacle is provided which can have various forms or geometries. The receptacle may have a size ranging from a millimeter up to dozens of centimeters. According to a particular embodiment, the receptacle may have a size of multiple centimeters (e.g., 1, 2, 3, 4, 5, 6, 7, 8, 9, 10, 11, 12, 13, 14, 15, 16, 17, 18, 19, or 20 cm). The inner dimensions of the receptacle may not be limited, as long as a plurality of biological compartments can generally pass the receptacle. According to one embodiment, the receptacle has a cylindrical shape. According to one embodiment, the receptacle is an empty spin column.

The receptacle may comprise an inlet and an outlet. The inlet may advantageously be configured to enable direct addition of the plurality of biological compartments in a suspension into the sieving module. Therefore, the inlet may have a diameter which is sufficient to enable a plurality of biological compartments flow or preferably a pipette tip pass and eject the plurality of biological compartments. For instance, a pipette comprising a tip capable of holding a plurality of biological compartments in form of a suspension in the range of 1 µL to 10 mL may at least partially enter through the inlet. Afterwards, the pipette may eject all or some of the plurality of biological compartments, which is then present inside the receptacle. The inlet may preferably be open but can also be closed or closable if desired.

The receptacle outlet may have the same or different size as the inlet. Preferably the receptacle outlet is smaller in diameter than the inlet. Therefore, advantageously the plurality of biological compartments passing the receptacle outlet (e.g. the sieved plurality of biological compartments) are focused to a narrower section. However, the receptacle outlet may not be as small as it would represent a significant fluid resistance, which would reduce the flow of the plurality of biological compartments (e.g., to such an extent that some kind of generation of a pressure differential would be required).

According to one embodiment, wherein the sieving module does not comprise a separate holder, the receptacle may comprise means for directing the plurality of biological compartments laterally (preferably after sieving the plurality of biological compartments). Therefore, the receptacle may comprise a passage and a lateral outlet as disclosed herein.

### Sealing and sieve holders

According to one embodiment, the receptacle comprises an inlet and a receptacle outlet and a sieve, wherein the sieve is provided between the two openings, wherein the first opening and the second opening are configured such that a plurality of biological compartments can pass the openings. According to one embodiment, the receptacle is configured to hold a sieve such that the plurality of biological compartments added through the second opening can only pass the receptacle by passing the sieve. Therefore, the sieve is preferably held by one or more sealing/sieve holders. For instance, the sieve may be provided with one or more sealing/sieve holder which hold the sieve and furthermore, seal the circumference of the sieve in order to prevent leakage of the plurality of biological compartments around the sieve. Therefore, the plurality of biological compartments predominantly pass the sieve, unless the biological compartments are aggregated such that the compartments cannot pass the sieve (e.g. too large to pass the sieve). Preferably, the receptacle is configured to hold the sieve by providing a sieve between two sealing/sieve holders which are inserted into the receptacle.

The sealing/sieve holders may have any kind of geometry and size, as long as the sealing/sieving holder facilitate sealing of the sieving module such that the predominant plurality of biological compartments is sieved. The sealing/sieve holders may have an outer circumference which matches the inner circumference of the receptacle. Moreover, the sealing/sieve holders may preferably comprise one or more central openings, which enable the plurality of biological compartments to contact the sieve of the sieving module. According to one embodiment, the sealing/sieve holders have approximately a ring-like shape. According to one embodiment, the ring-like shaped sealing/sieve holders have a flat contact surface, wherein the flat contact surface preferably is directed to the sieve in the assembled state. According to a particular embodiment, two sealing/sieve holders with a ring-shape having a flat contact surface directed to the sieve are provided. In such a particular embodiment, the receptacle preferably is an empty column.

### Sieve

According to one embodiment, the sieve has a mean mesh size, which is larger than the size of the plurality of biological compartments and smaller than the aggregates. According to one embodiment, the mean mesh size of the sieve is selected from the range of 5 µm to 200 µm, preferably is the range of 10 to 150 µm, 20 to 100 µm or 30 to 80 µm. According to a particular embodiment, the mesh size of the sieve is approximately 40 µm. According to one embodiment, the mesh size is selected from the group consisting of 10 µm, 15 µm, 20 µm, 25 µm, 30 µm, 35 µm, 40 µm, 45 µm, 50 µm, 55 µm, 60 µm, 65 µm, 70 µm, 75 µm, 80 µm, and 100 µm, preferably 30 µm, 35 µm, 40 µm, 45 µm, 50 µm, more preferably 40 µm.

The sieve may me made of nylon or PET.

### The holder in general

According to the present disclosure, the sieving module may comprise a holder configured to laterally direct a fluid, e.g. comprising the plurality of biological compartments. The holder may receive a receptacle comprising a sieving element. This embodiment allows to use e.g. available consumables as receptacles that may be placed into the holder which is provided as a further aspect of the present disclosure. Fluid exiting the receptacle is received by the holder and laterally directed as described herein. The holder may advantageously provide a contact surface for contacting a portion of the receptacle to thereby, hold the receptacle. For instance, the holder may comprise a cylindrical opening and a narrowing section configured to engage with the bottom portion of the receptacle, wherein the receptacle outlet is not directly contacted (although partial contact is within the scope of the present disclosure). Advantageously, the holder may be configured to be secured to a location adjacent to the containment (e.g. well) into which the sieved fluid is laterally directed by the holder. The present disclosure is not limited to particular geometrical configurations. It is however preferred to stably hold the receptacle.

### The passage for lateral direction

A holder is provided for holding a receptacle configured to laterally direct a fluid, preferably comprising a plurality of biological compartments passing the receptacle. According to one embodiment, the holder comprises a passage configured to laterally direct a fluid, e.g. comprising a plurality of biological compartments, passing the receptacle, wherein preferably laterally a containment is provided, such as a well or other reservoir.

According to the present disclosure, the sieving module may comprise a holder for laterally directing the plurality of biological compartments. Therefore, the holder may comprise a passage below the receptacle outlet. According to a preferred embodiment, some distance is provided between the receptacle outlet and the holder in order to allow a plurality of biological compartments to flow onto the passage. The distance thus advantageously allows the plurality of biological compartments to pass the receptacle outlet (e.g. the plurality of biological compartments which have passed the sieve can flow out of the receptacle). The distance may be selected from the range of 0.1 mm to multiple centimeters. According to one embodiment, part of the receptacle outlet is directly in contact with the passage such that at least part of the receptacle outlet is not in contact with the passage (e.g., allowing the plurality of biological compartments to pass into the passage).

Moreover, the holder can advantageously direct a fluid, e.g. comprising the plurality of biological compartments, laterally. This has the advantage, that the sieved fluid does not vertically pass (e.g. out of the vertically arranged receptacle inlet and out), as this would necessitate removal of the sieving module in order to access the sieved plurality of biological compartments (e.g. by the pipetting module). In contrast, laterally directing the sieved fluid, preferably comprising a plurality of biological compartments as described herein, facilitates straight-forward automation. For lateral direction, the holder may comprise a passage that directs the sieved fluid into an adjacent containment or reservoir. The holder may advantageously comprise a passage configured such that the plurality of biological compartments which pass the receptacle outlet are directed by the passage towards a lateral position, wherein the lateral position is preferably a lateral containment.

### Lateral outlet

Preferably, the plurality of biological compartments passes the sieving module according to the present disclosure and is then laterally directed via the sieving module to a lateral outlet. The lateral outlet may be provided by the holder (e.g., the holder comprises a lateral outlet) or the sieving module, if provided without a holder, comprises a lateral outlet. Therefore, the sieving module may comprise a passage, preferably below the sieve, that allows to laterally direct the plurality of biological compartments towards a lateral outlet. The lateral outlet may have any kind of geometry. In case a passage is provided, the lateral outlet may geometrically be substantially equal to the circumference of the passage. In certain embodiments, the lateral outlet comprises a small recess towards the lateral position in order to prevent the plurality of biological compartments from contacting the outer walls of the sieving module.

### The passage

The particular passage geometry, size and tilting angle of the passage of the holder may not be limited according to the present disclosure, as long as a fluid, e.g. comprising a plurality of biological compartments, is directed laterally. In order to laterally direct the plurality of biological compartments, the passage may be tilted, e.g. with regard to the sieve. This allows to simplify the flow of the sieved fluid into a neighboring reservoir, such as an adjacent well. The tiling angle, e.g. between the passage and the sieve, may be selected from an angle above 0° and 90° or less than 90°. Preferably the tilting angle may be selected from 5°, 10°, 15°, 20°, 25°, 30°, 35°, 40°, 45°, 50°, 55°, 60°, 65°, 70°, 75°, 80°, or 85°, preferably 25°, 30°, 35°, 40°, 45°, 50°, 55°, 60°, 65°. The passage may have a geometry selected from ellipsoidal, round, rectangular or irregular shapes. According to one embodiment, the passage has the shape of a channel, wherein the channel is at least partially or completely surrounded. According to a preferred embodiment, the passage corresponds to a groove, wherein the groove can have various geometries. According to a particular embodiment, the passage corresponding to a groove may have a half ellipsoidal surface area for laterally directing the plurality of biological compartments without leakage. Other geometries are within the scope of the present disclosure, including box-like shapes or irregular shapes.

The tilting angle may be chosen such to support a good dripping of the fluid, e.g. to ensure that the fluid is transferred into the lateral reservoir such as an adjacent well. The angle may be e.g. at least 45° to ensure a good flow out of the sieved fluid. Other configurations are also feasible.

### Recess of the holder to engage with well plate

According to one embodiment, the holder comprises a recess for holding or arranging the holder on top of a containment. The holder according to the present disclosure may comprise a widened bottom section in order to provide a more stable stand. However, other options to improve stability are applicable in scope of the present disclosure. According to a particular embodiment, the holder comprises a recess, which is configured to contact the top of a containment (e.g. the containment of a well plate, for instance, a round or rectangular well plate format).

According to one embodiment, the receptacle or the holder comprises a pedestal being adapted to fit to a containment, preferably a well plate, such that the receptacle or the holder can be positioned on the containment and the fluid which passes the sieve is directed laterally, preferably into a lateral containment. The pedestal may have any kind of geometry. According to an exemplary embodiment, the pedestal is a rim around the holder or receptacle that is larger than the containment it is desired to stand on. Accordingly, part of the holder or receptacle may reach into the containment.

Furthermore, a part of the holder may be inserted into the containment in order to improve the stability.

According to one embodiment, the sieving module is provided as a consumable which comprises a corresponding recess or pedestal for arranging the sieving module on top of a containment, such as the well of a well plate.

### Consumable

According to one embodiment, the receptacle comprising the sieve and optionally further components, such as the herein disclosed components, may be provided as a single part (e.g., as a consumable). E.g. a receptacle may be provided that comprises a sieving element and is capable of laterally directing a fluid as described herein. In such embodiments, a holder is not required to laterally direct the fluid as this function is provided directly by the receptacle. However, using a holder as described herein in combination with a receptacle, which may be a conventional receptacle, has advantages as described herein. It in particular allows to use conventional receptacles, while the holder allows to laterally direct the sieved fluid towards a lateral reservoir or containment, such as a well of a well plate. The holder may be reusable and may also be used with different receptacles (e.g. comprising different sieves). This is advantageous as it provides flexibility and renders conventional receptacles better suitable for use in automated systems.

According to one embodiment, the sieving module comprising the different disclosed components are provided separately or partially assembled. This has the advantage, that the user can flexibly change the components of the sieving module, e.g. for separating the sieve or sealing. This allows e.g. to exchange the sieve element in order to insert a sieve element having the desired mesh size. According to another embodiment, the sieving module is provided as one part, e.g. in form of a consumable, wherein the user can easily exchange the sieve module. According to one embodiment, the sieving module may partially comprise a consumable. For instance, the receptacle comprising the sieve and optionally the sealing/sieve holder(s) may be a consumable, while the holder for holding the receptacle is reusable.

The sieving module is highly advantageous when being used in conjunction with the present method according to the first aspect. Throughout cell processing protocols aggregates may be formed. It is disclosed in scope of the present disclosure to remove aggregates of biological compartments. Otherwise the efficiency of the barcode-labeling of RNA will be reduced over the course of the split-and-pool workflow. This challenge is solved by the present disclosure by providing a sieving module. Therefore, the sieving module may comprise clamping a sieve (e.g. with a mesh size of 40 µm, further mesh sizes were described above) between two sealing/sieve holders. The sealing/sieve holders may be two rings, which are combined with the sieve (e.g., by providing a ring to each side of the sieve). The sieve having one sealing/sieve holder at each side may be combined with a receptacle, which preferably comprises a hollow, elongated body. The receptacle may have the form of an empty spin column. The sieving module may further comprise a holder, which is configured to hold a column and can direct a plurality of biological compartments flowing through laterally into an adjacent containment (e.g. well of a well plate).

Conventionally, these sieves are used in such a way that the plurality of biological compartments flowing through is always collected underneath the sieve. Yet, in automated system, such as robotic pipetting platforms, this means that the sieve must be removed before the pipette tip can further process the underlying plurality of biological compartments that have been sieved. Such procedures require specialized robotic configurations in order to perform them automatically. Thus, these steps are oftentimes performed manually, leading to additional costs for personnel, e.g. who need to remove the sieve in order to provide access to the sieved fluid. Moreover, the workflow may be delayed in case the removal of the sieve does not immediately take place. As a result, the performance of the workflow may be reduced.

The present disclosure advantageously avoids the prior art drawbacks by providing an advantageous sieving module. Details of the sieving module were already described above. According to a preferred embodiment, the sieving module comprises a holder comprising a channel which can direct a fluid, preferably comprising the plurality of biological compartments (e.g. cell suspension) via lateral flow into a lateral containment. For instance, the plurality of biological compartments may be directed to a neighboring containment, such as an empty well. Therefore, advantageously, it is possible for a pipette to process the plurality of biological compartments immediately without having to remove the sieve itself. The pipette can directly access the sieved fluid that was transferred laterally into a neighboring reservoir. In particular, a robotic pipetting module can directly take up the plurality of biological compartments (e.g. sieved cell suspension) without the need to first remove the sieving module. Hence, advantageously the process of sieving can be performed automatically by the sieving module of the present disclosure. The sieving module according to the present disclosure thus avoids specialized equipment of the automated platform for removing the sieving module in order to access a plurality of biological compartments below (such as the prior art) and is shown in **Fig. 15****.**

In Fig. 15 a photograph of the receptacle in form of a hollow, elongated body is depicted. Moreover, the sieve and the sealing/sieve holders are depicted, which can be stacked and inserted into the hollow, elongated body. The components may not be limited to the particular shown components and configurations and other components and configurations are readily available to the skilled person throughout the present disclosure. According to a particular embodiment, the sealing/sieve holders are ring-shaped and approximately have an outer circumference at the inner circumference of the receptacle. The receptacle may have the shape of a hollow, elongated body and may be a column (as described according to one embodiment of the present disclosure). Therefore, two sealing/sieving holders may hold the sieve in between, wherein this assembly is then inserted into the hollow, elongated body. Moreover, the hollow, elongated body comprises two openings, wherein the sieve is provided between the two openings. Preferably, the sealing/sieve holders may have a contact surface onto which the assembly of sealing/sieving holders and the membrane in between can be put. As a result, said assembly may be stably arrested inside the hollow, elongated body. (e.g., to prevent the sieve from clogging the first opening). Moreover, the openings are configured such that a plurality of biological compartments can pass the openings.

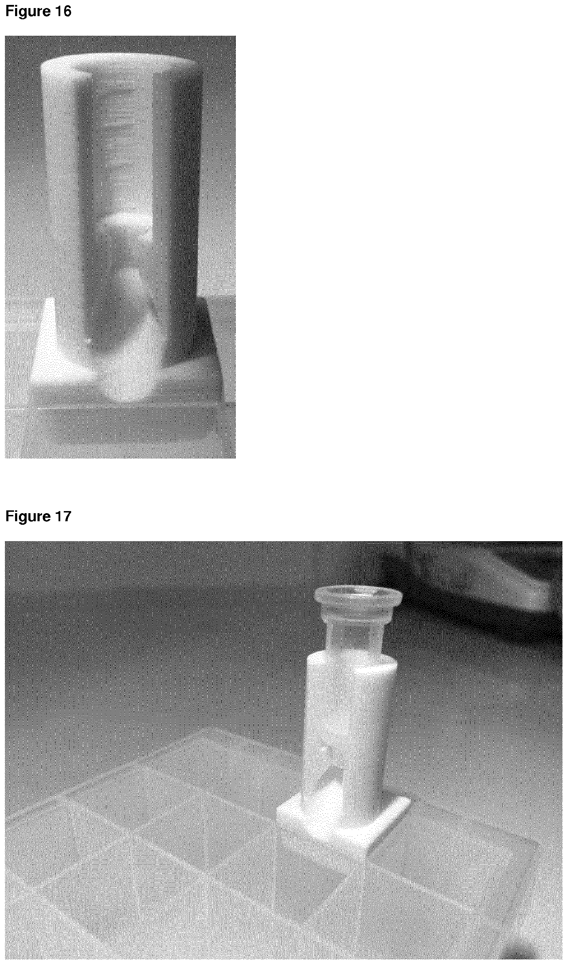

In **Fig. 16** an embodiment of the holder is photographically shown. The holder is capable of receiving and holding the receptacle comprising the sieve. The holder may not be limited to the particular shown configuration and other configurations are readily available to the skilled person throughout the present disclosure. In **Fig. 16** a holder is shown, which is configured to laterally direct the plurality of biological compartments passing the lateral outlet of a receptacle (e.g., hollow, elongated body). The holder that is shown as exemplary embodiment in **Fig. 16** is capable of receiving and holding the receptacle shown in **Fig. 15****.** The lateral direction may be achieved by a passage, which can be closed or open. Depicted is an open passage, wherein the flow of the plurality of biological compartments can be directly observed. As shown in the photography (see also **Fig. 17**), the holder is preferably provided inside a containment (e.g. a well providing a reservoir), wherein laterally another containment is present. Other modes are readily available (e.g. the holder may be clamped), but advantageously at a lateral position a containment may be provided. The holder may furthermore comprise an opening, into which the receptacle can be inserted. Inside the opening, a contact surface may be provided for contacting a portion of the receptacle in order to hold the receptacle. Thereby, a tight and secure fitting of the receptacle inside the holder can be achieved.

**Fig. 17** shows a photography of an embodiment of the sieving module according to the present disclosure. The sieving module is provided by a holder which comprises a receptacle that is assembled with the holder. As shown in the photography, a receptacle which is a hollow, elongated body is held by a holder. The holder may be provided in contact with a containment (e.g. well plate, well plate having rectangular well openings). The holder according to the Figure comprises a passage below the first opening of the held hollow, elongated body. The fluid exiting this opening of the receptacle is directed to the passage. As disclosed herein, the fluid may comprise biological compartments that were sieved. The passage can advantageously direct a plurality of biological compartments passing the receptacle towards a lateral outlet. Lateral direction may comprise directing the biological compartments into a containment which is laterally positioned. This is illustrated in **Fig. 17****.** The passage directs the fluid to the adjacent containment (here a reservoir provided by the adjacent well). In embodiments, the passage is provided in an elongated form which extends over or into the adjacent containment. Providing an extended elongated passage would e.g. allow to direct the sieved fluid into the center of the adjacent well. **Fig. 17** shows the receptacle of **Fig. 15** assembled to the holder of **Fig. 16****,** whereby the sieving module is provided, the sieving module being shown assembled onto a well-plate. The holder comprises a pedestal which allows a tight fighting of the holder onto the containment. As is illustrated, the holder may (e.g. with the pedestal) extend partially into the containment to provide a tight and stable fighting (see also **Fig. 16**). The fitting may be fluid-tight. The sieving module may not be limited to the particular shown configuration of **Fig. 17** and other configurations are readily available to the skilled person throughout the present disclosure.

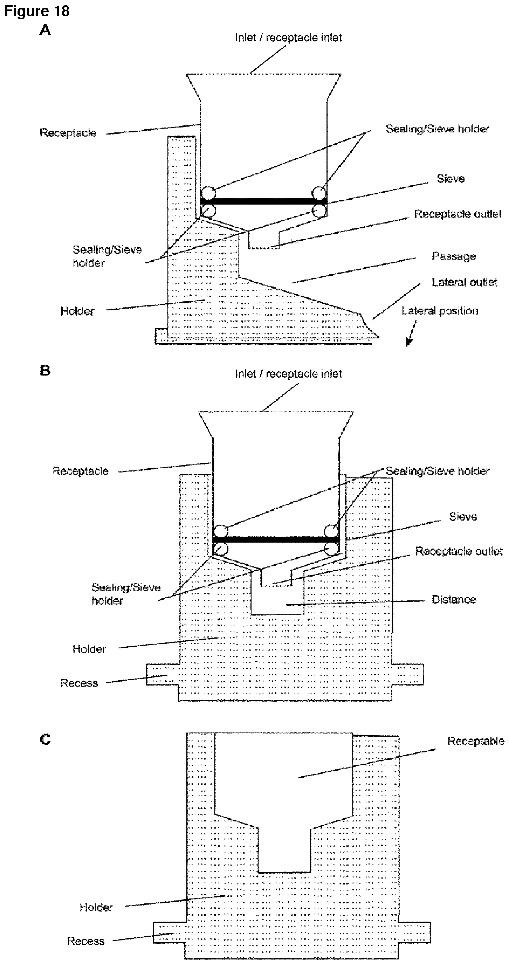

In **Fig. 18****,** cross sections of the sieving module according to one embodiment is schematically illustrated. In **Fig. 18** **A,** a longitudinal cross-section is illustrated and in **Fig. 18** **B** the transverse to the longitudinal cross section is depicted. The particular components of the Figure are described in the present disclosure and it is here referred thereto. The sieving module is not limited to the particular illustrated embodiment (i.e. not limited to the Figure). In **Fig. 18** **A**, a lateral passage is depicted, wherein the lateral passage is open as disclosed herein. Therefore, the plurality of biological compartments passing the sieve may be laterally directed by the passage towards a lateral position, wherein at the lateral position preferably a containment is provided. The transverse to the longitudinal cross section **(****Fig. 18 B)** illustrates furthermore a distance between the receptacle outlet and the holder in order to leave space in between the receptacle outlet and the holder for the plurality of biological compartments to flow out of the receptacle outlet and being laterally directed towards the lateral outlet. **Fig. 18** **C** illustrates an embodiment of the receptable of the holder configured for receiving a receptacle.

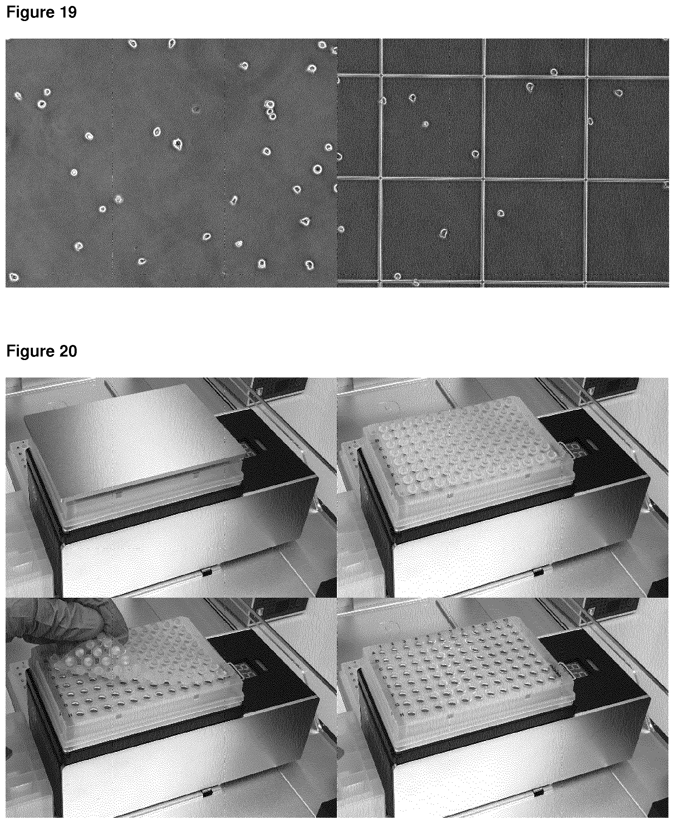

**Fig. 19** shows microscopic images of a plurality of biological compartments (e.g., cells). The exemplified images show a control of Jurkat cells (left) compared to cells that have passed a 40 µm sieve (right). Optically, there is no difference of the cells by the sieving process. Moreover, no cell aggregates are visible after sieving of the cells (right picture).

### The sieving module

The sieving module according to the present disclosure has been described above. Important characteristics and embodiments are further described below.

The present disclosure provides a sieving module, preferably for removing objects, such as aggregates from a plurality of biological compartments, the sieving module comprising:
- an inlet and a lateral outlet of a fluid path,
- and a sieve, wherein the sieve is provided in the fluid path;
   - wherein the inlet and the lateral outlet are configured such that a fluid, e.g. comprising a plurality of biological compartments, can pass the inlet and lateral outlet; and
   - wherein the lateral outlet is directed such that the fluid which passed the sieve is laterally directed with regard to the inlet.

This sieving module represents a further aspect of the present invention. It may be advantageously used in conjunction with the method according to the present invention. Important advantages were already described above.

Further features are described in the following. One or more of the following characteristics may be fulfilled.

According to one embodiment, the inlet is provided by a receptacle, wherein the receptacle comprises preferably a hollow, elongated body.

The lateral outlet may also be provided by the receptacle.

In embodiments, the lateral outlet is provided by a holder which is adapted to hold the receptacle and establishing a fluid path between the receptacle and the holder.

A lateral outlet may be an outlet which confines an angle between the longitudinal axis and the outlet which is different from 0°, especially greater than 10°, greater than 20°, greater than 30°, greater than 40°, greater than 50°, greater than 60°, greater than 70° or greater than 80°.

In embodiments, the receptacle or the holder comprises a pedestal being adapted to fit to a well plate such that the receptacle or the holder can be positioned on the well plate and the fluid which passed the sieve can be directed into a well. As disclosed herein, the sieving module allows to laterally pass a sieved fluid to an adjacent well. Being located in an adjacent well, the sieved fluid is directly accessible to a transfer device, such as a pipette, which may thus easily aspirate and transfer the sieved fluid from the well for further use. This is an important advantage compared to conventional methods/systems, in which the sieved fluid is provided underneath the sieving module and therefore, cannot be directly accessed by a pipette without prior removal of the sieving module. Therefore, the advantageous sieving module is particularly suitable for use in automated methods and systems.

The sieving module can be implemented into an automated process. Advantageously, this can be done without particular devices being required of or for moving the sieving module. The sieved fluid is directly accessible upon lateral transfer to a neighboring containment such as an adjacent well or other type of reservoir.

In advantageous embodiments, the sieving module comprises a holder for laterally directing the plurality of biological compartments. As disclosed herein, the holder may comprise a receptacle as disclosed herein, which comprises a sieve. Advantageously, the sieving module may thus comprise a holder and a receptacle comprising a sieve.

The holder may comprise a passage configured such that the plurality of biological compartments, which pass the sieve, which is preferably located inside a receptacle, is directed by a passage towards a lateral direction, wherein in the lateral direction extends to a lateral outlet.

The receptacle may be configured to hold the sieve by providing a sieve between two sealing/sieve holders which are inserted into the receptacle, which is preferably an elongated, hollow body. The hollow, elongated body may be provided in form of a column.

Advantageously, the biological compartments which are directed laterally can be accessed by one robotic pipetting module without transfer or movement of the sieving module.

The sieve of the sieving module has a mean mesh size, which is larger than the size of the plurality of biological compartments and smaller than the aggregates. This allows to efficiently remove the undesired aggregates, while the biological compartments of interest are comprised in the sieved fluid (see e.g. Fig. 19). Suitable mesh sizes for the sieve were already disclosed above. In embodiments, the sieve has a mean mesh size of about 40 µm.

In embodiments, the sieving module is provided as one part, e.g. in form of a consumable such as a cartridge.

In embodiments, the sieving module comprises a receptacle with a sieve element (e.g. a hollow, elongated body such as a column comprising a sieve) and a holder adapted to receive the receptacle. The holder comprises a recess for holding the holder on top of a containment. The holder may furthermore comprise a pedestal. Suitable and preferred embodiments of the holder and the receptacle are described elsewhere herein.

### The holder

Also provided as one aspect of the present disclosure is a holder for holding a receptacle configured to laterally direct a plurality of biological compartments passing the receptacle. The holder was described in detail above and it is referred to the respective disclosure. The holder provides a further aspect of the present disclosure.

Further and preferred features are described in the following.

The holder comprises in a preferred embodiment a passage configured to laterally direct a fluid, e.g. comprising a plurality of biological compartments passing the receptacle. The passage may be tilted to support the flow of the fluid. Suitable tilting angles are described elsewhere. The holder may be assembled to or onto a containment, such as a well of a well plate. The holder may comprise a recess and/or a pedestal, in order to simplify the mounting of the holder to or onto the containment. A further containment is preferably provided laterally. This lateral containment is in embodiments adjacent to the containment with the assembled/mounted holder (see e.g. Figs. 16 and 17). The lateral containment (e.g. a well of a well plate) may receive the fluid that was laterally transferred by the holder. Details are described elsewhere.

The receptacle that can be introduced into the holder is preferably a hollow, elongated body. Details have been described above. The receptacle may be provided by a column. The column may comprise a sieve element. The sieve element allows the passage of the biological compartments of interest (e.g. cells, nuclei), while retaining undesired aggregates.

The holder may advantageously be used in order to improve the use of a receptacle (e.g. a column comprising a sieve element) within automated systems. The holder is configured to receive a receptacle (see e.g. **Figs. 16** and **17**). As disclosed herein, a fluid that exits the receptacle through an opening (e.g. the tip of a column) is received by the holder, preferably a lateral passage of the holder. The fluid flows through the lateral passage and may exit the passage/holder through the lateral opening. The lateral opening is positioned such that the fluid may be transferred to a neighboring containment (such as an adjacent well) where the fluid is advantageously directly accessible for a transfer device such as a pipet. The lateral opening may be provided at or near the side wall of the neighboring containment so that the laterally transferred fluid may flow along the side walls into the containment. The lateral passage may also extend to or into the neighboring containment so that the fluid exiting through the lateral opening of the holder is directed to the interior space of the containment.

### Method for sieving a fluid

Also provided is a method for sieving a fluid, preferably for removing aggregates from a suspension comprising a plurality of biological compartments using a sieving module or a system according to the present disclosure, comprising the steps of:
(a) adding a fluid, preferably comprising a plurality of biological compartments through an inlet, whereby the fluid passes the sieve, wherein larger objects, such as aggregates, do not pass the sieve; and
(b) laterally directing the sieved fluid, preferably comprising a plurality of biological compartments, through a lateral outlet, preferably into a containment located adjacently.

Details of the sieving method were already described above. This sieving method can be performed in the context of the method according to the present disclosure, e.g. in order to remove aggregates of the biological compartments of interest. In preferred embodiments, the sieving method is therefore performed as process step of the method according to the first aspect. The sieving method is advantageous, as it is effective (see e.g. also Fig. 19) and easy to automate. Therefore, the sieving method/step may be performed in an automated manner. It may be performed using the sieving module of the present disclosure. Details of the sieving module and associated advantages are described elsewhere in detail and it is referred to the respective disclosure which also applies here.

### The system

Also provided is a sieving system for sieving a fluid, preferably for removing aggregates from a plurality of biological compartments, the sieving system comprising:
- two openings of a fluid path, each opening being accessible from substantially the same direction such that especially the fluid path can be accessed by one robotic pipetting module without transfer or movement of the sieving module.

A sieving system for sieving a fluid, preferably for removing aggregates from a plurality of biological compartments, the sieving system comprising:
- a holder for holding a receptacle configured to laterally direct a fluid, preferably comprising a plurality of biological compartments, passing the receptacle; and
- a receptacle comprising a hollow body and a sieve located therein.

Details and advantageous embodiments of the holder and the receptacle are described elsewhere herein.

Further features are described in the following. One or more of the following characteristics may be fulfilled:
The system may further comprise one or more containments, preferably an array of containments, more preferably a well plate. The system may comprise an array of containments and wherein the holder is provided onto the array of containments, wherein the holder may either form a connection with a containment or is held by either means to be provided into or onto the array of containments. The system may comprise an array of containments and wherein the holder is located in/on one containment and wherein a further containment is arranged laterally. As disclosed herein, the holder may laterally direct the fluid (e.g. through a lateral passage) into the containment that may be provided adjacent to the containment with the holder. The laterally transferred fluid is thereby directly accessible for a transfer device such as a pipet. The sieving system may advantageously be incorporated into an automated system for performing a workflow that comprises one or more sieving steps. It may advantageously be used for performing a sieving step within the method according to the first aspect, wherein preferably, the sieving step/method is performed in an automated manner using a robotic system.

### Mixing step and mechanism

The method according to the first aspect may furthermore comprise performing one or more mixing steps, preferably using an automated mixing mechanism.

Such mixing steps can be performed prior, during or subsequent to several core steps of the present method, e.g. in order to mix reagent solutions with the plurality of biological compartments and/or in order to maintain the biological compartments in suspension. It is preferably performed when new reagents are added to containments such as reservoirs and/or to maintain the biological compartments in suspension, e.g. during splitting, in particular when the splitting process is performed for longer time periods.

As cells typically sediment over time, cells may be heterogeneously distributed throughout a cell suspension. In order to transfer an approximately equal cell number with each aliquot, it is advantageous to distribute the biological compartments homogeneously in the suspension. E.g. the plurality of cells may be mixed prior to performing step (a). Mixing may furthermore be performed after optionally adjusting the concentration of biological compartments. Mixing may be performed prior to each aliquoting/transfer step and/or after a defined number of transfer step (e.g. after 2, 3, 4, 5, 6, 7, 8, 9 or 10 transfer steps). A mixing mechanism can be advantageously performed automatically according to the present disclosure. Therefore, the homogeneity of the sample can be advantageously improved.

Different manual mixing operations are available and known by the person skilled in the art. However, automated mixing operations are limited to simple pipetting operations. As the available pipetting volume (e.g. in a range of 200-500µl such as 300µl) used for transferring aliquots of the plurality of biological compartments into containments is typically comparably low compared to the sample volume to be mixed (e.g. several ml), these conventional mixing operations are insufficient to mix the plurality of cells adequately. As a result, the barcode labeling efficiency may be reduced, as the number of biological compartments per containment may vary.

According to the present disclosure, an advantageous mixing operation is provided, that allows to use the pipette tip as mixing element in an automated manner. It allows to mix a large sample volume using a small pipette tip volume for mixing. The mixing operation of the present disclosure allows to sufficiently mix the sample, e.g. to homogenously mix the plurality of cells in a suspension. This supports that an approximately equal number of cells can be transferred with each aliquot.

The mixing operation may include contacting a mixing element such as the portion of the pipetting module (e.g. part of the pipette tip), with the sample, e.g. the suspension comprising the plurality of cells. Then the pipetting module may perform a movement, whereupon the contacting portion performs the movement inside the sample (e.g. inside the cell suspension). The particular movement may not be limiting. According to one embodiment, the movement comprises an ellipsoidal movement, which may include a circular movement. According to one embodiment, the movement follows approximately an "8".

The mixing may further include transferring a portion of the plurality of cells from one position inside the containment into another position of the same containment. According to one embodiment, a portion of the sample is transferred from position A to position B within the same containment. In particular, a certain volume (e.g. 100, 200, 300 µL, etc.) may be taken up by the pipetting module from position "A" inside the containment. Afterwards, the volume may be ejected by the pipetting module at position "B". This step may be performed multiple times, wherein the position "A" and "B" preferably vary. For instance, position A1 may be at the very upper center of the containment and position B1 at the upper border; position A2 may be at the lower border to the containment and position B2 may be at the lower center of the containment. The particular position of position A and B may not be limiting to the mixing step.

According to a preferred embodiment, both mixing steps (movement and transfer described above) are advantageously combined. In particular, first mixing may take place by movement of the pipetting module, followed by transfer of a portion of the sample from one position inside the containment into another position of the same containment. According to one embodiment, the combination of both mixing steps can be repeated multiple times. For instance, the steps may be performed sequentially, which is repeated one, two, three, four, five or more times.

According to a preferred embodiment, an automated mixing step is performed which preferably comprises the following substeps:
(i) a mixing element, preferably comprising a pipette tip, is contacted with the sample to be mixed, such as with the plurality of biological compartments which are preferably provided in form of a suspension, and performs an ellipsoidal movement; and
(ii) a portion of the sample is transferred from position A to position B within the same containment using the mixing element;
wherein substeps (i) and (ii) can be performed in any order and wherein preferably, substeps (i) and (ii) are repeated one or more times. Position A and B are preferably chosen differently during repetition. The ellipsoidal movement may include a circular or multi-circular movement, e.g. in form of an 8.

Hence, also provided is a solution to the problem to mix a large sample volume using a small pipetting volume by a combination of pipetting and the translation or rotation movement of the pipette tip. This movement can be performed by the automated system, while the tip is immersed in the respective liquid or suspension providing the sample to be mixed. The algorithm used by the automated system, described in words, is conceptually as follows:
1. move the pipette tip several times in a circle in the liquid
2. take fluid into the tip at one point, move the tip to another point and release the volume again
3. repeat basic steps 1 and 2 repeatedly, in combination and at different locations, in different ways, to produce the mixing or homogenization of the suspension.

The mixing step can be performed on an automated robotic platform. The mixing operation may be controlled by an algorithm, computer program or sequence of operation instructions in any suitable form, e.g. any suitable programming language, for automating the method steps.

The mixing operation can be further improved by adding further translation and rotation movements. For example, not only a rotational movement is advantageous, but also the repeated movement in form of an "8".

The mixing step can be performed at multiple points throughout the method according to the present disclosure. For instance, mixing can be carried out before transferring aliquots of the plurality of cells and/or during or after pooling of the aliquots in step (b).

### Use of a heating module, such as an automatically closable cycling module

During performing the method according to the first aspect, heating may be required. E.g. for performing a reverse transcription, typically heating is required. Heating causes evaporation of sample liquid, i.e. the liquid surrounding the plurality of biological compartments. Therefore, the containments (e.g. wells) comprising the biological compartments should be closed by a lid. However, such a closing step is challenging to automate.

The present disclosure advantageously allows to seal containments (e.g. wells) in an automated manner and therefore prevents evaporation. As a result, the present disclosure allows to heat the containments and allows to perform reactions at elevated temperatures, e.g. for performing a reverse transcription.

A method for heating containments, e.g. wells of a well-plate, the method comprising:
(a) subjecting the containments to a temperature controlled platform configured for holding the containments, wherein the containments are preferably provided as wells of a well plate;
(b) sealing the containments by adding a sealing cover, which is preferably a flexible mat; and
(c) heating the containments.

Wherein in step (b) axial and optionally vertical movements are performed in order to apply the sealing cover for sealing the containments.

The present disclosure provides a heating module, such as an automatically lockable heating module or a cycling module. The heating module, such as an automatically lockable heating module can be implemented into the system of the present disclosure. It provides a further aspect of the present disclosure.

A heating module, such as a cycling module, comprising
- a temperature controlled platform configured for holding containments, wherein the containments are preferably provided as wells of a well plate;
- a sealing cover configured so that it can be applied onto the containments for sealing the containments.

As described herein, such heating module is automatically lockable/closable.

Further features that may be provided alone or in combination are provided in the following:
- wherein the sealing cover is a flexible mat.
- wherein the heating module further comprises a cover plate.
- wherein the cover plate has one or more of the following features:
   - the cover plate holds the sealing cover, preferably a flexible mat;
   - the cover plate is a mechanically rigid plate;
   - the cover plate is a metal plate, preferably an aluminium plate; and/or
   - the cover plate comprises engagement means, wherein the engagement means are configured to engage with a portion of the pipetting module, preferably the pipetting tip;
- wherein the heating module further comprises components to perform axial and optionally vertical movements, wherein the components may have one or more of the following features:
   - one component corresponds to a device for performing an axial movement, preferably comprising a holding device/holder and a sliding device; and/or
   - one components corresponds to a device for performing a vertical movement;
- wherein the vertical movement is performed using the pipetting module which optionally comprises a pipetting tip;
- wherein a device for performing a vertical movement comprises a mechanical or electromechanical actuation, preferably a lifting magnet;
- wherein the heating module such as the automatically lockable heating module is controlled automatically;
- wherein the heating module such as the automatically lockable heating module is controlled in an automated manner, wherein in particular the temperature and the sealing of the containments by a sealing cover is controlled in an automated manner;
- wherein a control unit is provided which is adapted to control the temperature, the pipetting module, and/or a device for performing a vertical movement.
- wherein the control unit can be part of the device;
and/or
- wherein the control unit is a unit which is functionally connected to or part of the automatically lockable heating module.

Heating elements are used on the pipetting robot platform to adjust the temperature of samples comprised in containments (e.g. wells of a well plate). However, containments, which may be wells of a well plates, typically must be closed manually in order to prevent liquids from evaporating out of the wells. Such a step requires the experimenter to be present at the particular time point(s), causing additional personnel costs and potentially delay/stop of the workflow until the lid is added into the well plate.

The present disclosure advantageously avoids the drawbacks of the prior art. In particular, the disclosure provides an automatically lockable heating module. The automatically lockable heating module allows to fully automate the heating process without significant material loss due to evaporation. Therefore, the present disclosure allows to apply a sealing cover in an automated manner, which seals the containments, e.g. the wells of a well-plate.

The sealing cover can be applied to close the containments either by e.g. stepping or servo motors, or by using the pipetting module as described in the present disclosure in conjunction with the counting module. For instance, the pipetting module may be applied as a mechanical lever to move the sealing cover into the right position over the well-plate to be sealed the by the sealing cover. Therefore, a mechanism may be applied, corresponding to the sliding device described in conjunction with the cell counting module. E.g. the pipetting module may axially move the sealing cover to the right position. The heating module, in particular the automatically lockable heating module is therefore advantageous for automation, as it can automatically lock/close the containments (e.g., a well plate). Otherwise, either the fluid volume in wells would (partially) evaporate if the temperature was too high and/or the incubation time too long, or the interaction of a user would be necessary to close and open the containments (e.g. well plates). Furthermore, the risk of cross-contaminations is reduced.

The basis for this setup is, for example, realized in the following configuration: A temperature-controlled module on the deck of the platform carries a well plate, which is fitted precisely into an aluminium holder in order to optimize thermal conductivity. A sealing cover may be used to seal the individual wells on the plate when a mechanically rigid plate rests on it from above. The automation solution moves the cover plate including the flexible mat onto the wells.

According to the present disclosure, a heating module such as an automatically lockable heating module comprises a temperature controlled platform configured for holding containments, wherein the containments are preferably provided in form of a well plate and a sealing cover configured to be applied onto the containments for sealing the containments. As disclosed herein, protrusions provided on the sealing mat may extend into the containments such as the wells for tight sealing. The heating module such as the automatically lockable heating module may not be limited to a particular temperature controlled platform. Any kind of temperature controlled platform may be applied in conjunction with the presently disclosed heating module such as the automatically lockable heating module.

A heating platform based on air-cooled Peltier elements may be used. A temperature controlled platform, e.g. Opentrons TempDeck may be used which may be modified as described herein.

The heating module such as the automatically lockable heating module according to the present disclosure comprises a sealing cover configured to be added onto the containments for sealing the containments. Thereby, advantageously, liquid is prevented from evaporating out of the containments and into the surrounding atmosphere. Thus, the liquid remains predominantly inside the containments.

### Mechanism of adding the sealing cover

In order to provide the sealing cover configured to be added onto the containments for sealing the containments various methods may be followed according to the present disclosure. According to a particular embodiment, a similar configuration as the configuration of the counting module may be applied. Accordingly, the sealing cover may be provided on a holder (holding device), wherein the holder can be axially moved using a sliding device. In order to perform an axial movement, advantageously the pipetting module may be used. Accordingly, the holder may be moved such that the sealing cover is contacted with the containments to seal the containments. Therefore the holder/holding device, sliding device and further components described in conjunction with the counting module may advantageously be used for sealing the containments using a sealing cover. Details of the holding device (holder) and the sliding device were described above.

According to a particular embodiment, the heating module such as the automatically lockable heating module further comprises a sliding device for axially directing an object using a pipetting module, wherein the sliding device is configured to hold the sealing cover. Preferably the sliding device is configured for holding the sealing cover and a cover plate. According to a particular embodiment, the cover plate comprises engaging means configured for engaging with a part of the pipetting module. According to one embodiment, the cover plate comprises engagement means, wherein the engagement means are configured to engage with a portion of the pipetting module, preferably the pipetting tip. Engaging means and options for sliding devices have been described in conjunction with the counting module and the disclosures also apply here.

### Further vertical movement

According to one embodiment, a holding device/holder and sliding device may be applied (which have been described in the present disclosure) to perform an axial movement and transfer the sealing cover above the containments. Afterwards an additional vertical movement may be performed. Any means to perform the further vertical movement may be applicable in scope of the present disclosure. According to one embodiment, the holding device/holder may be moved by a pipetting module in axial direction. The holding device/holder may hold the sealing cover, wherein holding preferably comprises a sealing cover provided below the holding device/holder. Afterwards, means for vertically moving the sealing cover may be applied in order to put the sealing cover onto the containments. According to one embodiment, mechanical or electromechanical actuators may be applied in order to apply a defined force, e.g. in order to perform a vertical movement. Other means, such as a servo-motor may also be applied. According to one embodiment, a lifting magnet may be applied, wherein the lifting magnet may be controlled by the help of gravity and/or springs. The vertical movement is preferably performable bidirectionally. For instance, the sealing cover may be first axially moved above the containments, followed by vertical movement, sealing the containments. Then the holding device/holder can either be moved counter-vertically and counted-axially or may be held at the position throughout a heating operation. If the holding device/holder is held at the position, vertical movement may be performed when the temperature incubation is finished. Then the sealing cover may be moved vertically (e.g. upwards). This may be followed by axial movement (according to the described embodiments).

### Removal of the sealing cover

According to one embodiment, the sealing cover may be removed by any appropriate method known by the skilled person. According to one embodiment, the sealing cover is removed manually. According to a preferred embodiment, the sealing cover is removed automatically. In order to remove the sealing cover automatically various methods may be followed according to the present disclosure. As described before, the sealing cover may be vertically moved (e.g. lifted) in order to remove the sealing cover. Accordingly, the sealing cover may afterwards be axially moved. Axial movement may be achieved by any known means but in particular by the means described above (and described in conjunction with the counting module).

According to one embodiment, a cover plate is held by the holding device/holder, preferably at the bottom, wherein the sealing cover is attached to the cover plate. According to such an embodiment, the cover plate may be axially and vertically moved as described above, wherein a sealing cover is attached to the cover plate.

According to one embodiment, more than one heating module is provided or used in the method according to the first aspect. For instance, two, three or four heating modules may be provided or used. According to an exemplary embodiment, one heating module may be provided for the reverse transcription reaction according to embodiments of the method according to the first aspect of the present disclosure. Moreover, heating modules may be provided for every ligation reaction that is performed according to embodiments of the disclosed method. For instance, two ligation reactions may be performed, using two heating modules. According to a particular embodiment, three heating modules are provided. In order to automatically close/lock the containments, which may be provided on the temperature controlled platform of the heating module, it may be required to provide individual components for automatically closing the containments (i.e. one, two, three or four holders and sliding devices and flexible mats). According to another embodiment, one component may be sufficient for automatically closing/locking multiple containments present on different temperature controlled platforms of the heating module. For instance, one holder and sliding device according to the present disclosure may be configured such that the holder and sliding device is capable of closing the containments (e.g. well plates by a flexible mat) on two, three or four different temperature controlled platforms of the heating module. Different combinations of such automated closing/locking procedures are applicable in scope of the present disclosure.

### Cover plate

According to one embodiment, the sealing cover is in contact with a cover plate. The contact between the cover plate and the sealing cover may be permanent or reversible. In case of a reversible contact, the user may exchange the sealing cover before, during or after performing the method according to the present disclosure. Exchanging may advantageously facilitate prevention of any cross-contaminations. The sealing cover may therefore be washable or provided as a consumable, which the user can easily exchange.

According to one embodiment, the cover plate is a mechanically rigid plate. Mechanical rigidity may be advantageous to stably hold the sealing cover. Therefore, the cover plate may be composed of any material, as long as the material or material composition is mechanically rigid enough to hold the sealing cover. According to one embodiment, it is preferred to provide a mechanically rigid cover plate that facilitates the maintenance of the temperature adjusted by the temperature controlled platform. According to a particular embodiment, the cover plate is a metal plate, which may comprise aluminium.

According to one embodiment, the cover plate may additionally be temperature controlled. Therefore, the cover plate may support the temperature control of the temperature controlled platform in order to provide a homogeneous temperature between the platform and the cover plate.

### Sealing cover

According to a preferred embodiment, the sealing cover is a flexible mat. The flexible mat may be configured to seal the containments when contacting the containments. According to a preferred embodiment, the sealing cover contacts the containments such that liquid that evaporated inside the containment predominantly stays inside the containment. Therefore, a sealing cover may be configured such that the opening of the containment is covered by the sealing cover. Moreover, it may be advantageous to provide a flexible mat that covers also the circumference of the containment in order to prevent leakage of (evaporated) liquid at the edges of the containment. According to one embodiment, the sealing cover is flat or structured, wherein a structured sealing cover may improve sealing of the containments. According to a particular embodiment, the containments are arranged in form of a well plate (e.g. 24, 48, 96, 384 wells) and the sealing cover seals predominantly all of the containments. A sealing cover may be provided in form of a flexible mat that covers the containments arranged in form of a well plate. Therefore, the sealing cover may be provided in form of a flexible mat. The flexible mat may be flat or structured.

According to an exemplary embodiment also depicted in **Fig. 20****,** a metal cover plate may be used having a flexible mat reversible attached thereto. The cover plate holding the flexible mat may be moved according to the present disclosure (e.g. via an axial movement induced utilizing the pipetting module) in order to move the flexible mat onto the containments. According to the photography in **Fig. 20****,** the containments are arranged in form of a 96 well plate and the flexible mat is moved on top of the containments. The flexible mat according to the photography is structured such that small features (e.g. protrusions) are present that are partially inside the well plate when the flexible mat is provided on the containments. The flexible mat may afterwards be removed by a similar mechanism as the mechanism that moved the flexible mat onto the containments.

### Measures against loosing cells

During the whole workflow, there is a risk that biological compartments (e.g. cells or cell nuclei) are lost, e.g. due to adhesion to plastic parts (e.g. pipette tips or containment walls, such as the walls of the wells) or because they are not transferred (e.g. by pipetting) because they remain in fluidic residuals remaining in a containment such as a well.

The present disclosure also addresses these problems as described in further detail below.

### Addition of adhesion reducing compounds

The method may further comprise contacting the plurality of biological compartments with at least one adhesion reducing compound. This advantageously reduces the adhesion of cells to walls of plastics (pipette tips or walls of containment) or the membrane or cell sieve, thereby improving the cell retention rate. Moreover, the one or more adhesion reducing compounds improve uptake of fluid residues of the plurality of biological compartments which otherwise remain on the surface(s) of containments, such as wells of a well plate.

According to one embodiment, at least one adhesion reducing compound is in contact with the plurality of cells during washing and/or fluid exchange. For example, at least one adhesion reducing compound may be added to the plurality of biological compartments (e.g. a cell suspension). The addition of the at least one adhesion reducing compound may take place before, after or during one or more of the steps of the disclosed method. The at least one adhesion reducing compound may be incorporated into one or more of the reagents that are contacted with the plurality of biological compartments. Preferably, at least one adhesion reducing compound is present when the plurality of biological compartments (such as cells or cell nuclei) are present in a solution.

In a preferred embodiment, the adhesion reducing compound is a detergent, more preferably a non-ionic detergent. The detergent may be selected from the group consisting of polyoxyethylene alkylphenyl ethers, polyoxyethylene-polyoxypropylene block copolymers and polyoxyethylene fatty alcohol ethers, and optionally is selected from polyoxamers, such as poloxamer 407 (Pluronic F127) and polyoxyethylene alkylphenyl ethers, such as Triton X100. According to one embodiment, the at least one adhesion reducing compound is Pluronic F127 and/or Triton X100. Other adhesion reducing compounds are readily available to the person skilled in the art and can be advantageously used here. The present disclosure is not limited to these specific adhesion reducing compounds and alternatives may be identified by the skilled person in view of the disclosure provided herein.

The at least one adhesion reducing compound, which preferably is a non-ionic detergent may be provided e.g. at a final concentration of at least 5mg/L, at least 10mg/L, at least 15mg/L or at least 20mg/L when being in contact with the biological compartments.

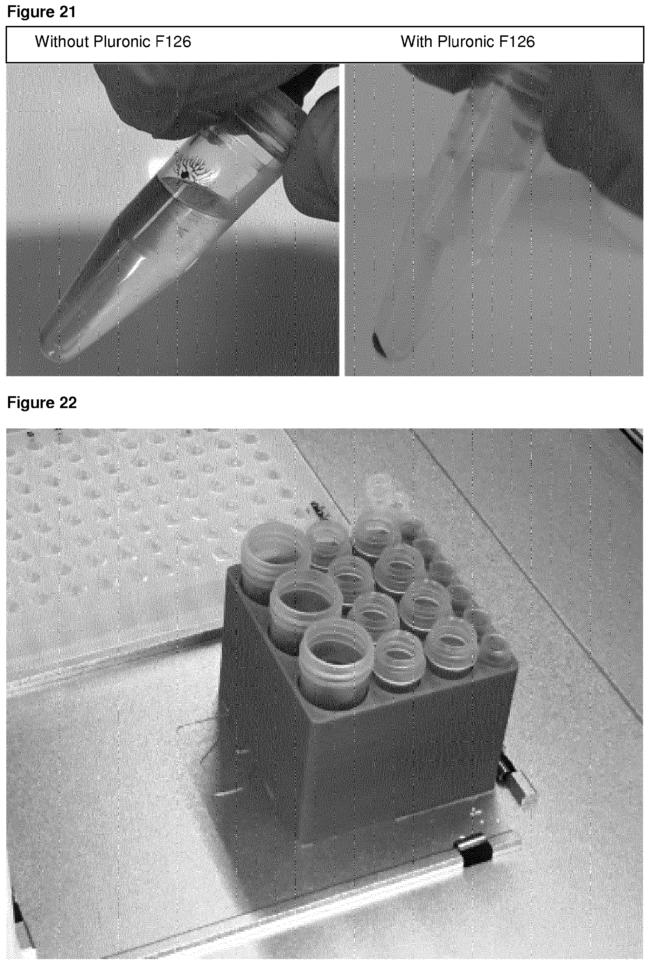

To visualize the effect of including at least one adhesion reducing compound, visible particles (so-called polybeads) were added to a buffer that simulates the chemical situation during reverse transcription (see SPLiT-seq protocol V3.0). The results are shown in **Fig. 21****.** This suspension was first centrifuged in a plastic tube without the addition of a detergent. Here, a strong adhesion of the suspended objects to the plastic walls was observed (see left image). Adding the adhesion reducing compound Pluronic F127 (e.g. in a final concentration of 0.02 g/L) to the same suspension, completely eliminated the adhesion (see right image).

This experiment (see the results of which are illustrated in **Fig. 21**) clearly shows how the presence of at least one adhesion reducing compound influences the adhesion of suspended objects and thus also biological compartments such as cells. The favorable influence of Pluronic is furthermore documented in the literature, for example for bacterial cells (see Appendices: Boardman, A. K. et al. "Comparison of anti-fouling surface coatings for application in bacteremia diagnostics" Anal. Methods 5(1) (2013): 273-280, Treter, J. et al., "Washing-resistant surfactant coated surface is able to inhibit pathogenic bacteria adhesion", Applied Surface Science 202 (2014): 147-154). Boardman *et al.* describes further adhesion reducing compounds which can also be applied according to the present disclosure.

The influence of incorporating at least one adhesion reducing compound on adhesion with respect to a successful automated split-and-pool workflow (e.g., the SPLiT-seq workflow) was investigated in different situations. In order to test the influence of an adhesion reducing compound also for the present system when processing fixed cells as biological compartments, a situation was experimentally simulated in which cells were distributed on the wells of a 96-well plate. These were incubated thermally, then removed and counted so that cell numbers were available before and after incubation. For the wells previously treated with Pluronic F127 as adhesion reducing compound, the recovery was 81% compared to 72% for control wells without Pluronic.

This overall demonstrates that the presence of adhesion reducing compounds such as Pluronic F127 can have a significant positive effect on the split-and-pool workflow (e.g., the SPLiT-seq workflow), as the total number of biological compartments (such as cells) successfully leaving the workflow can be kept high. Both the addition of at least one adhesion reducing compound to suspended fluids / buffers and the prior coating of plastic material with at least one adhesion reducing compound are feasible. According to one embodiment, containments for receiving biological compartments and/or consumables used for transferring biological compartments are contacted, preferably coated, with at least one adhesion reducing compound prior to contact with the plurality of biological compartments;

### Other measures

Alternatively and/or in addition to incorporating at least one adhesion reducing compound to address the problem of cell loss, the overall workflow may also be optimized at many points during pipetting.

### Examples are

- Multiple pipetting up and down in each individual containment (e.g. well), the contents of which are fed into the pool.
- In the same process, the pipette tips are preferably moved very close to the lowest point of the containment so that as little residual fluid as possible remains in the well after the pipetting step.
- Once the actual suspension of biological compartments has been completely removed from a containment such as a well, it is partially rinsed with further fluid to make any soluble adherent biological compartments transferrable.
- The special interaction of the components in the first type filter module described in detail elsewhere herein was created precisely to prevent losses of biological compartments.

In addition, pipetting from containments such as wells or other containers is arranged in such a way that as many biological compartments as possible are detached from the containments walls by multiple mixing with the pipette before a suspension comprising biological compartments (e.g. cells) is removed. In one embodiment, for pooling in step (c), the same pipette tip is used for collecting preferably all aliquots from their containments (e.g., 96 well plate), since the biological compartments (e.g. cells) at the pipette tip can then be expected to become saturated. Hence, the same pipette tip may be used for collecting aliquots for pooling step (c).

### Use of a cooling module

The method described herein and furthermore the automated system described herein may furthermore comprise a cooling module. The cooling module can be combined with the method and system of the present disclosure.

An active cooling element on the platform ensures the stability of e.g. enzymes up to the point of use and may also be used to inactivate and thus stop reactions such as the RT reaction. According to the present disclosure such a cooling element is provided by a cooling module. The cooling module may comprise establishing a thermal contact to a metal carrier, such as an aluminium carrier by means of a Peltier element. This metal carrier such as the aluminium carrier is capable of holding individual reagent vessels. The module is modelled for the given space in a pipetting robot in order to cool the required number of reagents in the required volume in a space-saving and effective way. This means that even more components can be added to the module, as the full space of the SBS footprint is not yet used.

A cooling module may be provided in order to provide a reduced temperature (preferably less than room temperature). For instance, it may be desired to cool the reagents to a temperature of 15 °C or less, 12 °C or less, 10 °C or less, 8 °C or less, preferably 6 °C or less, or 4°C or less.

According to one embodiment, the cooling module may comprise a metal carrier, such as an aluminium carrier in thermal contact by a Peltier element, wherein the metal carrier is configured to hold at least one reagent vessel. The reagent vessel may comprise the reagents required to perform a reaction (e.g. enzymes, such as a reverse transcriptase or ligase).

According to one advantageous embodiment, the cooling module is reduced in size in contrast to prior art cooling modules allows for more space (e.g. more space on the platform operated by a pipetting module).

According to one embodiment, the metal carrier, which preferably is an aluminium carrier is configured to hold more than one reagent vessel. It may be in particular advantageous to configure the carrier such that the number of reaction vessels are held that need to be held for performing a desired method. For instance, according to some embodiments of the present disclosure, it may be desirable to provide at least as many holders as reaction mixtures are required. For instance, it may be required to provide at least two positions, where one position holds a reaction vessel comprising compounds to perform a polymerization reaction, while another position holds a reaction vessel comprising compounds for performing a ligase reaction. According to one embodiment, the carrier is configured to hold more than 1 reaction vessel, more than 2 reaction vessels, more than 3 reaction vessels, more than 4 reaction vessels, more than 5 reaction vessels, more than 6 reaction vessels, more than 7 reaction vessels, more than 8 reaction vessels, more than 9 reaction vessels, or more than 10 reaction vessels. According to one particular embodiment, the aluminium carrier is configured to hold 17 reaction vessels or more.

According to one embodiment, the metal carrier is configured to hold more than one reagent vessel, wherein the reaction vessels have a different size. For instance, it may be desired to hold a reaction vessel of a larger size in order to cool larger volumes insides the large reaction vessel. Moreover, it may be desired to provide a carrier configured to hold a reagent vessel, wherein the reaction vessel has a small size. Therefore, advantageously, low volumes of the reaction vessel may be quickly cooled by the cooling module. According to one embodiment, the carrier is configured to hold more than one reagent vessel, wherein the reaction vessels have a different size, preferably 2 or more different sizes, 3 or more different sizes, or 4 or more different sizes.

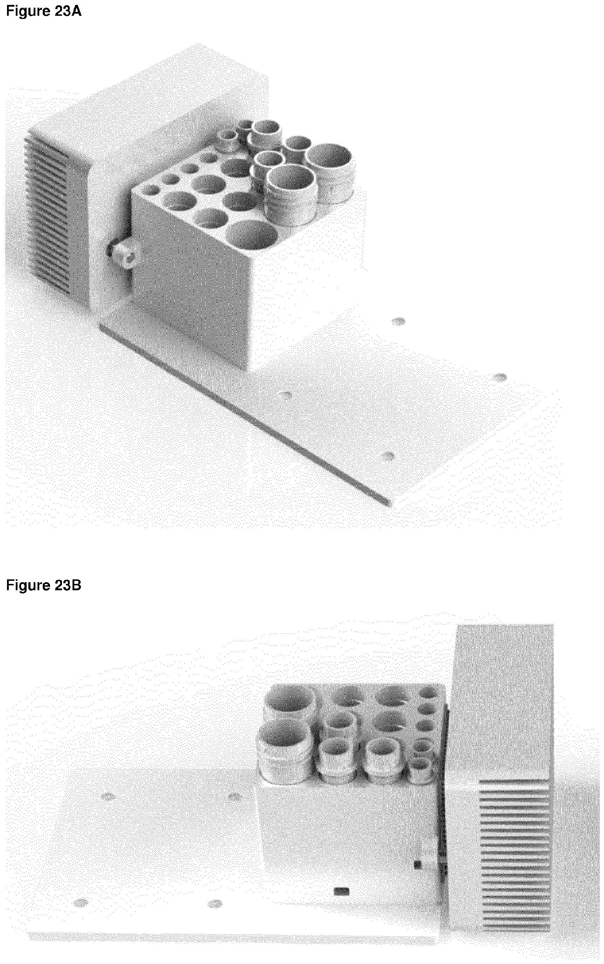

The concept of this metal carrier, which preferably is an aluminium carrier, was modelled as a 3D print as shown in **Fig. 22** to **23B****.**

**Fig. 22** shows a photography of an aluminium holder of the cooling module according to a particular embodiment, wherein the cooling module is configured to hold 17 reaction vessels, wherein the reaction vessels have 3 different sizes.

**Fig. 23A** and **23B** show photographs of an exemplary embodiment of the cooling module from different angles. Therein a Peltier element is illustrated and the aluminium holder configured to hold reaction vessels.

Also disclosed is a cooling module, the module comprising a metal carrier, preferably an aluminium carrier in thermal contact by a Peltier element, wherein the metal carrier is configured to hold at least one reagent vessel, wherein preferably, the reagent vessel comprises the reagents required to perform a polymerization reaction.

The cooling module may have one or more of the following characteristics:
- wherein the cooling module has a reduced size;
- wherein the metal carrier is provided by a metal having a high thermal capacity, preferably selected from copper and aluminium;
- wherein the metal carrier is configured to hold more than one reagent vessel, in particular 20 reaction vessels or less can be held; and/or
- wherein the metal carrier is configured to hold more than one reagent vessel, wherein the reaction vessels have a different size.

Also disclosed is a method for cooling a reagent using a cooling module, the method comprising the steps of (a) adding a reagent vessel holding a reagent to an aluminium carrier, wherein the aluminium carrier is configured to hold at least one reagent vessel; and (b) cooling the reagent vessel, whereby the reagent is cooled. Such method may be performed using an automated system as described herein.

### Biological compartments and target molecules

The biological compartments within the scope of the method may correspond to any known biological compartment or a multitude of biological compartments. According to one preferred embodiment, a biological compartment is a cell. In case the biological compartment is a cell, the RNA may be present inside the cell. Therefore, the whole cellular RNA may be barcode labeled (e.g. RNA present in cytosol, nucleus, mitochondria, etc.). In case the biological compartment is a cell, the cell may be selected from any known cell. The cell may be selected from the group consisting of prokaryotic and eukaryotic cells. The cell may be selected from the group comprising mammalian cells, yeast cells and bacterial cells. The cell may be selected from the groups consisting of bacteria, archaea, plants, animals, fungi, slime moulds, protozoa, and algae. According to a preferred embodiment, the cell is selected from animal cells, preferably human cells. According to one embodiment, the cell is selected from cell culture cell lines. According to another embodiment, the cell is selected from the group consisting of stem cells, bone cells, blood cells, muscle cells, fat cells, skin cells, nerve cells, endothelial cells, sex cells, pancreatic cells, and cancer cells. In some embodiments, the biological compartments may be adherent cells (e.g., adherent mammalian cells). Fixing, permeabilizing, and/or reverse transcription may be conducted or performed on adherent cells (e.g., on cells that are adhered to a plate). For example, adherent cells may be fixed, permeabilized, and/or undergo reverse transcription followed by trypsinization to detach the cells from a surface. Alternatively, the adherent cells may be detached prior to fixing. In some other embodiments, the adherent cells may be detached (e.g. by trypsination) prior to the fixing and/or permeabilizing steps.

According to one embodiment, the biological compartment corresponds to one or more cellular compartments. Cellular compartments may be the cell nucleus, mitochondrion, chloroplast, peroxisome, lysosome, endoplasmic reticulum, Golgi apparatus, vesicle and microtubule. According to one preferred embodiment, the biological compartment is a cell nucleus. It may be advantageous to barcode label the RNA only present in the nucleus, as viable single cells are not required. Use of nuclei may advantageously allow barcode labeling RNA postmortem human tissues or tissues, wherein the extraction of viable intact cells represent a hurdle. Moreover, Lake *et al.* found that nuclear transcriptomes can be representative of the whole cell (Lake, B. B. et al. "A comparative strategy for single-nucleus and single-cell transcriptomes conforms accuracy in predicted cell-type expression from nuclear RNA." 7 (2017): 6031).

The plurality of biological compartments may correspond to a plurality of the same type of biological compartments (e.g. the same cell type or nuclei of the same cell type). Alternatively, the plurality of biological compartments may correspond to a mixture of types of biological compartments (e.g. multiple different cell types from one sample or multiple samples). The plurality of biological compartments may also correspond to a mixture of different biological compartments described above (e.g. a mixture of cells and cellular nuclei). According to a particular embodiment, the plurality of biological compartments may be selected from one or more cell types or nuclei derived from one or more cell types from a particular sample (e.g. a tissue section, tissue extract, biological fluid, etc.).

According to a particularly preferred embodiment, the biological compartments are cells or cellular nuclei. Eukaryotic cells, nuclei or other cellular particles and prokaryotic cells are preferred biological compartments. According to one embodiment, the biological compartments do not comprise droplets produced by microfluidics.

The target molecules may be selected from at least one of RNA, cDNA, DNA, protein, peptide, and antigen. In certain embodiments, the target molecules are macromolecules. In various embodiments, the molecules are selected from at least one of RNA, cDNA, DNA, protein, peptides, and/or antigens. According to one embodiment, the method is for labeling RNA molecules, wherein preferably, the RNA molecules are reverse transcribed to cDNA. As described herein, it is preferred that RNA is reverse transcribed to cDNA.

### Biological compartments in solution

The plurality of biological compartments is preferably surrounded by a liquid. The plurality of biological compartment are preferably provided in form of a suspension. The plurality of biological compartments may be in contact with a liquid, which may be a buffered solution. The plurality of biological compartments may be dispersed inside the solution in order to form a suspension. When referring throughout the present disclosure to a plurality of biological compartments, these will be generally understood as a plurality of biological compartments provided in form of a suspension, unless otherwise indicated (e.g., according to one embodiment during the exchange of the liquid surrounding the biological compartments, the liquid may be predominantly removed, leading to a wet solid of the biological compartments). Preferably, the plurality of biological compartments is not dry.

### FURTHER EXEMPLARY EMBODIMENTS

According to the present disclosure, a method is disclosed for labeling target molecules within a plurality of biological compartment with a combination of barcode labels. It represents an embodiment of the method according to the first aspect. In the following, particular embodiments of the disclosed method are described:
In one embodiment, a method for labeling target molecules within a plurality of biological compartments with a combination of barcode labels, the method comprising:
(i) optionally but preferably counting of biological compartments and calculating the number of the plurality of biological compartments, preferably by an automatic counting module as described herein;
(ii) separating a plurality of biological compartments into at least two aliquots;
(iii) contacting the aliquots of the plurality of biological compartments with a reverse transcription primer comprising a first barcode sequence different for each aliquot to form a hybrid;
(iv) performing a reverse transcription reaction wherein the hybrid of the reverse transcription primer and RNA form a template for the reverse transcription reaction;
(v) merging the aliquots of the plurality of biological compartments to provide a pool;
(vi) optionally but preferably exchanging the liquid surrounding the plurality of biological compartments by a filter module, preferably as described herein;
(vii) separating a plurality of biological compartments into at least two aliquots;
(viii) contacting the aliquots of the plurality of biological compartments with a barcode label comprising a second barcode sequence different for each aliquot to form a hybrid;
(ix) perform a ligation reaction;
(x) merging the aliquots of the plurality of biological compartments; and optionally but preferably, removing at least a portion of aggregates by passing the plurality of biological compartments through a sieving module; and
(xi) repeating steps (vii) to (x), wherein a barcode label comprising a third barcode sequence is used instead of the barcode label comprising a second barcode sequence.

### (i) Optionally but preferably counting of biological compartments and calculating the number of the plurality of biological compartments, preferably by an automatic counting module;

According to one embodiment, the number/concentration of the plurality of biological compartments needs to be determined. For counting the biological compartments, a counting module may be advantageously applied (described elsewhere in the present disclosure and it is here referred thereto).

According to the present disclosure the plurality of biological compartments are fixed prior to counting and preferably also permeabilized. This may be achieved using the filter module according to the present disclosure. According to one embodiment, at least a portion of aggregates are removed, preferably after fixing and permeabilizing the plurality of biological compartments, by passing the plurality of biological compartments (e.g., cells) through a sieving module according to the present disclosure.

### (ii) Separating a plurality of biological compartments into at least two aliquots;

The plurality of biological compartments may be separated into at least two aliquots according to the means of the present disclosure which can also be applied here (e.g. via the pipetting module).

### (iii) Contacting the aliquots of the plurality of biological compartments with a reverse transcription primer comprising a first barcode sequence different for each aliquot to form a hybrid;

In a next step, the aliquots of the plurality of biological compartments are contacted with a reverse transcription primer comprising a first barcode sequence different for each aliquot to form a hybrid. Accordingly, the reverse transcription primer comprises a first barcode sequence, wherein the first barcode sequence contacted with a given aliquot is the same, and wherein a different first barcode sequence is used for different aliquots. The aliquots may correspond to portions of the plurality of biological compartments. These may be transferred into separate containments, which may be provided in form of a well plate.

Preferably the reverse transcription primer is deposited into each containment prior to transferring the aliquots of the plurality of cells. The reverse transcription primer comprising the first barcode sequence may be provided in liquid form (e.g. in form of a solution) or in dried form. For instance, the reverse transcription primer may be provided in a lyophilized and/or surface adsorbed form.

The reverse transcription primer preferably comprises an oligonucleotide. The reverse transcription primer advantageously comprises an oligonucleotide sequence, which is different for each aliquot of the plurality of biological compartments. The reverse transcription primer may comprise an oligonucleotide sequence of a particular length, e.g. 6 or more nucleotides. Moreover, the reverse transcription primer may comprise an oligonucleotide that is capable of hybridizing to cellular RNA. According to one exemplary embodiment, the oligonucleotide capable of hybridizing to the cellular RNA may be a repeat of the nucleotide "T", e.g. a poly d(T). The step may not be limited to the particular reverse transcription primer and other reverse transcription primer may be readily found in the prior art cited herein (see e.g. Rosenberg et al., 2018; SPLiT-seq V3.0 protocol; US 2016/0138086; WO 2019/060771).

### (iv) Performing a reverse transcription reaction wherein the hybrid of the reverse transcription primer and RNA form a template for the reverse transcription reaction

After contacting the plurality of biological compartments with the reverse transcription primer, the formed hybrid represents a template for a polymerization reaction, such as a reverse transcription reaction. Afterwards, a polymerization reaction may be performed, preferably utilizing a reverse transcriptase. After the reverse transcription primer formed a template with the RNA, it represents a substrate for a polymerase reaction. In order to generate a stable RNA template for the subsequent steps, the cellular/nuclear RNA can be enzymatically polymerized. The polymerization reaction is preferably performed by a reverse transcriptase. Thereby, the cDNA of the mRNA is generated.

### (v) Merging the aliquots of the plurality of biological compartments;

According to one embodiment, the aliquots of the plurality of biological compartments are merged after the reverse transcription. According to one embodiment, the pipetting module may transfer the aliquots of the plurality of biological compartments from the containments (e.g. the well plate) into a collection containment. Therefore, the aliquots are merged to form one "pool". Preferably mixing operations according to the present disclosure may be performed.

### (vi) Optionally but preferably exchanging the liquid surrounding the plurality of biological compartments by a filter module

According to one embodiment, preferably the liquid surrounding the plurality of biological compartments is exchanged by a filter module according to the present disclosure. The filter module is described herein and it is here referred to the respective disclosures/sections.

### (vii) Separating a plurality of biological compartments into at least two aliquots

The plurality of biological compartments may be separated into at least two aliquots according to the means of the present disclosure which can also be applied here (e.g. via the pipetting module).

### (viii) Contacting the aliquots of the plurality of biological compartments with a barcode label comprising a second barcode sequence different for each aliquot to form a hybrid

According to the method of the present disclosure the plurality of biological compartments is contacted with a barcode label, wherein the barcode label is different for each aliquot of the plurality of biological compartments, and wherein the barcode label hybridizes to the product according to step (iv). According to one embodiment, the product is the result of a reverse transcription of the RNA present in the biological compartments. Reverse transcription of the RNA provides cDNA. Preferably, the product according to step (iv) comprises a region, which can hybridize to the barcode label. In particular, it may be desired to incorporate a region by the first barcode label according to step (iv) into the product of step (iv) that comprises a hybridizing region. Such a hybridizing region may be referred to as an "adapter sequence". It advantageously may be capable of hybridizing to the barcode label.

According to a preferred embodiment, the barcode label is provided into the containments before transferring the aliquots of the plurality of biological compartments into the containments. The barcode label may comprise a universal linker oligonucleotide with partial complementarity to a second oligonucleotide containing the second barcode sequence. The second barcode sequence may be preferably unique for each aliquot of the plurality of biological compartments (i.e. for each containment). These oligonucleotides are hybridized prior to the contacting step. By the hybridization an oligonucleotide may be generated (referred to as barcode label) comprising three distinct functional domains:
- a 5' overhang that is complementary to the 3' overhang present on the product according to step (iv); e.g. the polymerized RNA present in the plurality of cells (e.g. a cDNA molecule (may originate from RT primer or previous barcoding round)),
- a barcode sequence (e.g., second barcode sequence) comprising an oligonucleotide sequence which is unique for each aliquot of the plurality of cells (i.e. for each containment), which may be referred to as the barcode sequence, and
- a 3' overhang complementary to the 5' overhang present on the oligonucleotide to be subsequently ligated (e.g., next/subsequent (e.g., third) barcode label).

An incubation step may be performed after contacting the plurality of biological compartments with the barcode label. An exemplary incubation may be performed for 30 min at 37 °C. During incubation, the barcode label advantageously hybridizes to the product according to step (iv). Therefore, the barcode label diffuses into the plurality of biological compartments and therein interacts with the polymerized RNA (i.e. the RNA which was polymerized by during the polymerization step). Therefore, part of the polymerized RNA, which may derive from step (iv) may hybridize to part of the barcode label. The formed hybrid then may provide a template for a ligation reaction. The embodiment may not be limited to the particular barcode label and other barcode labels may be readily accessible by the skilled person and also may be disclosed in the prior art cited herein (see Rosenberg et al., 2018; SPLiT-seq V3.0 protocol; US 2016/0138086; WO 2019/060771).

### (ix) Perform a ligation reaction;

According to the method of the present disclosure, a ligation reaction is performed. The ligation reaction in step (ix) may be referred to as the "first ligation". The ligation reaction can be advantageously performed inside the plurality of biological compartments. Ligation has been described elsewhere in the present disclosure and it is here referred thereto. A ligation reaction may be performed by the heating module, such as the automatically lockable heating module, allowing for heating to elevated temperatures, such as 95 °C. According to one embodiment, the ligation reaction ligates the barcode label comprising the second barcode sequence and the reverse transcribed RNA (which can be the cDNA strand comprising the first barcode sequence). According to one embodiment, blocking agents may be applied after ligation. Blocking agents may be selected from blocking oligonucleotides. Blocking ensures that unbound DNA barcodes cannot mislabel cDNA in future barcoding rounds.

### (x) Merging the aliquots of the plurality of biological compartments; and optionally but preferably, removing at least a portion of aggregates by passing the plurality of biological compartments through a sieving module

According to one embodiment, the aliquots of the plurality of biological compartments are merged after the reverse transcription. According to one embodiment, the pipetting module may transfer the aliquots of the plurality of biological compartments from the containments (e.g. the well plate) into a collection containment. Therefore, the aliquots are merged to form one "pool". Preferably mixing operations according to the present disclosure may be performed.

According to one embodiment, optionally but preferably at least a portion of aggregates is removed by passing the plurality of biological compartments through a sieving module according to the present disclosure.

### (xi) Repeating steps (vii) to (x), wherein a barcode label comprising a third barcode sequence is used instead of the barcode label comprising a second barcode sequence.

According to step (xi), steps (vii) to (x) are repeated, wherein a barcode label comprising a third barcode sequence is used instead of the barcode label comprising a second barcode sequence.

According to a preferred embodiment, the barcode label is provided into the containments before transferring the aliquots of the plurality of cells into the containments. Moreover, the barcode label may comprise a universal linker oligonucleotide with partial complementarity to a third oligonucleotide containing a third barcode sequence. The third barcode sequence may be preferably unique for each aliquot of the plurality of biological compartments (i.e. for each containment). These oligonucleotides are hybridized prior to the contacting step. By the hybridization an oligonucleotide may be generated comprising four distinct functional domains:
- a 5' overhang that is complementary to the 3' overhang present on the ligated RNA present in the plurality of cells (e.g. a cDNA molecule (may originate from prior ligation));
- a third barcode sequence comprising an oligonucleotide sequence which is unique for each aliquot of the plurality of cells (i.e. for each containment), which may be referred to as third barcode sequence;
- optionally, a unique molecular identifier (UMI) sequence, allowing to analyze the RNA molecule in a highly quantitative manner, which is advantageous for an absolute analysis; and
- optionally, a PCR primer.

The embodiment may not be limited to the particular barcode label and other barcode labels may be readily accessible by the skilled person and also may be disclosed in the prior art cited herein (see e.g. Rosenberg et al., 2018; SPLiT-seq V3.0 protocol; US 2016/0138086; WO 2019/060771).

An incubation step and a ligation reaction may be performed as disclosed above. Afterwards the plurality of biological compartments may be merged. After merging the aliquits of the plurality of biological compartments one "pool" may be generated. The plurality of biological compartments may comprise aggregates of biological compartments (e.g. multiple cells which are associated). These may be preferably removed by the sieving module according to the present disclosure.

The automated system described herein allows to integrate the above described novel modules and conventional pipetting robotics (e.g., pipetting module) on one platform. This allows to automate a central part of the split-and-pool workflow, such as the SPLiT-seq workflow as described herein. The newly developed modules enable this degree of automation. The automated system furthermore enables the very simple operation of the entire automation system by means of a graphical user interface.

The present disclosure thereby makes an important contribution to the prior art platforms by achieving an automation of the SPLiT-seq workflow. The automated workflow combines in embodiments existing technical solutions with the novel modules described herein.

This disclosure in particular provides individual modules which allow, in particular in combination, to effectively automate central, complex parts of the SPLiT-seq method/workflow. The basic platform of the automated system is a pipetting robot on which one or more, such as two or more or all, of the individual modules may be placed. The individual modules, their features and integration into the entire workflow are described herein.

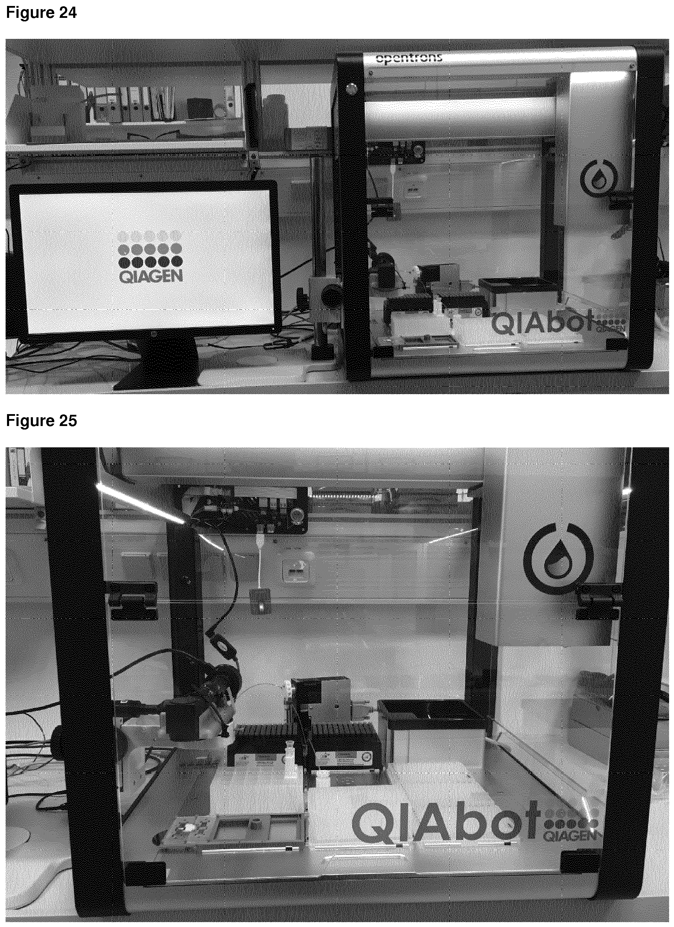

**Figs. 24 to 26** illustrate a possible experimental set-up of the automated system, with the computer screen illustrating the screen that will be attached to the main body of the instrument.

In the shown embodiment, cooling and heating elements are not yet directly integrated on the deck of the automated system, but can be present as described herein.

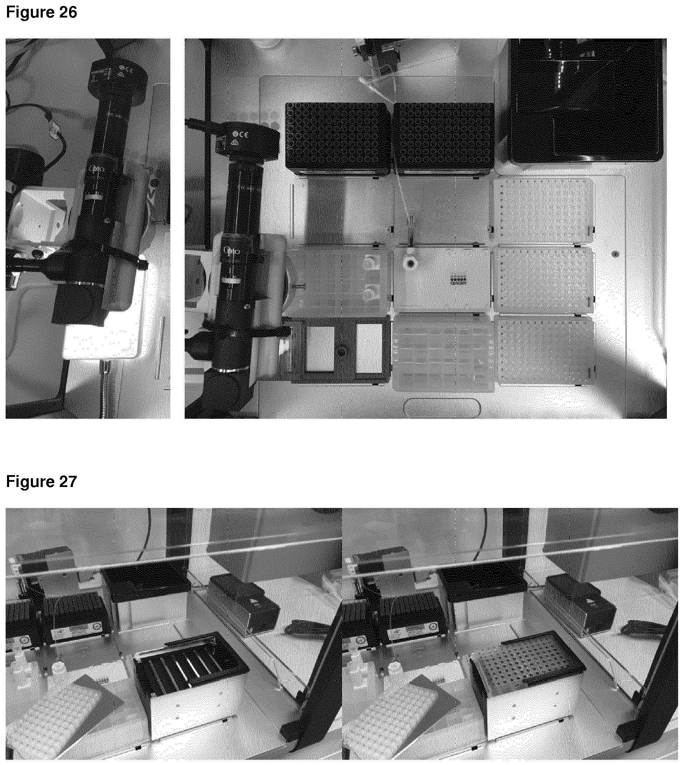

The individual modules can in turn have several configurations, such as exchanging the light source for the microscope as shown in **Fig. 26****.**

In addition to the technical solutions provided by the method and the individual modules described herein, an important aspect of the present disclosure is the technical solution created by their interaction. This allows to provide a "platform" for automation and enables an automated workflow such as for the core of the SPLiT-seq protocol. The details for this interaction result from the multitude of programmed files and the associated framework (hardware and software) on which they are executed. In particular, an advantageous interaction results in the following functionality:
- At the beginning of the workflow, a sample of fixed single cells (or other biological compartments) and other reagents are placed on the platform.
- If necessary, the platform takes over the cooling of certain components until they are used, for example enzymes.
- The platform mixes ready-to-use mixes ("ReadyMix") from the individual components.
- One option is for the platform to use an integrated imaging device such as a microscope together with image analysis algorithms to automatically measure the cell count of the individual cells provided. Alternatively, the cell count must be adjusted by the user prior to the workflow.
- The cell suspension is kept homogeneous on the platform, which may be necessary before sedimentation in case of longer waiting times. For this purpose, the advantageous mixing method described herein can be used.
- The cells are divided on a multiwell plate in form of aliquots ("split" process), and the multiwall plate is placed on/in a thermal element. In this multiwell plate, special DNA primers are deposited which preferably function as reverse transcription primer and preferably introduce well-specifically a barcode sequence.
- A mixed ReadyMix for a reverse transcription reaction ("RT") is added to each well of the multiwell plate (or the wells may already incorporate a reverse transcription reaction composition prior to addition of the aliquots). As described herein, if the reverse transcription primer comprises a first barcode sequence, it is preferred that each aliquot is contacted with a barcode label comprising a barcode sequence that differs from the barcode sequences of the barcode labels that are contacted with other aliquots.
- The well-plate is preferably sealed and the reverse transcription (RT) reaction is carried out. This can be done, e.g., by thermocycling. The platform may thus comprise an integrated thermo-cycling-module. This allows to perform the reverse transcription in an automated manner. In the process, mRNA in the biological compartments (e.g. cells) is transcribed into cDNA and are at the same time provided with well-specific barcodes in case a barcoded reverse transcription primer is used (as it is also described in the prior art).
- After the RT reaction, the contents of the individual wells and thus the aliquoted biological compartments are collected in the same containment, e.g. a well or basin ("pool" process). The distribution of the biological compartments in the pool is randomized, e.g. by mixing. This ensures that the location of biological compartments that were previously in the same well/aliquot are randomized within the provided pool.
- This fluid comprising the pool is filtered on the filter module described herein so that the suspending fluid can be changed. This eliminates the need for tedious and error-prone processes such as centrifugation which can lead to a loss of cells. Remainders of the RT reaction and in particular reverse transcription primers are removed by exchanging the fluid. This fluid/buffer exchange allows to provide the plurality of biological compartments in a buffer/solution that is suitable for further processing steps and further barcode attachment, which is preferably assisted by ligation.
- A Ligations-ReadyMix comprising reagents necessary for performing a ligation reaction is provided and preferably contacted with the pool of biological compartments. This ligase mix may form a suspension with the biological compartments. The plurality of biological compartments (pool) is then again separated into at least n aliquots, preferably by transferring portions of the plurality of biological compartments into wells of another multiwell plate ("split" process). Well-specific barcodes are available and preferably preset into the wells of this multiwell plate, as is known in the prior art and described herein.
- This multiwell plate is preferably located on a thermocycling element. As described herein, the loaded well-plates may be automatically sealed.
- In the subsequent ligation incubation, the well-specific barcode labels for each well are attached to the cDNA's in the cells which may be supported by ligation.
- Subsequently, blocking oligonucleotides may optionally be added to prevent further ligations after a period of incubation.
- The contents of the single wells are again collected and pooled, mixed and thus randomized.
- Sieving the obtained pool using the sieving module described herein allows to remove aggregates if formed. E.g. the sieving module may comprise a mesh having a mesh size of 40 µm, which retains larger cell aggregates that could otherwise worsen the randomization process.
- For performing a second ligation reaction to attach the next barcode label, the biological compartments are transferred preferably together with Ligations ReadyMix to another multiwell plate with further well-specific barcode labels. Similar to the first ligation reaction, the blocking, pooling and sieving steps can then be performed.

The automated workflow illustrated above thus provides the transcription of mRNA in the individual cells to cDNA. At the same time, this cDNA receives combinatorial barcodes added by the split-and-pool workflow, which later allow single cell assignment of the mRNAs during sequencing.

The technology covers the core of the Split Seq workflow. Technologically, it offers the following improvements and enhancements:
- At the beginning, the step of biological compartment (e.g. cell) fixation (e.g. with formalin) can be automated (see SplitSeq Protocol V3.0). In addition to the pipetting steps, a further filter module would be required to allow performing the fixing process on the automated system. As disclosed herein, this filter module is conceptually identical to the filter module described herein.
- By adapting the membrane in the filter module, the workflow is also designed to process cell nuclei instead of whole cells.
- The subsequent processing at the end of the workflow (lysis, purification and creation of an NGS library as disclosed in the prior art), may also be integrated on the automated system by including according processing modules. Examples include, e.g. a magnetic module as an additional element. A potentially suitable module would be for example the MagDeck from Opentrons. The MagDeck may be provided as a further module on the deck of the platform and integrated into the workflow as illustrated in **Fig. 27****.**

Hence, the present disclosure advantageously provides
1. An overall system for automating the core of the SPLiT-seq protocol.
   - It provides the user with the ability to perform the core of the SPLiT-seq workflow without special skills (e.g. omission e.g. from the skill of pellet washing).
   - It provides the first automation solution for the core part of the SPLiT-seq workflow.
2. Cell counting and sliding mechanism using the pipetting module
   - The present disclosure allows to provide counting chambers by mechanical fixation on the pippeting robot deck.
   - The sliding mechanism described herein makes the same counting chambers available for an imaging device such as a microscope adjacent to the working chamber/space accessible to the pipetting head.
   - The use of the pipetting module or pipetting head in a novel manner allows to move components without additional electronics. This furthermore extends/maximizes the reachable working volume/space of the pipetting-robot.
   - The use of cell staining to reduce the need for phase contrast to allow simpler brightfield imaging.
   - In the overall context allowing automatic cell counting via algorithms to ultimately adjust the algorithmic protocol of the workflow while running ("dynamic protocol").
3. Algorithms
   - Frontend and backend, which on the one hand allow the user a simple experimental setup and on the other hand seclude the complexity of the SPLiT-seq workflow from the user.
   - Mixing algorithm against the background of volumes to be mixed that are significantly larger than pipette tip volumes for mixing the fluid.
   - An algorithm that allows to automate the most important part of the SPLiT-seq workflow.
   - A dynamic protocol: Dynamic adjustment of the protocol during its performance/runtime (processing of cell count)
4. Filter module for changing solutions surrounding the plurality of biological compartments
   - Provides an advantageous, simple technical solution for washing biological compartments (e.g. cells), e.g. to carry out a necessary buffer change.
   - Enables ligation on the automated SPLiT-seq platform after reverse transcription by allowing to exchange the buffer in an automated manner.
   - Allows automatic fixing of biological compartments such as cells for the SPLiT-seq workflow.
5. Cell sieves allowing the removal of cell aggregates
   - A static sieve structure with lateral deflection of the fluid eliminates the need to remove the sieve structure in order to make the fluid accessible to the pipetting robot or pipetting module.
6. Measures against cell loss
   - Use of surfactants/detergents
   - Pipetting technology designed to replace adhesive cells.
7. Thermoelements
   - The SPLiT-seq workflow can be automated by fully automated closing of the lids.

### Optimized user interface

According to the present disclosure, an optimized graphical user interface is also provided for an automated workflow. In scope of the SPLiT-seq workflow, it is one object to hide the complexity of the entire system from the user as far as possible. In this way, it is possible for the untrained person to implement the complicated SPLiT-seq workflow.

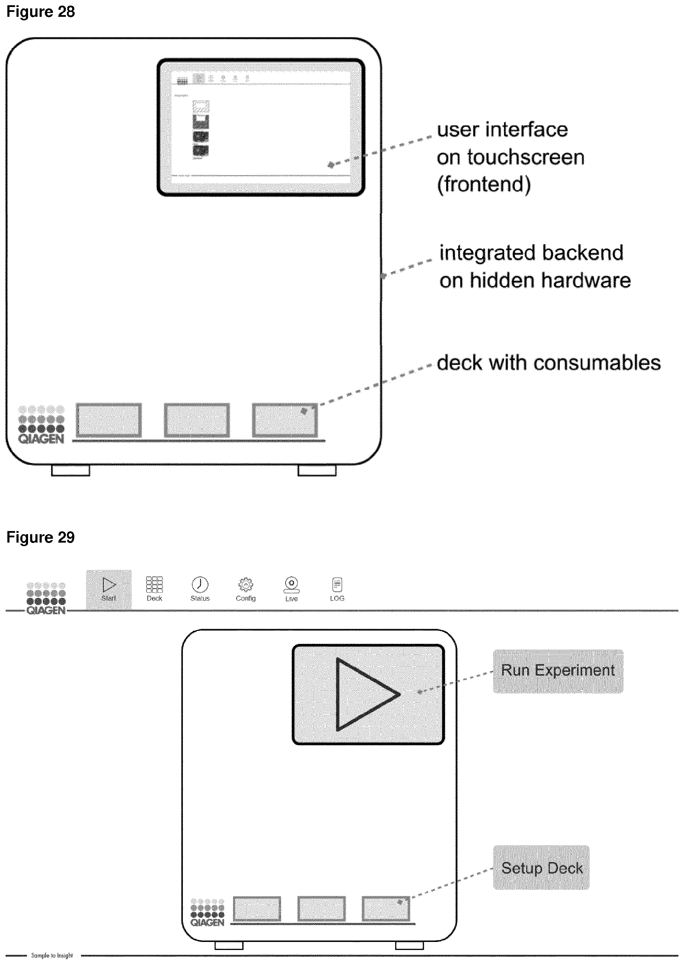

According to the present disclosure, one central aspect of an optimized user interface comprises a frontend that initially guides the user to fully equip the deck of the disclosed system (e.g., including a pipetting robot) with consumables such as pipette tips or reagents. The frontend is then designed to start the SPLiT-seq workflow at the touch of a button. An important component for this user interaction is a touch screen, which is attached to the instrument as is illustrated in **Fig. 28****.**

Behind the frontend (= graphical user interface), the backend works on hardware that is hidden from the user. The algorithms of the backend pursue the goal of taking over the robotics control, which can be implemented in a variety of ways. In the current version, this backend consists of Python routines that run on a PC and other routines on a Raspberry Pi in the prototype. The Raspberry Pi in turn acts with other hardware boards in the instrument. The entire process is encapsulated by the user and automatically executed in the background. A complete integration is also currently being implemented, characterized by the fact that all hardware components are integrated in the prototype. This makes the complete system self-sufficient and requires only one power source, the consumables and the sample itself to run.

As an example of the user guidance concept, the current application is cited in whose main menu the user has the choice of either starting the run or receiving the instructions to correctly occupy the deck of the robot. The main menu is kept intuitive as is illustrated in **Fig. 29****.**

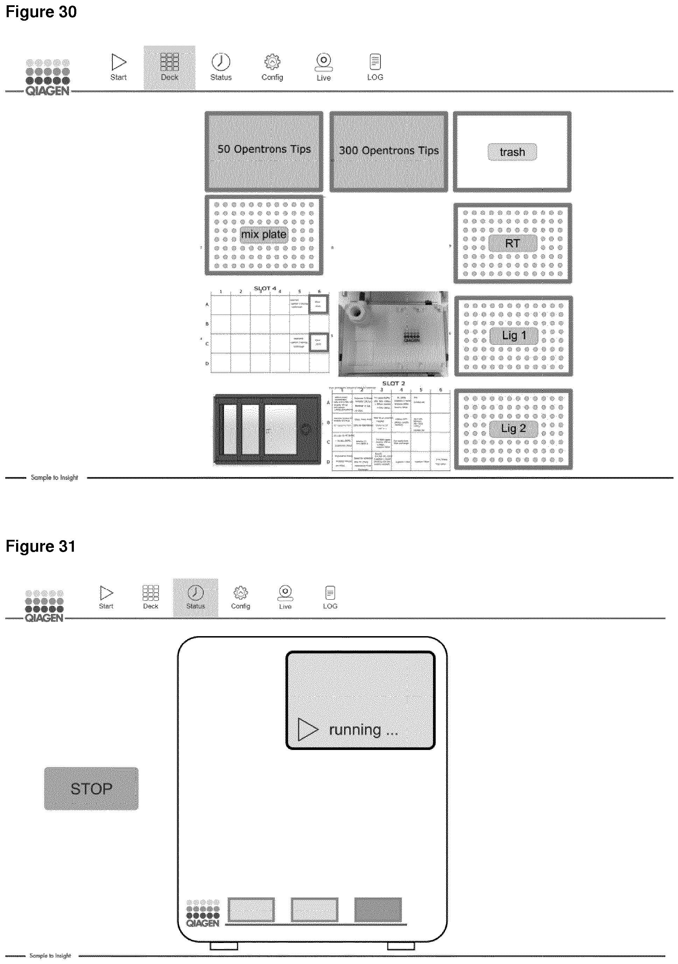

If the user selects "Setup Deck", visual and textual instructions are given as illustrated in **Fig. 30****.**

Back in the main menu the complex workflow can easily be started with "Run Experiment", as is illustrated in **Fig. 31****.**

### A pipetting module

Any pipetting module may be applicable in conjunction with the disclosed modules. The pipetting module is precise enough to navigate/reach the desired position (e.g., the opening of a well plate such as a 24, 48, 96 or 384 well plate). Prior art pipetting modules that may be applicable in conjunction with the present disclosure include but are not limited to system from Opentrons, Tekan, Hamilton, QIAGEN. The pipetting module according to the present disclosure can advantageously transfer fluids (e.g. biological compartments as a suspension; reagent compositions, etc.) from one containment to another in an automated manner. Moreover, the pipetting module can aspirate and dispense fluids. In between the aspiration and dispensing, fluids are advantageously transferred, wherein the fluids are present in a pipette tip. Transferring may not be desired, when the fluid is mixed by dispensing and aspirating. The volume which can be maximally aspired is typically dependent on the size of the pipette and the used pipette tip and shall not be limiting in scope of the present disclosure. According to one embodiment, the volume which can be aspired and dispensed by the pipetting module ranges from 1 µl to 10 ml. According to a particular embodiment, the volume that is or can be maximally aspired and dispensed is 5 ml or less, 3 ml or less or 1 ml or less. It may be 900 µl or less, 800 µl or less, 700 µl or less, 600 µl or less or 500 µl or less.

According to one embodiment, the pipetting module comprises a pipetting head connected to a pipette. The pipette may be reversibly attached to a pipette tip, wherein the attachment and removal of the pipette tip can be advantageously achieved automatically (e.g., controlled by a sequence of commands from a processing unit). For instance, it may be desired to exchange the pipette tip after transferring fluids or in between fluid transfer operations in order to avoid cross-contaminations. Therefore, the pipette tip can be automatically exchanged. It may also be desired to exchange the pipette tip before and/or after using the counting module or counting system according to the present disclosure in order to avoid cross-contaminations.

According to a preferred embodiment, the pipetting module comprises further components to perform movements in two or preferably, three dimensions, e.g. in x-, y- and z-direction. For instance, the pipetting module may perform first a movement in x- and y-direction, followed by movement in z-direction (e.g., downwards movement). In another instance, the pipetting module may first perform a movement in z-direction (e.g., upwards/lifting movement), followed by movement in x- and y-direction. According to some embodiment disclosed herein, the movement of the pipetting module is advantageously used to support mixing of fluids (e.g., rotational movement when the pipette tip is in contact with a fluid, such as a suspension of biological compartments). Details are described in conjunction with the mixing operation.

According to one embodiment, the pipetting module can access a certain area, which may be referred to as a platform (also robot platform, robotic platform, automated robotic platform, automated platform, automated pipetting platform, robotic pipetting platform, etc.). The disclosed modules such as the sliding device allow to extend the accessible area by moving an object to an area that is not accessible by the pipetting module. Therefore, the accessible area is not reduced by components (e.g., a microscope) and moreover, collision of the pipetting module and the components is prevented.

The pipetting module typically comprises a processing unit or has an electronic interface to a processing unit. This advantageously allows to control the pipetting module, respectively, the pipetting module operations. In particular, a sequence of commands for processing operation, controls and/or monitoring functions may be utilized to control the pipetting module in an automated manner.

Within the framework of this invention, various modules are disclosed which may each have one or more control units with regard to their execution and implementation. If individual modules are combined, the individual control units of the modules can be used. It is possible that one of several control units assumes an integrative task in such a way that this control unit is superior to other control units. Alternatively or additionally, an additional higher-level control unit may be provided which controls and/or monitors at least some of the individual control units. It is also possible to provide an overall control unit, especially an overall control unit which combines control for at least two of the modules. It is also possible to use control units which are amended by a sequence of commands for processing operations, controls or monitoring functions.

### THE FILTER MODULE ACCORDING TO A FURTHER ASPECT

According to a further aspect, a filter module is provided for exchanging a liquid surrounding a plurality of objects, preferably biological compartments or for separating a liquid from a plurality of objects, preferably biological compartments. The filter module is described elsewhere in the present disclosure and it is here referred thereto.

### THE METHOD FOR REMOVING AND PREFERABLY EXCHANGING A LIQUID SURROUNDING A PLURALITY OF OBJECTS ACCORDING TO A FURTHER ASPECT

According to a further aspect, a method is provided for removing and preferably exchanging a liquid surrounding a plurality of objects, preferably biological compartments or for separating a liquid from a plurality of objects. The method is described elsewhere in the present disclosure and it is here referred thereto.

### THE USE OF A FILTER ELEMENT ACCORDING TO A FURTHER ASPECT

According to a further aspect, a use is provided of a filter element, preferably a membrane, for exchanging a liquid surrounding a plurality of objects, preferably biological compartments or for separating a liquid from a plurality of objects, preferably biological compartments, wherein a filter element, preferably a membrane, is used to filter the objects, wherein a device is used which applies a pressure differential acting across the a filter element, which preferably is a membrane. The use is described elsewhere in the present disclosure and it is here referred thereto.

### THE METHOD OF EXCHANGING A LIQUID SURROUNDING A PLURALITY OF BIOLOGICAL COMPARTMENTS ACCORDING TO A FURTHER ASPECT

According to a further aspect, a method is provided of exchanging a liquid surrounding a plurality of biological compartments using a filter module. The method is described elsewhere in the present disclosure and it is here referred thereto.

### THE COUNTING MODULE ACCORDING TO A FURTHER ASPECT

According to a further aspect, a counting module is provided. The counting module is described elsewhere in the present disclosure and it is here referred thereto.

### THE SLIDING DEVICE FOR AXIALLY DIRECTING AN OBJECT USING A PIPETTING MODULE ACCORDING TO A FURTHER ASPECT

According to a further aspect, a sliding device is provided for axially directing an object using a pipetting module. The sliding device is described elsewhere in the present disclosure and it is here referred thereto.

### THE METHODS FOR COUNTING BIOLOGICAL COMPARTMENTS ACCORDING TO A FURTHER ASPECT

According to a further aspect, methods are provided for counting biological compartments and calculating the number of a plurality of biological compartments by an automatic counting module. The method and embodiments thereof are described elsewhere in the present disclosure and it is here referred thereto.

### THE SYSTEM FOR COUNTING BIOLOGICAL COMPARTMENTS AND CALCULATING THE NUMBER OF A PLURALITY OF BIOLOGICAL COMPARTMENTS ACCORDING TO A FURTHER ASPECT

According to a further aspect, a system is provided for counting biological compartments and calculating the number of a plurality of biological compartments. The system is described elsewhere in the present disclosure and it is here referred thereto.

### THE SIEVING MODULE ACCORDING TO A FURTHER ASPECT

According to a further aspect, a sieving module is provided. The sieving module is described elsewhere in the present disclosure and it is here referred thereto.

### THE HOLDER ACCORDING TO A FURTHER ASPECT

According to a further aspect, a holder is provided for holding a receptacle configured to laterally direct a plurality of biological compartments passing the receptacle is provided. The holder is described elsewhere in the present disclosure and it is here referred thereto.

### THE USE OF THE SIEVING MODULE AND/OR THE HOLDER ACCORDING TO A FURTHER ASPECT

According to a further aspect, a use of the sieving module and/or holder is provided for removing aggregates of biological compartments, preferably aggregates of cells and/or nuclei, in processing protocols, in particular in split-and-pool workflows, such as a method according to the first aspect of the present disclosure. The use is described elsewhere in the present disclosure and it is here referred thereto.

### THE METHOD FOR SIEVING A FLUID ACCORDING TO A FURTHER ASPECT

According to a further aspect, a method is provided for sieving a fluid, preferably for removing aggregates from a suspension comprising a plurality of biological compartments using a sieving module or a system according to the present disclosure. The method is described elsewhere in the present disclosure and it is here referred thereto.

### THE SIEVING SYSTEMS ACCORDING TO A FURTHER ASPECT

According to a further aspect, sieving systems are provided for sieving a fluid, preferably for removing aggregates from a plurality of biological compartments. The sieving systems are described elsewhere in the present disclosure and it is here referred thereto.

### THE METHOD IS PROVIDED FOR HEATING CONTAINMENTS ACCORDING TO A FURTHER ASPECT

According to a further aspect, a method is provided for heating containments, e.g. wells of a well-plate. The method is described elsewhere in the present disclosure and it is here referred thereto.

### THE HEATING MODULE ACCORDING TO A FURTHER ASPECT

According to a further aspect, a heating module is provided. The heating module is described elsewhere in the present disclosure and it is here referred thereto.

### THE USE OF THE HEATING MODULE ACCORDING TO A FURTHER ASPECT

According to a further aspect, a use of the heating module for heating containments for performing a reverse transcription and/or a ligation reaction, wherein the heating module has one or more of the characteristics according to the present disclosure. The use is described elsewhere in the present disclosure and it is here referred thereto.

### THE AUTOMATED SYSTEM ACCORDING TO A FURTHER ASPECT

According to a further aspect, an automated system is provided for labeling target molecules within a plurality of biological compartments. The system is described elsewhere in the present disclosure and it is here referred thereto.

An automated system for labeling target molecules within a plurality of biological compartments is provided, the system comprising one or more of the following modules:
- a pipetting module;
- one or more filter modules for exchanging a liquid surrounding a plurality of cells in an automated manner;
- optionally, one or more sieving modules for removing aggregates from a plurality of cells in an automated manner;
- optionally, an automated counting module;
- optionally, a cooling module; and
- optionally, an automatically lockable heating module.

The individual elements were described above, in particular in the context of the method according to the first aspect and it is referred to the corresponding disclosure which also applies here. The automated system is furthermore described in the claims and further aspects and embodiments. The automated system preferably comprises a thermocycling module.

### FURTHER ASPECTS AND EMBODIMENTS

Also disclosed as further aspects and embodiments of the present disclosure which are also disclosed elsewhere herein are the following items:
1. A method for labeling target molecules within a plurality of biological compartments with a combination of barcode labels, the method comprising:
   (a) separating a plurality of biological compartments into at least two aliquots;
   (b) contacting barcode labels comprising a barcode sequence with the at least two aliquots comprising biological compartments,
      - wherein the barcode sequence of the barcode labels contacted with a given aliquot is the same, and
      - wherein a different barcode sequence is used for different aliquots;
   (c) combining aliquots comprising biological compartments into a pool; and
   (d) repeating steps (a), (b) and (c) using the combined pool.
2. The method according to item 1, wherein step (d) is repeated a number of times sufficient to generate a unique combination of barcode sequences for the target molecules in a single cell.
3. The method according to item 1 or 2, wherein the method is for barcoding nucleic acids within a biological compartment and wherein the method comprises generating cDNAs within the biological compartments by reverse transcribing RNAs into cDNA, preferably using a reverse transcription primer comprising a 5' overhang sequence.
4. The method according to any one of items 1 to 3, in particular item 3, wherein a reverse transcription reaction is performed prior to step (a).
5. The method according to one or more of items 1 to 4, in particular item 3 or 4, comprising performing an *in situ* reverse transcription reaction using a plurality of biological compartments, preferably cells, in form of a pool, wherein optionally, the 5' overhang of the reverse transcription primer provides an adapter sequence for the barcode label that is used in step (a).
6. The method according to one or more of items 1 to 5, in particular item 3 or 4, comprising performing an *in situ* reverse transcription reaction, wherein a first barcode label is introduced during reverse transcription,
   wherein preferably, the method comprises (i) separating a plurality of biological compartments into at least two aliquots; and (ii) contacting each of the aliquots with a reverse transcription primer comprising a barcode sequence,
   - wherein the barcode sequence of the reverse transcription primer is the same for a given aliquot, and
   - wherein a different barcode sequence is used for each of the n aliquots;
   and wherein preferably, the method comprises after reverse transcription combining the biological compartments of the aliquots thereby providing a plurality of compartments that can be subjected to step (a) of the method.
7. The method according to one or more of items 1 to 6, wherein the method comprises using a filter module for separating biological compartments from a liquid and/or for concentrating biological compartments within a liquid.
8. The method according to item 7, wherein using the filter module fulfills one of more of the following characteristics:
   - it is performed in an automated manner, preferably using a syringe pump;
   - it is performed after performing a reverse transcription reaction and/or after attachment of a barcode label;
   - a defined volume of liquid is removed, wherein preferably, a syringe pump generates a low pressure to suck a defined volume of the liquid through the filter element; and/or
   - it is performed for exchanging a liquid surrounding a plurality of biological compartments, wherein at least a part of the liquid surrounding the plurality of biological compartments is separated and wherein after separation, the method comprises contacting and mixing the plurality of biological compartments with another liquid; wherein optionally, the separation and contacting steps are repeated.
9. The method according to item 7 or 8, wherein a filter module according to one or more of items 74 to 95 is used and/or wherein the method according to one or more of items 98 to 103 is performed.
10. The method according to one or more of items 1 to 9, wherein the method comprises removing aggregates of biological compartments, such as cell aggregates, preferably by passing a fluid comprising biological compartments such as a suspension of biological compartments through a sieving module.
11. The method according to item 10, wherein the removal of the aggregates fulfills one of more of the following characteristics:
   - it is performed in an automated manner;
   - it is performed after pooling step (c) and/or after fixing the biological compartments; and/or
   - it is performed using a sieving module which passes the sieved fluid through a lateral outlet into a containment such as a reservoir, wherein preferably, the sieving module is placed onto or in one containment, e.g. a well of a well-plate, and laterally passes the sieved fluid into a neighboring containment, e.g. a well of a well-plate.
12. The method according to item 10 or 11, wherein the sieving method according to one or more of items 142 to 144 is used for removal of the aggregates and/or wherein a sieving module according to one or more of items 104 to 124 or a sieving system according to one or more of items 129 to 138 is used for removing aggregates.
13. The method according to one or more of items 1 to 12, further comprising performing a ligation reaction, preferably for ligating at least two of the barcode labels that are bound to the target molecules, wherein preferably the ligation is performed within the plurality of cells.
14. The method according to one or more of items 1 to 13, further comprising fixing and/or permeabilizing the plurality of biological compartments prior to step (a) and preferably prior to reverse transcription, if a reverse transcription is performed.
15. The method according to one or more of items 1 to 14, further comprising performing a biological compartment counting step, optionally a cell counting step.
16. The method according to item 15, wherein the biological compartment counting step fulfills one or more of the following characteristics:
   (i) it is performed in an automated manner;
   (ii) it is performed prior to step (a);
   (iii) it is performed after performing fixing and/or permeabilizing the biological compartments and prior to performing step (a);
   (iv) it is performed prior to performing a reverse transcription reaction;
   (v) it comprises the use of at least one counting chamber, optionally a hemocytometer, wherein the at least one counting chamber is optionally held by a holding device;
   (vi) it comprises subjecting a portion of the plurality of biological compartments to counting, wherein the portion is transferred into one or more counting chambers, optionally wherein the transfer occurs in an automated manner using a pipetting module; and/or
   (vii) counting involves staining the biological compartments to be counted and/or using an imaging device for counting.
17. The method according to item 15 or 16, wherein the counting step comprises transferring the biological compartments to be counted in an automated manner into one or more counting chambers provided at a filling position of an automated system by pipetting through a filling opening of the counting chamber, wherein the one or more counting chambers are held in position on the automated system by a holding device.
18. The method according to item 17, wherein the holding device holds the one or more counting chambers at the filling position for filling by a pipetting module and wherein after filling, the one or more counting chambers are transferred to a counting position for counting the biological compartments, wherein transfer to the counting position occurs by axially moving the holding device and thereby the held one or more counting chambers.
19. The method according to item 18, wherein the pipetting module is used for axially moving the holding device, wherein preferably the holding device comprises engagement means with which a part of the pipetting module, optionally a pipette tip, is capable of engaging.
20. The method according to item 18 or 19, wherein the holding device for holding the one or more counting chambers is engaged with a sliding device which provides guidance for the axial movement and wherein the holding device is axially movable in relation to the sliding device.
21. The method according to any one of items 18 to 20, wherein at the counting position, the counting chambers are in a position that is accessible for the optical path of an imaging device.
22. The method according to any one of items 15 to 21, wherein cell counting comprises using a counting module, preferably the counting module as defined in any one of items 38 to 49, more preferably an automatic counting module, or a system for counting according to any one of items 70 to 73 and/or wherein the counting method according to any one of items 57 to 69 is used.
23. The method according to one or more of items 1 to 22, in particular items 15 to 22, comprising adjusting the concentration of biological compartments prior to step (a), wherein preferably, the biological compartments are provided in form of a suspension, optionally wherein adjusting comprises adding a dilution solution to the plurality of biological compartments.
24. The method according to item 23, wherein adjusting the concentration fulfills one or more of the following characteristics:
   (i) adjustment is performed to lower the concentration of biological compartments by dilution so that the concentration of biological compartments is within an acceptable range, wherein the acceptable range is preferably predetermined;
   (ii) adjustment occurs after performing the cell counting step;
   (iii) adjustment of the concentration is performed in an automated manner;
   (iv) adjustment of the concentration is performed in a dynamic manner by an automated system using the results of the cell counting step, wherein preferably, the automated system compares the concentration determined as a result of a cell counting step with a predetermined reference value or reference range, wherein if the determined concentration is higher than the reference value of reference range, the automated system adds the required volume of a dilution solution to the plurality of biological compartments to achieve that the plurality of biological compartments are provided in a concentration that is within the predetermined reference value or reference range, wherein preferably the cell counting step is performed according to the method of any one of items 15 to 22;
   (v) wherein the dilution solution is an aqueous solution, wherein the dilution solution optionally corresponds to the liquid surrounding the plurality of biological compartments; and/or
   (vi) wherein the plurality of cells are concentrated using a filter module, preferably the filter module according to one or more of items 74 to 95.
25. The method according to item 23 or 24, said method comprising:
   (a) separating a plurality of biological compartments into a number (n) of aliquots, wherein n is at least 2;
   (b) contacting barcode labels comprising a barcode sequence with each of the n aliquots,
      - wherein the barcode sequence of the barcode labels contacted with a given aliquot is the same, and
      - wherein a different barcode sequence is used for different aliquots, preferably for each of the n different aliquots;
   (c) combining the n aliquots into a pool; and
   (d) repeating steps (a), (b) and (c) using the combined pool,
   wherein optionally, the number (n) of aliquots provided in step (a) are chosen using the results of the cell counting step, wherein preferably, the cell counting step is performed according to the method of any one of items 15 to 22.
26. The method according to item 25, wherein the number (n) of aliquots are determined by an automated system using the results of the cell counting step,
   wherein preferably, the automated system compares the concentration determined as a result of a cell counting step with a predetermined reference value or reference range, wherein if the determined concentration is lower than the reference value of reference range, the automated system separates the plurality of biological compartments into fewer aliquots compared to as when the determined concentration meets the reference value or reference range.
27. The method according to one or more of items 1 to 26, comprising performing one or more mixing steps, preferably using an automated mixing mechanism.
28. The method according to item 27, wherein mixing comprises using an automated mixing mechanism, wherein the automated mixing mechanism comprises the following substeps:
   (i) a mixing element, preferably comprising a pipette tip, is contacted with the sample to be mixed, such as with the plurality of biological compartments which are preferably provided in form of a suspension, and performs an ellipsoidal movement; and
   (ii) a portion of the sample is transferred from position A to position B within the same containment using the mixing element;
   wherein substeps (i) and (ii) can be performed in any order and wherein preferably, substeps (i) and (ii) are repeated one or more times.
29. The method according to item 28, wherein position A and B are chosen differently during repetition.
30. The method according to any one of items 27 to 29, wherein an ellipsoidal movement includes a circular or multi-circular movement, e.g. in form of an 8.
31. The method according to one or more of items 1 to 30, wherein at least one heating step is performed, and wherein during at least one heating step a heating module is used comprising
   - a temperature controlled platform configured for holding containments, wherein the containments are preferably provided as wells of a well plate; and
   - a sealing cover configured so that it can be applied onto the containments for sealing the containments.
32. The method according to item 31, wherein during at least one heating step a heating module according to one or more of items 151 to 161 is used, wherein the heating step is optionally performed by the method according to one or more of items 145 to 150.
33. The method according to one or more of items 1 to 32, further comprising contacting the plurality of biological compartments with at least one adhesion reducing compound.
34. The method according to item 33, having one or more of the following characteristics:
   (i) wherein at least one adhesion reducing compound is in contact with the plurality of biological compartments during washing and/or fluid exchange;
   (ii) wherein at least one adhesion reducing compound is present when the plurality of biological compartments are present in a solution;
   (iii) wherein at least one adhesion reducing compound is included in one or more reaction or processing solutions that are contacted with the biological compartments;
   (iv) wherein containments for receiving biological compartments and/or consumables used for transferring biological compartments are contacted, preferably coated with at least one adhesion reducing compound prior to contact with the plurality of biological compartments; and/or
   (v) wherein the at least one adhesion reducing compound has one or more of the following characteristics:
      (aa) the adhesion reducing compound is a detergent, preferably a non-ionic detergent;
      (bb) the adhesion reducing compound is a detergent selected from the group of polyoxyethylene alkylphenyl ether, polyoxyethylene-polyoxypropylene block copolymers and polyoxyethylene fatty alcohol ether, optionally selected from polyoxamers, such as poloxamer 407 (Pluronic F127) and polyoxyethylene alkylphenyl ethers, such as Triton X100; and/or
      (cc) the at least one adhesion reducing compound, which preferably is a non-ionic detergent is provided at a final concentration of at least 5mg/L, at least 10mg/L, at least 15mg/L or at least 20 mg/L.
35. The method according to one or more of items 1 to 34, wherein the same pipette tip is used for collecting aliquots for pooling step (c).
36. The method according to one or more of items 1 to 35, wherein the biological compartments are selected from cells or cell nuclei.
37. The method according to one or more of items 1 to 36, wherein the target molecules are selected from at least one of RNA, cDNA, DNA, protein, peptide, and antigen, preferably selected from nucleic acids, more preferably selected from RNA or cDNA.
38. A counting module comprising
   - a holding device configured to hold one or more counting chambers; and
   - a sliding device configured to axially direct the holding device.
39. The counting module according to item 38, wherein the holding device is engaged with the sliding device configured to axially direct the holding device.
40. The counting module according to item 38 or 39, wherein the counting module forms part of an automated system that comprises a pipetting module and optionally comprises an imaging device.
41. The counting module according to any one of items 38 to 40, wherein the holding device is in the counting module configured so that it can move axially, wherein moving axially preferably comprises moving the holding device between a filling position and one or more counting positions.
42. The counting module according to any one of items 38 to 41, wherein the holding device is configured so that it can engage with a pipetting module and wherein the holding device can be moved axially by a pipetting module upon engagement.
43. The counting module according to any one of the preceding items, wherein the counting module comprises a sliding device according to any one of items 52 to 56.
44. The counting module according to item 42 or 43, wherein the holding device comprises means configured for engaging with a part of a pipetting module, such as preferably an attached pipetting tip.
45. The counting module according to item 44, wherein the engaging means has one or more of the following characteristics:
   (i) it comprises a contact surface which can be used to exert a force on the holding device in axial direction;
   (ii) it comprises a three dimensional geometry, wherein the geometry is selected from the group comprising cube, tetrahedron, pyramid, prism, octahedron, cylinder, cone, sphere, torus, and combinations thereof;
   (iii) it comprises an opening configured to be engaged with a part of a pipetting module, preferably a pipet tip; and/or
   (iv) it comprises a cylindrical geometry with an opening capable of engaging with part of a pipetting module.
46. The method according to any one of items 42 to 45, wherein the distance between the engaging position of the pipetting module and the counting chamber when mounted to the holder is increased by providing a channel between the filling opening and the counting chambers, wherein the channel is preferably configured to allow the plurality of biological compartments to move through the channel into the counting chambers.
47. The counting module according to one or more of items 38 to 46, wherein the counting module comprises mounted to the holding device one or more counting chambers configured to receive biological compartments through a filling opening.
48. The counting module according to item 47, wherein the counting module has one or more of the following characteristics:
   (i) the counting module comprises more than one counting chamber, wherein each counting chamber is filled by a different filling opening;
   (ii) the holding device is configured to hold the one or more counting chambers in position on an automated system, wherein the holding device gives the filling opening(s) of the one or more counting chambers mounted to the holding device defined coordinates, so that the one or more counting chambers can be filled by pipetting using a pipetting module; and/or
   (iii) wherein more than one counting chamber is provided, configured to receive biological compartments from the plurality of biological compartments, which preferably differ in their concentration by providing different dilutions.
49. The counting module according to any one of items 38 to 48, furthermore comprising an imaging device, preferably a microscope.
50. Use of the counting module according to one or more of items 38 to 49 for counting biological compartments in an automated manner, wherein an imaging device, preferably a microscope takes images of the biological compartments.
51. The use according to item 50, comprising using an algorithm for automatic data evaluation, wherein the algorithm counts the biological compartments in the images.
52. A sliding device for axially directing an object using a pipetting module,
   - wherein the object is configured to engage with the pipetting module.
53. The sliding device according to item 52, wherein the sliding device comprises a sliding frame, which is configured to receive the object, preferably a holding device for holding one or more counting chambers.
54. The sliding device according to item 52 or 53, wherein the sliding device is provided by one or more tracks or rails that guide the movement of the object, preferably a holding device for holding one or more counting chambers.
55. The sliding device according to any one of items 52 to 54, wherein the sliding device is configured to hold a holding device, preferably for holding one or more counting chambers, wherein the sliding device is configured to allow axial movement of the holding device and thus the counting chambers into a position that is accessible for an optical path of an imaging device.
56. The sliding device according to any one of items 52 to 55, wherein the object, preferably a holding device for a counting chamber, is engaged with the sliding device configured to axially direct the object, wherein upon engagement of a pipetting tip of a pipetting module with the object and carrying out a movement in the direction of displacement from the engagement position by the pipetting module, the object is slided axially, guided by the sliding device which preferably provides a sliding frame.
57. A method for counting biological compartments and calculating the number of a plurality of biological compartments by a counting module, preferably an automatic counting module, the method comprising:
   (a) filling one or more counting chambers with biological compartments of the plurality of biological compartments, wherein the one or more counting chambers are mounted to a holding device;
   (b) moving the holding device axially;
   (c) imaging biological compartments present in the one or more counting chambers using an imaging device; and
   (d) optionally automatically counting the biological compartments and calculating the number of the plurality of biological compartments.
58. The method according to item 57, wherein step (a) comprises transferring the biological compartments in an automated manner into the one or more counting chambers provided at a filling position of an automated system by pipetting through a filling opening of a counting chamber using a pipetting module, wherein the one or more counting chambers are held in position on the automated system by the holding device.
59. The method according to item 57 or 58, wherein moving the holding device axially in step (b) is performed by a pipetting module.
60. The method according to any one of items 57 to 59, wherein the holding device is engaged with a sliding device which provides guidance for the axial movement and wherein the holding device is axially movable in relation to the sliding device, wherein preferably the sliding device is a sliding device as defined in any one of items 52 to 56.
61. The method according to any one of items 57 to 60, wherein steps (a) to (c) are performed in consecutive order and wherein after axially moving the holding device in step (b), the counting chambers are in a position accessible for the optical path of the imaging device used in step (c).
62. The method according to any one of items 59 to 61, wherein the holding device comprises engagement means with which a part of the pipetting module, optionally a pipette tip, is capable of engaging.
63. The method according to one or more of items 59 to 62, wherein in step (b) the pipetting module engages with the holding device in particular by the engagement means, followed by axial movement from the filling position of step (a) to a position for imaging in step (c).
64. The method according to item 62 or 63, wherein the distance between the engaging means for the pipetting module and the one or more counting chambers mounted to the holder is chosen such that the pipetting module and the imaging device do not come in contact with each other upon axial movement of the holding device to the position for imaging in step (c).
65. The method according to item 64, wherein the distance between the engaging position of the pipetting module and the counting chamber when mounted to the holder is chosen from the range of 1 to 10 cm.
66. The method according to any one of steps 59 to 65, wherein
   - the pipetting module performs a vertical movement towards the engaging means of the holding device for engaging with the holding device; and/or
   - after step (c) the pipetting module performs an axial movement in the opposite direction as in step (b), to bring the holding device back into the initial position.
67. The method according to one or more of items 57 to 66, wherein the biological compartments are filled into one or more filling openings of the one or more counting chambers at one or more dilution ratios, preferably a dilution series, wherein each dilution ratio is filled into a different filling opening.
68. The method according to one or more of items 57 to 67, wherein prior to step (a) the biological compartments of the plurality of biological compartments are further processed, wherein further processing preferably comprises one or more of the following characteristics:
   (i) the biological compartments of the plurality of biological compartments are transferred into a containment;
   (ii) changing the optical properties of the biological compartments, preferably by staining the biological compartments; and/or
   (iii) staining the biological compartments with a composition comprising trypan blue.
69. The method according to one or more of items 57 to 68, wherein the method comprises one or more of the following characteristics:
   (i) the holding device engages with a sliding device as defined in any one of items 52 to 56, the sliding device preferably comprising a sliding frame;
   (ii) in step (c) the imaging device acquires images, which are used for automatically counting the biological compartments;
   (iii) in step (d) biological compartments are counted automatically, wherein the biological compartments are cells or nuclei, preferably in a suspension;
   (iv) the imaging device has one or more of the following characteristics:
      (aa) it is configured to acquire microscopic images;
      (bb) it comprises a microscope;
      (cc) it comprises a bright field microscope capable of imaging the biological compartments present in the one or more cell counting chambers;
      (dd) it comprises a bright field microscope coupled with illumination;
      (ee) the imaging device or parts of the imaging device are manually or automatically movable; and/or
      (ff) it comprises a microscope configured to move automatically;
   (v) it uses a counting module as defined in any one of items 38 to 49; and/or
   (vi) it uses a counting system as defined in any one of items 70 to 73.
70. A system for counting biological compartments and calculating the number of a plurality of biological compartments, the system comprising:
   - a pipetting module;
   - one or more counting chambers configured to receive biological compartments of the plurality of biological compartments through a filling opening;
   - a holding device configured to hold the one or more counting chambers;
   - a sliding device configured to axially direct the holding device using a pipetting module; and
   - optionally an imaging device.
71. The system according to item 70, wherein the system further comprises a processing unit and a program for automatically adjusting the biological compartment concentration.
72. The system according to item 71, wherein the automatically adjustment comprises one or more of the following features:
   (i) liquid is added to the plurality of biological compartments;
   (ii) the plurality of cells are concentrated using a filter module, preferably the filter module as defined in any one of items 74 to 95;
   (iii) liquid surrounding the plurality of biological compartments is exchanged or separated from the plurality of biological compartments using a filter module as defined in any one of items 74 to 95, wherein preferably the exchanged liquid or liquid remaining after separating has a lower volume than the initial liquid surrounding the biological compartments; and/or
   (iv) transferring aliquots of the plurality of biological compartments comprises transferring less aliquots.
73. The system according to any one of items 70 to 72, wherein the system has one or more of the characteristics:
   (i) the at least one counting chamber is configured to receive biological compartments through a filling opening and/or is provided by a hemocytometer;
   (ii) the holding device has one or more of the characteristics as defined in any one of items 39 to 48;
   (iii) the sliding device has one or more of the characteristics as defined in any one of items 52 to 56; and/or
   (iv) the imaging device has one or more of the characteristics as defined in any one of items 49 or 69 (iv).
74. A filter module for exchanging a liquid surrounding a plurality of objects, preferably biological compartments, or for separating a liquid from a plurality of objects, preferably biological compartments, the filter module comprising:
   - a feed portion, an effluent portion and a filter element, preferably a membrane, wherein the filter element is provided between the feed portion and the effluent portion;
   - wherein the feed portion or the effluent portion is configured to be connected to a device capable of generating a pressure differential; and
   - wherein the feed portion is configured such that liquid and/or liquid comprising a plurality of biological compartments can be fed into the feed portion.
75. The filter module according to item 74, wherein the feed portion is provided as top part and wherein the effluent portion is provided as bottom part of the filter module.
76. The filter module according to item 74 or 75, wherein in connection with the filter element the feed portion forms a containment, into which a plurality of biological compartments can be filled, optionally wherein the containment is dimensioned to receive a volume of 25mL or less, 20mL or less, 15mL or less or 10mL or less.
77. The filter module according to any one of items 74 to 76, wherein the feed portion of the filter module has one or more of the following characteristics:
   (i) the feed portion has an elongated body portion;
   (ii) the feed portion has a longitudinal axis that intersects the filter element, preferably a membrane; and/or
   (iii) wherein an opening of the feed portion is spaced apart from the filter element.
78. The filter module according to one or more of items 74 to 77, wherein the effluent portion of the filter module has one or more of the following characteristics:
   (i) the effluent portion has an elongated body portion;
   (ii) the effluent portion has a longitudinal axis that intersects the filter element;
   (iii) an opening of the effluent portion is spaced apart from the filter element;
   (iv) the effluent portion, preferably provided as bottom part, is configured to be connected to a tubing;
   (v) a first opening of the effluent portion is configured to be connected to a device capable of generating a pressure differential; and/or
   (vi) the effluent portion, preferably the opening of the effluent portion, is connected via a tubing to the device.
79. The filter module according to one or more of items 74 to 78, wherein the feed portion is a component being connectable or being connected to the effluent portion and wherein the filter element is secured between the feed portion and effluent portion.
80. The filter module according to one or more of items 74 to 79, having one or more of the following characteristics:
   (i) the feed portion and effluent portion are connected forming an elongated body, wherein the elongated body comprises a contact surface capable of holding a sealing mean/filter element holder; and/or
   (ii) wherein the feed portion, preferably provided as top part, and the effluent portion, preferably provided as bottom part, are configured to be connected by a frictional fit, form fit and/or adhesive bonding, optionally selected from screwing, clamping and snap-fit assembly.
81. The filter module according to one or more of items 74 to 80, wherein the filter module comprises an elongated body comprising a feed portion, preferably provided as top part, and an effluent portion, preferably provided as bottom part, configured to be connected and secure a filter element, preferably a membrane, between the feed portion and the effluent portion, optionally wherein securing includes one or more sealing means/filter element holders.
82. The filter module according to one or more of items 74 to 81, wherein the filter element, preferably a membrane, is secured between the feed portion and the effluent portion by using at least one sealing means or at least one filter element holder between the filter element and the feed portion and/or the filter element and the effluent portion.
83. The filter module according to one or more of items 74 to 82, wherein a first sealing means or filter element holder is provided between the filter element and the feed portion, the first sealing means or filter element holder being in contact with a first surface of the filter element and a second sealing means or filter element holder is in contact with the second surface of the filter element, being the surface opposite the first surface.
84. The filter module according to item 82 or 83, wherein the sealing means or filter element holder comprises a structural element, the structural element being adapted to interact with the filter element, preferably for holding or positioning the filter element with regard to the sealing means/filter element holder.
85. The filter module according to item 84, wherein the structural element is a reception for the filter element, which preferably is a membrane, such that the sealing means or filter element holder at least partially surrounds the filter element.
86. The filter module according to item 84 or 85, wherein the feed portion and one of the sealing means or filter element holder each comprise a structural element, the structural elements being adapted to interact with each other, preferably for holding or positioning the sealing means/filter element holder by or to the feed portion.
87. The filter module according to any one of items 84 to 86, wherein the effluent portion and one of the sealing means or filter element holder each comprise a structural element, the structural elements being adapted to interact with each other, especially for holding or positioning the sealing means/filter element holder by or to the effluent portion.
88. The filter module according to one or more of items 74 to 87, wherein the filter element, which preferably is a membrane, has a mean pore size which is smaller than the size of the biological compartments to be filtered.
89. The filter module according to one or more of items 74 to 88, wherein the filter element has one or more of the following characteristics:
   (i) it is a surface filter, such that the biological compartments can be collected at the surface of the filter element;
   (ii) it is configured to substantially retain the plurality of biological compartments;
   (iii) it is capable of at least partially separating the plurality of biological compartments form the liquid surrounding the biological compartments;
   (iv) it has a mean pore size ranging from 0.01 µm to 30 µm, preferably less than 15 µm, less than 10 µm, less than 8 µm, more preferably less than 3 µm or approximately 0.1 µm;
   (v) it is provided by a membrane;
   (vi) it is provided by a membrane which comprises or consists of a film or foil having pores or holes of a defined mean pore size; and/or
   (vii) wherein the membrane is a track etched membrane, preferably having a pore size of less than 8 µm, less than 3 µm, more preferably approximately 0.1 µm or less.
90. The filter module according to one or more of items 74 to 89, wherein the filter module, preferably the bottom part of the filter module, comprises a stand or a recess onto which the filter module can stably stand and/or a clamping or screwing mechanism is provided to the bottom part of the filter module suitable to stably hold the filter module during pipetting operations.
91. The filter module according to one or more of items 74 to 90, comprising or being connected to a device capable of generating a pressure differential, wherein the device capable of generating a pressure differential is a syringe pump, wherein preferably the effluent portion of the filter module is connected to the syringe pump.
92. The filter module according to one or more of items 74 to 91, being implemented into an automated system, preferably an automated system as defined in any one of items 163 to 179.
93. The filter module according to one or more of items 74 to 92, wherein the operation of the filter module is controlled in an automated manner, wherein in particular the pressure differential is controlled in an automated manner.
94. The filter module according to one or more of items 74 to 93, wherein a control unit is provided which is adapted to apply a pressure differential, especially the control unit being in functional connection with the device capable of generating a pressure differential.
95. The filter module according to item 94, wherein the control unit has one or more of the following characteristics:
   (i) it is part of the device capable of generating a pressure differential;
   (ii) it is a unit which is functionally connected to or part of a control unit for controlling feed of the liquid and/or biological compartments;
   (iii) the control unit and/or the device capable of generating a pressure differential are/is in functional connection with a pressure sensor; and/or
   (iv) it is configured to allow a liquid in the feed portion before and after filtering.
96. Use of a filter module according to any one of items 74 to 95 for exchanging a liquid surrounding a plurality of biological compartments or for separating a liquid from a plurality of biological compartments, wherein the filter element is used to filter the plurality of biological compartments.
97. The use according to item 96, wherein exchanging a liquid surrounding a plurality of biological compartments or separating a liquid from a plurality of biological compartments is performed in a split-and-pool workflow, preferably is performed one or more times in the method according to any one of items 1 to 37.
98. A method for removing and preferably exchanging a liquid surrounding a plurality of objects, preferably biological compartments or for separating a liquid from a plurality of objects, preferably biological compartments, the method comprising:
   - feeding the liquid comprising the plurality of objects, preferably biological compartments, into a feed portion, which is separated from an effluent portion by a filter element, preferably a membrane,
   - generating a pressure differential is by applying an overpressure to the feed portion and/or by applying a negative pressure to the effluent portion,
   wherein preferably, the filter module as defined in any one of items 74 to 95 is used.
99. The method according to item 98, for exchanging a liquid surrounding a plurality of biological compartments using the filter module as defined in any one of items 74 to 95, wherein at least a part of the liquid surrounding the plurality of biological compartments is separated by the generated pressure differential and wherein after separation, the method comprises contacting and mixing the plurality of biological compartments with another liquid; wherein optionally, the separation and contacting steps are repeated.
100. The method according to item 98 or 99, wherein a syringe pump is used for generating the pressure differential, wherein the syringe pump removes a defined volume of the liquid, wherein preferably, the syringe pump generates a low/negative pressure to suck a defined volume of the liquid through the filter element.
101. The method according to any one of items 98 to 100, wherein the liquid surrounding the plurality of objects, preferably biological compartments comprises one or more compounds selected from one or more of the following:
   - one or more compounds present during or after fixing a plurality of biological compartments;
   - one or more compounds present after performing a reverse transcription reaction; and/or
   - one or more compounds present after performing a ligase reaction.
102. The method according to one or more of items 98 to 101, wherein the method is performed after performing a reverse transcription reaction and/or after attachment of a barcode label.
103. The method according to one or more of items 98 to 102, wherein the method is performed in an automated manner.
104. A sieving module, preferably for removing objects, such as aggregates from a plurality of biological compartments, the sieving module comprising:
   - an inlet and a lateral outlet of a fluid path;
   - and a sieve, wherein the sieve is provided in the fluid path;
      - wherein the inlet and the lateral outlet are configured such that a fluid can pass the inlet and the lateral outlet; and
      - wherein the lateral outlet is directed such that the fluid which passed the sieve is laterally directed with regard to the inlet.
105. The sieving module according to item 104, wherein the sieving module comprises a passage below the sieve that allows to laterally direct the fluid, preferably comprising a plurality of biological compartments, towards the lateral outlet.
106. The sieving module according to item 104 or 105, wherein the sieving module is provided as one part or as two or more parts, wherein the two or more parts are provided in an assembled state or in a state wherein it is assembled by the user.
107. The sieving module according to any one of items 104 to 106, wherein the sieving module comprises a receptacle.
108. The sieving module according to item 107, wherein the receptacle
   (i) provides the inlet;
   (ii) comprises an inlet and an outlet;
   (iii) comprises an inlet and an outlet and a sieve between the inlet and the outlet; and/or
   (iv) comprises a hollow, elongated body and optionally has a cylindrical shape.
109. The sieving module according to item 107 or 108, wherein the sieving module comprises a receptacle comprising two openings and a sieve, wherein the sieve is provided between the two openings, wherein the openings are configured such that the fluid, preferably comprising the plurality of biological compartments in singularized form, can pass the openings.
110. The sieving module according to any one of items 107 to 109, wherein the receptacle comprises the sieve and comprises means for laterally directing the plurality of biological compartments after sieving to the lateral outlet.
111. The sieving module according to any one of items 107 to 110, wherein the lateral outlet is provided by the receptacle.
112. The sieving module according to one or more of items 107 to 110, wherein the sieving module comprises a receptacle and a holder for holding the receptacle, the holder being configured to laterally direct a fluid passing the held receptacle.
113. The sieving module according to item 112, wherein the holder comprises a receptable for receiving the receptacle.
114. The sieving module according to any one of items 112 to 113, wherein the lateral outlet is provided by the holder which is adapted to hold the receptacle and establishing a fluid path between the receptacle and the holder, wherein the holder is configured to laterally direct the plurality of biological compartments passing the receptacle outlet to the lateral outlet provided by the holder.
115. The sieving module according to item 112 or 114, wherein the holder comprises a passage configured such that the fluid passing the sieve located inside a receptacle is directed by the passage towards a lateral direction, wherein the lateral direction extends to the lateral outlet.
116. The sieving module according to any one of items 112 to 115, wherein the holder receives a receptacle comprising a sieve and wherein fluid exiting the receptacle is received by the holder and is laterally directed by a passage configured to laterally direct a fluid, wherein preferably, the passage is tilted with regard to the sieve to simplify the flow of the sieved fluid through the passage to the lateral outlet.
117. The sieving module according to one or more of items 104 to 116, wherein the lateral outlet is an outlet which confines an angle between the longitudinal axis and the outlet which is different from 0°, especially greater than 10°, greater than 20°, greater than 30°, greater than 40°, greater than 50°, greater than 60°, greater than 70°, or greater than 80°.
118. The sieving module according to one or more of items 104 to 117, wherein the laterally directed fluid is accessible by a robotic pipetting module without transfer or movement of the sieving module.
119. The sieving module according to one or more of items 104 to 118, wherein the sieve is held by one or more sealings or sieve holders, wherein preferably the receptacle is configured to hold the sieve by providing a sieve between two sealing or sieve holders which are inserted into the receptacle.
120. The sieving module according to one or more of items 104 to 119, wherein the sieve has one or more of the following characteristics:
   (i) it has a mean mesh size, which is larger than the size of the plurality of biological compartments and smaller than the undesired aggregates, wherein the aggregates are retained by the sieve;
   (ii) it has a mean mesh size selected from the range of 5 µm to 200 µm, preferably from the range of 10 to 150 µm, 20 to 100 µm or 30 to 80 µm;
   (iii) it has a mean mesh size of approximately 40 µm; and/or
   (iv) it is made of nylon or PET.
121. The sieving module according to any one of items 104 to 120, wherein the sieving module is a consumable which comprises a recess or pedestal for arranging the sieving module on a containment, preferably a well of a well plate.
122. The sieving module according to item 121, wherein the receptacle of the sieving module or the holder of the sieving module, if the sieving module comprises a holder, comprises a pedestal being adapted to fit to an array of containments, preferably a well plate, such that the receptacle or the holder can be positioned on a containment, preferably a well of a well plate, and wherein the fluid passing the sieve is directed into a neighboring containment, preferably a neighboring well of a well plate.
123. The sieving module according to item 121 or 122, wherein the holder comprises a recess for holding the holder on top of a containment.
124. The sieving module according to one or more of items 112 to 123, wherein the holder is a holder as defined in any one of items 125 to 128.
125. A holder for holding a receptacle, wherein the holder is configured to laterally direct a fluid, preferably comprising a plurality of biological compartments, passing the receptacle.
126. The holder according to item 125, wherein the holder comprises a receptable for receiving the receptacle.
127. The holder according to item 125 or 126, wherein the holder comprises a passage, wherein the passage has one or more of the following characteristics:
   (i) it is configured to laterally direct a fluid passing the held receptacle, optionally to a laterally provided containment;
   (ii) it is provided below the outlet of the held receptacle;
   (iii) it is configured such that a fluid passing the held receptacle, is directed by the passage towards a lateral direction, wherein the passage extends to a lateral outlet;
   (iv) it directs the sieved fluid into an adjacent containment or reservoir;
   (v) it is tilted to support the lateral flow of the fluid;
   (vi) it has a geometry selected from ellipsoidal, round, rectangular or irregular shapes;
   (vii) it has the shape of a channel, wherein the channel is preferably at least in parts fully surrounded by the holder material; and/or
   (viii)it corresponds to a groove, wherein the groove preferably has a half ellipsoidal surface area.
128. The holder according to any one of items 125 to 127, wherein the holder has one or more of the following characteristics:
   (i) it is configured to laterally direct the fluid, preferably comprising a plurality of biological compartments, passing an outlet of the receptacle;
   (ii) it comprises a pedestal or recess, preferably for holding the holder on top of a containment;
   (iii) it comprises a pedestal as defined in item 121 or 122;
   (iv) it comprises a contact surface for contacting a portion of the receptacle to thereby hold the receptacle;
   (v) it comprises a cylindrical opening and a narrowing section configured to engage with the bottom portion of the receptacle, wherein the receptacle outlet is not directly contacted; and/or
   (vi) wherein the receptacle is a hollow, elongated body, preferably comprising a sieve element.
129. A sieving system for sieving a fluid using a sieving module, preferably for removing aggregates from a plurality of biological compartments, the sieving system comprising:
   - two openings of a fluid path, each opening being accessible from substantially the same direction such that especially the fluid path can be accessed by one pipetting module without transfer or movement of the sieving module.
130. The system according to item 129, wherein the sieving system comprises a sieving module as defined in any one of items 104 to 124.
131. A sieving system for sieving a fluid, preferably for removing aggregates from a plurality of biological compartments, the sieving system comprising:
   - a holder for holding a receptacle, wherein the holder is configured to laterally direct a fluid, preferably comprising a plurality of biological compartments, passing the receptacle; and
   - a receptacle comprising a hollow body and a sieve located therein.
132. The system according to item 131, wherein the holder is a holder as defined in any one of items 125 to 128.
133. The system according to items 131 or 132, wherein the holder and the receptacle form part of a sieving module as defined in any one of items 104 to 124.
134. The system according to any one of items 129 to 133, wherein the system further comprises one or more containments, preferably an array of containments, more preferably a well plate.
135. The system according to item 134, wherein the system comprises an array of containments.
136. The system according to item 135, wherein the holder is provided onto or is suitable to be provided onto the array of containments, optionally wherein when the holder is provided onto the array of containments, it may either form a connection with a containment or is held by other means to be provided into or onto the array of containments.
137. The system according to item 136, wherein the holder is configured such that when the holder is located in or on one containment of the array, a further containment is arranged laterally and wherein the fluid that is laterally directed by the holder is directed into the lateral containment.
138. The system according to any one of items 129 to 137, wherein the sieving system can be operated in an automatic manner and preferably is an automated system.
139. Use of the sieving module according to any one of items 104 to 124 or the sieving system according to any one of items 129 to 138 for removing objects.
140. Use according to item 139, for removing aggregates of biological compartments from a plurality of biological compartments comprised in a liquid, preferably aggregates of cells and/or nuclei, in processing protocols, in particular in split-and-pool workflows, preferably in a method according to any one of items 1 to 37.
141. Use according to item 140, for removing aggregates from a suspension comprising a plurality of biological compartments, preferably cells.
142. A method for sieving a fluid, preferably for removing aggregates from a plurality of biological compartments, using a sieving module according to any one of items 104 to 124 or a sieving system according to any one of items 129 to 138, comprising the steps of:
   (a) adding a fluid, preferably comprising a plurality of biological compartments, through an inlet, whereby the fluid passes the sieve, wherein larger objects, such as aggregates, do not pass the sieve; and
   (b) laterally directing the sieved fluid, preferably comprising a plurality of biological compartments, through a lateral outlet.
143. The method according to item 142, wherein the method comprises loading the fluid to be sieved using a pipetting module of an automated system and wherein the sieved fluid is laterally directed to a lateral position, preferably an adjacent containment, wherein the lateral position is accessible by a pipetting module.
144. The method according to item 142 or 143, wherein the method comprises one or more of the following characteristics:
   (i) it is performed in an automated manner;
   (ii) it is performed in the method according to any one of items 1 to 37 after pooling step (c) and/or after fixing the biological compartments;
   (iii) it is performed after/during fixation and permeabilization a plurality of biological compartments in order to remove aggregates;
   (iv) the sieved fluid that is laterally directed in step (b) comprises predominantly a plurality of singularized biological compartments; and/or
   (v) after step (b), a pipetting module directly takes up the sieved fluid, without removing the sieving module or sieving system.
145. A method for heating containments, preferably wells of a well-plate, the method comprising:
   (a) subjecting the containments to a temperature controlled platform configured for holding the containments, wherein the containments are preferably provided as wells of a well plate;
   (b) sealing the containments by adding a sealing cover, which is preferably a flexible mat; and
   (c) heating the containments.
146. The method according to item 145, wherein in step (b) axial and preferably vertical movements are performed in an automated manner, in particular using a pipetting module, for adding the sealing cover.
147. The method according to item 145, wherein steps (b) and (c) are performed automatically, preferably comprising one or both of the following characteristics:
   (i) wherein the sealing cover is provided using a holding device and a sliding device, wherein the holding device can be axially moved, preferably wherein the sliding device is a sliding device according to one or more of items 52 to 56; and/or
   (ii) wherein axial movement is induced by a pipetting module, wherein preferably engagement means are configured to engage with a portion of the pipetting module, preferably the pipetting tip, wherein engaging means are preferably engaging means according to one or more of items 44 to 45.
148. The method according to any one of items 145 to 147, wherein after step (c) the sealing cover is removed, preferably removed automatically, optionally wherein removing comprises one or both of the following characteristics:
   (i) the sealing cover is vertically moved, in particular the sealing cover is lifted; and/or
   (ii) the sealing cover is moved axially, wherein axial movement is preferably performed using a pipetting module, and wherein axial movement is preferably performed after a vertical movement.
149. The method according to one or more of items 145 to 148, wherein the method comprises one or more, preferably all of the following characteristics:
   (i) in step (b) axially moving the sealing cover above the containments;
   (ii) in step (b) axially moving the sealing cover by a pipetting module, wherein a holding device holds the sealing cover, wherein holding preferably comprises providing a sealing cover in direction of the containments when provided above the containments;
   (iii) in step (b), vertically moving the sealing cover to provide the sealing cover onto the containments, preferably by mechanical or electromechanical actuators, more preferably using a lifting magnet, wherein vertically moving preferably is performed after performing an axial movement;
   (iv) optionally, after step (c) vertically moving the sealing cover and, preferably axially moving the sealing cover, to remove the sealing cover from the containments.
150. The method according to item 149, wherein axially moving comprises using a holding device and a sliding device according to one or more of items 38 to 56.
151. A heating module, such as a cycling module, comprising
   - a temperature controlled platform configured for holding containments, wherein the containments are preferably provided as wells of a well plate; and
   - a sealing cover configured so that it can be applied onto the containments for sealing the containments.
152. The heating module according to item 151, wherein the heating module is automatically lockable/closable.
153. The heating module according to item 151 or 152, wherein the sealing cover is applied onto the containments by axially and preferably by vertically moving the sealing cover, preferably by using a pipetting module.
154. The heating module according to any one of items 151 to 153, wherein the heating module further comprises components to perform axial and optionally vertical movements, wherein the components may have one or more of the following features:
   (i) one component corresponds to a device for performing an axial movement, preferably comprising a holding device and a sliding device, preferably wherein the axial movement is performed using the pipetting module which optionally comprises a pipetting tip;
   (ii) one components corresponds to a device for performing a vertical movement, preferably wherein a device for performing a vertical movement comprises a mechanical or electromechanical actuation, preferably a lifting magnet;
   (iii) wherein components to perform an axial movement comprise a holding device and a sliding device, preferably a holding device and a sliding device according to one or more of items 38 to 56; and/or
   (iv) the components are provided as one component capable of performing axial and vertical movements.
155. The heating module according to one or more of items 151 to 154, wherein the heating module has one or more of the following characteristics:
   (i) wherein the heating module is controlled in an automated manner, wherein in particular the temperature and the sealing of the containments by a sealing cover is controlled in an automated manner;
   (ii) wherein a control unit is provided which is adapted to control the temperature, the pipetting module, and/or a device for performing a vertical movement;
   (iii) wherein the control unit can be part of the device;
   (iv) wherein the control unit is a unit which is functionally connected to or part of the automatically lockable heating module; and/or
   (v) wherein two, three or four heating modules are provided that are automatically lockable/closable by two, three or four holders and sliding devices or by less holders and sliding devices, preferably one holder and sliding device.
156. The heating module according to one or more of items 151 to 155, wherein the sealing cover has one or more of the following characteristics:
   (i) it is a flexible mat, preferably configured to seal the containments when contacting the containments;
   (ii) it comprises protrusions which extend into the containments such as the wells for sealing;
   (iii) it is flat or structured;
   (iv) it is a flexible mat, wherein the mat is flat or structured; and/or
   (v) it comprises engagement means, wherein the engagement means are configured to engage with a portion of the pipetting module, preferably the pipetting tip.
157. The heating module according to one or more of items 151 to 156, wherein the sealing cover is a flexible mat, wherein the flexible mat seals predominantly all of the containments, wherein the containments are arranged in form of a well plate.
158. The heating module according to one or more of items 151 to 157, wherein the containments are arranged in form of a well plate (e.g. 24, 48, 96, 384 wells) and the sealing cover seals predominantly all of the containments.
159. The heating module according to one or more of items 151 to 158, wherein the heating module further comprises a cover plate.
160. The heating module according to one or more of items 151 to 159, wherein the cover plate is held by the holder, preferably at the bottom of the holder, wherein the sealing cover is attached to the cover plate.
161. The heating module according to one or more of items 151 to 160, wherein the cover plate has one or more of the following features:
   (i) it is in contact with the sealing cover, wherein the contact is permanent or reversible;
   (i) it holds the sealing cover, preferably a flexible mat;
   (ii) it is a mechanically rigid plate;
   (iii) it is a metal plate, preferably an alumina plate;
   (iv) it comprises engagement means, wherein the engagement means are configured to engage with a portion of the pipetting module, preferably the pipetting tip;
   (v) facilitates the maintenance of the temperature adjusted by the temperature controlled platform; and/or
   (vi) it is temperature controlled.
162. Use of a heating module as defined in any one of items 151 to 161 for heating containments for performing a reverse transcription and/or a ligation reaction.
163. An automated system, the system comprising one or more of the following modules:
   - a pipetting module;
   - one or more filter modules for exchanging a liquid surrounding a plurality of biological compartments or for separating a liquid from a plurality of biological compartments;
   - optionally, one or more sieving modules for removing objects, such as aggregates from a plurality of biological compartments;
   - optionally, a counting module;
   - optionally, a cooling module; and
   - optionally, a heating module.
164. An automated system comprising two or more of the following elements
   - a pipetting module;
   - at least one filter module as defined in any one of items 74 to 95;
   - at least one sieving module as defined in any one of items 104 to 124;
   - at least one counting module as defined in any one of items 38 to 49; and/or
   - at least one sliding device as defined in any one of items 52 to 56.
165. The system according to item 164, further comprising a heating module and/or a cooling module.
166. The system according to item 164 or 165, wherein the automated system comprises
   - a pipetting module;
   - at least one filter module as defined in any one of items 74 to 95; and
   - at least one sieving module as defined in any one of items 104 to 124.
167. The system according to any one of items 164 to 166, wherein the automated system comprises
   - a pipetting module;
   - at least one counting module as defined in any one of items 74 to 95; and
   - at least one sliding device as defined in any one of items 104 to 124.
168. The system according to any one of items 164 to 167, wherein the automated system comprises
   - a pipetting module;
   - at least one filter module as defined in any one of items 74 to 95; and
   - at least one counting module as defined in any one of items 38 to 49; and/or at least one sliding device as defined in any one of items 52 to 56.
169. The system according to any one of items 164 to 168, wherein the automated system comprises
   - a pipetting module; and
   - at least one sieving module as defined in any one of items 104 to 124.
170. The system according to any one of items 164 to 169, wherein the automated system comprises
   - a pipetting module;
   - at least one filter module as defined in any one of items 74 to 95;
   - at least one sieving module as defined in any one of items 104 to 124; and
   - at least one counting module as defined in any one of items 38 to 49.
171. The system according to any one of items 164 to 170, wherein the automated system comprises
   - a pipetting module;
   - at least one filter module as defined in any one of items 74 to 95;
   - at least one sieving module as defined in any one of items 104 to 124; and
   - at least one sliding device as defined in any one of items 52 to 56, optionally wherein the sliding device forms part of a counting module as defined in any one of items 38 to 49.
172. The system according to any one of items 163 to 171, comprising a filter module as defined in any one of items 74 to 95 and wherein the automated system has one or more of the following characteristics:
   (i) the filter module is connected to a device capable of generating a pressure differential, wherein preferably the effluent portion of the filter module is connected to the device;
   (ii) the filter module is connected to a syringe pump, wherein preferably the effluent portion of the filter module is connected to the syringe pump;
   (iii) the pressure differential applied to the filter module is controlled in an automated manner;
   (iv) the automated system comprises a control unit which is adapted to apply a pressure differential, optionally wherein the control unit has one or more of the following characteristics:
      (aa) it is in functional connection with the device capable of generating a pressure differential;
      (bb) it is part of the device capable of generating a pressure differential;
      (cc) it controls the feed of a liquid, preferably comprising biological compartments, to the filter module;
      (dd) it is configured to allow a liquid in the feed portion before and after filtering;
      (ee) the control unit and/or the device capable of generating a pressure differential are/is in functional connection with a pressure sensor;
   (v)the filter element has one or more of the following characteristics:
      - it is provided by a membrane which comprises or consists of a film or foil having pores or holes of a defined mean pore size; and/or
      - it is provided by a membrane, wherein the membrane is a track etched membrane, preferably having a pore size of less than 8 µm, less than 3 µm, more preferably approximately 0.1 µm or less; and/or
   (vi) the automated system comprises two or more filter modules as defined in any one of items 74 to 95.
173. The system according to any one of items 163 to 172, comprising a sieving module as defined in any one of items 104 to 124 and wherein the automated system has one or more of the following characteristics:
   (i) the automated system is configured so that a pipetting module transfers a fluid to the inlet of the sieving module and wherein a pipetting module can retrieve the sieved fluid without removing the sieving module;
   (ii) the comprised sieving module directs the sieved fluid to a position that is lateral to the sieving module, wherein preferably the sieved fluid is directed to a lateral containment, preferably a well of a well-plate that is located adjacent to a well that comprises the sieving module; and/or
   (iii) the automated system comprises two or more sieving modules as defined in any one of items 104 to 124.
174. The system according to any one of items 163 to 173, comprising a counting module as defined in any one of items 38 to 49 and wherein the automated system has one or more of the following characteristics:
   (i) it comprises an imaging device, preferably a microscope;
   (ii) the holding device of the counting module comprises means configured for engaging with a part of a pipetting module, such as preferably an attached pipetting tip;
   (iii) the automated system is configured so that a pipetting module can engage with the holding device of the counting module and wherein the holding device is moved axially by a pipetting module upon engagement; and/or
   (iv) it comprises two or more counting modules as defined in any one of items 38 to 49.
175. The system according to any one of items 163 to 174, comprising a heating module, optionally wherein the heating module is a heating module according to one or more of items 151 to 161.
176. The system according to one or more of items 163 to 175, wherein the system further comprises a processing unit, wherein the processing unit is provided by a separate unit or may be integrated into the pipetting module, filter module, counting module and/or heating module.
177. The system according to one or more of items 163 to 176, wherein the automated system is controlled by an algorithm for automating a method.
178. The system according to item 176 or 177, wherein the modules have an interface with the processing unit, wherein the interface is preferably an electronic interface.
179. The system according to any one of items 176 to 178, wherein the automated system comprises one or more filter modules and further comprise a device capable of generating a pressure differential, preferably a syringe pump, wherein the device has an interface with the processing unit.
180. Use of the automated system according to any one of items 163 to 179 for performing a split-and-pool-workflow in an at least partially automated manner.
181. Use of the automated system according to any one of items 163 to 179 for performing the method according to any one of items 1 to 37.
182. Use of the system according to any one of items 163 to 179 for labeling target molecules within a plurality of biological compartments in an at least partially automated manner, wherein labeling target molecules within a plurality of biological compartments is preferably performed by a method according to any one of items 1 to 37.

As used herein, the term "comprising" is to be construed as encompassing both "including" and "consisting of", both meanings being specifically intended, and hence individually disclosed embodiments in accordance with the present invention.

This invention is not limited by the exemplary method disclosed herein, and any methods, uses, systems and materials similar or equivalent to those described herein can be used in the practice or testing of embodiments of this invention. Numeric ranges are inclusive of the numbers defining the range. The headings provided herein are not limitations of the various aspects or embodiments of this invention which can be read by reference to the specification as a whole.

As used in the subject specification, the singular forms "a", "an" and "the" include plural aspects unless the context clearly dictates otherwise. Reference to "the disclosure" and "the invention" and the like includes single or multiple aspects taught herein; and so forth. Aspects taught herein are encompassed by the term "invention".

It is preferred to select and combine preferred embodiments described herein and the specific subject-matter arising from a respective combination of preferred embodiments also belongs to the present disclosure.

## Claims

1. A method for labeling target molecules within a plurality of biological compartments with a combination of barcode labels, the method comprising:
(a) separating a plurality of biological compartments into at least two aliquots;
(b) contacting barcode labels comprising a barcode sequence with the at least two aliquots comprising biological compartments,
- wherein the barcode sequence of the barcode labels contacted with a given aliquot is the same, and
- wherein a different barcode sequence is used for different aliquots;
(c) combining aliquots comprising biological compartments into a pool; and
(d) repeating steps (a), (b) and (c) using the combined pool,
and wherein the method comprises performing at least one of the following steps:
(i) separating biological compartments from a liquid and/or concentrating biological compartments within a liquid in an automated manner using a filter module which comprises
∘ a feed portion, an effluent portion and a filter element, wherein the filter element is provided between the feed portion and the effluent portion;
∘ wherein the feed portion or the effluent portion is configured to be connected to a device capable of generating a pressure differential; and
∘ wherein the feed portion is configured such that liquid comprising a plurality of biological compartments can be fed into the feed portion;
and wherein separating and/or concentrating comprises feeding a liquid comprising the plurality of biological compartments into the feed portion of the filter module and generating a pressure differential, whereby liquid passes the filter element and biological compartments are retained at the filter element;
and/or
(ii) removing aggregates of biological compartments in an automated manner by passing a fluid comprising biological compartments through a sieving module, wherein the sieving module comprises:
∘ an inlet and a lateral outlet of a fluid path;
∘ and a sieve, wherein the sieve is provided in the fluid path;
∘ wherein the inlet and the lateral outlet are configured such that a fluid can pass the inlet and the lateral outlet; and
∘ wherein the lateral outlet is directed such that the fluid which passes the sieve is laterally directed with regard to the inlet.

2. The method according to claim 1, wherein the automated separating and/or concentrating step is performed and has one or more of the following characteristics:
(i) it is performed after performing a reverse transcription reaction and/or after attachment of a barcode label;
(ii) a syringe pump is used for generating the pressure differential, wherein the syringe pump removes a defined volume of the liquid, wherein preferably, the syringe pump is connected to the effluent portion of the filter module and generates a negative pressure to suck a defined volume of the liquid through the filter element; and/or
(iii) it is performed for exchanging a liquid surrounding a plurality of biological compartments, wherein at least a part of the liquid surrounding the plurality of biological compartments is separated by the generated pressure differential and wherein after separation, the method comprises contacting and mixing the plurality of biological compartments with another liquid; wherein optionally, the separation and contacting steps are repeated.

3. The method according to claim 1 or 2, wherein the automated separating and/or concentrating step is performed and wherein the used filter module has one or more of the following characteristics:
(i) the filter module comprises an elongated body comprising a feed portion, preferably provided as top part, and an effluent portion, preferably provided as bottom part, configured to be connected and secure a filter element between the feed portion and the effluent portion, optionally wherein securing includes one or more sealing means/filter element holders;
(ii) in connection with the filter element the feed portion of the filter module forms a containment into which a plurality of biological compartments can be filled, optionally wherein the containment is dimensioned to receive a volume of 25mL or less, 20mL or less, 15mL or less or 10mL or less;
(iii) the filter element of the filter module has one or more of the following characteristics:
(aa) it is a surface filter, such that the biological compartments can be retained and collected at the surface of the filter element;
(bb) it is configured to substantially retain the plurality of biological compartments;
(cc) it is provided by a membrane;
(dd) it is provided by a membrane which comprises or consists of a film or foil having pores or holes of a defined mean pore size; and/or
(ee) it is provided by a track etched membrane, preferably having a pore size of less than 8 µm, less than 3 µm, more preferably approximately 0.1 µm or less; and/or
(iv) the filter module is a filter module as defined in claim 24.

4. The method according to any one of claims 1 to 3, wherein the automated aggregate removal step is performed and has one or more of the following characteristics:
(i) it is performed after pooling step (c) and/or after fixing the biological compartments;
(ii) the sieving module is placed onto or in a containment on an array of containments, optionally a well of a well-plate, and laterally passes the sieved fluid into a neighboring containment, optionally a neighboring well of a well-plate;
(iii) it comprises loading the sieving module with the fluid to be sieved using a pipetting module of an automated system, wherein the sieved fluid is laterally directed by the sieving module to a lateral position, preferably an adjacent containment, and wherein the lateral position is accessible by a pipetting module without removal of the sieving module; and/or
(iv) after laterally directing the sieved fluid through the lateral outlet, a pipetting module directly takes up the sieved fluid without removing the sieving module.

5. The method according to any one of claims 1 to 4, wherein the automated aggregate removal step is performed and wherein the used sieving module has one or more of the following characteristics:
(i) the sieving module comprises a passage below the sieve that allows to laterally direct the fluid comprising a plurality of biological compartments towards the lateral outlet;
(ii) the sieving module comprises a receptacle comprising two openings and a sieve, wherein the sieve is provided between the two openings, wherein the openings are configured such that the fluid comprising the plurality of biological compartments in singularized form can pass the openings;
(iii) wherein
(aa) per variant A the sieving module comprises a receptacle, preferably a hollow, elongated body, which comprises the sieve and comprises means for laterally directing the plurality of biological compartments after sieving to the lateral outlet, and wherein the lateral outlet is provided by the receptacle; or
(bb) per variant B the sieving module comprises a receptacle and a holder for holding the receptacle, the holder being configured to laterally direct a fluid passing the held receptacle, and wherein the lateral outlet is provided by the holder;
(iv) the sieving module is a consumable which comprises a recess or pedestal for arranging the sieving module on a containment, preferably a well of a well plate; and/or
(v) the sieve comprised in the sieving module has one or more of the following characteristics:
(aa) it has a mean mesh size, which is larger than the size of the plurality of biological compartments and smaller than the undesired aggregates, wherein the aggregates are retained by the sieve;
(bb) it has a mean mesh size selected from the range of 10 to 150 µm, 20 to 100 µm, 30 to 80 µm, 30 to 60µm or 30 to 50µm;
(cc) it has a mean mesh size of approximately 40 µm; and/or
(dd) it is made of nylon or PET.

6. The method according to any one of claims 1 to 5, wherein the automated aggregate removal step is performed and wherein the used sieving module comprises a receptacle and a holder for holding the receptacle, the holder being configured to laterally direct a fluid passing the held receptacle, and wherein the holder of the sieving module has one or more of the following characteristics:
(i) the holder comprises a receptable for receiving the receptacle;
(ii) the lateral outlet is provided by the holder, wherein the holder is configured to laterally direct the plurality of biological compartments passing the receptacle outlet to the lateral outlet provided by the holder;
(iii) the holder comprises a passage configured such that the fluid passing the sieve located inside a receptacle is directed by the passage towards a lateral direction, wherein the lateral direction extends to the lateral outlet; and/or
(iv) wherein the holder receives the receptacle comprising the sieve and wherein fluid exiting the receptacle is received by the holder and is laterally directed by a passage configured to laterally direct a fluid, wherein preferably, the passage is tilted with regard to the sieve to simplify the flow of the sieved fluid through the passage to the lateral outlet.

7. The method according to any one of claims 1 to 6, wherein step (d) is repeated a number of times sufficient to generate a unique combination of barcode sequences for the target molecules in a single biological compartment, such as a single cell or single cell nucleus.

8. The method according to one or more of claims claim 1 to 7, wherein the method is for barcoding nucleic acids within a biological compartment and wherein the method comprises generating cDNAs within the biological compartments by reverse transcribing RNAs into cDNA, preferably using a reverse transcription primer comprising a 5' overhang sequence, and wherein preferably, a reverse transcription reaction is performed prior to step (a).

9. The method according to one or more of claims 1 to 8, in particular claim 8, comprising performing an *in situ* reverse transcription reaction using a plurality of biological compartments, preferably cells, in form of a pool, wherein optionally, the 5' overhang of the reverse transcription primer provides an adapter sequence for the barcode label that is used in step (a).

10. The method according to one or more of claims 1 to 9, in particular claim 8, comprising performing an *in situ* reverse transcription reaction, wherein a first barcode label is introduced during reverse transcription,
wherein preferably, the method comprises (i) separating a plurality of biological compartments into at least two aliquots; and (ii) contacting each of the aliquots with a reverse transcription primer comprising a barcode sequence,
- wherein the barcode sequence of the reverse transcription primer is the same for a given aliquot, and
- wherein a different barcode sequence is used for each of the n aliquots;
and wherein preferably, the method comprises after reverse transcription combining the biological compartments of the aliquots thereby providing a plurality of compartments that can be subjected to step (a) of the method.

11. The method according to one or more of claims 1 to 10, further comprising performing a biological compartment counting step, such as a cell counting step, optionally wherein the biological compartment counting step fulfills one or more of the following characteristics:
(i) it is performed in an automated manner;
(ii) it is performed prior to step (a);
(iii) it is performed after performing fixing and/or permeabilizing the biological compartments and prior to performing step (a);
(iv) it is performed prior to performing a reverse transcription reaction;
(v) it comprises the use of at least one counting chamber, optionally a hemocytometer, wherein the at least one counting chamber is optionally held by a holding device;
(vi) it comprises subjecting a portion of the plurality of biological compartments to counting, wherein the portion is transferred into one or more counting chambers, optionally wherein the transfer occurs in an automated manner using a pipetting module;
(vii) it involves the use of a counting module comprising a holding device configured to hold one or more counting chambers and a sliding device configured to axially direct the holding device, wherein the holding device is engaged with the sliding device configured to axially direct the holding device; and/or
(viii) counting involves staining the biological compartments to be counted and/or using an imaging device for counting.

12. The method according to claim 11, wherein the counting step comprises transferring the biological compartments to be counted in an automated manner into one or more counting chambers provided at a filling position of an automated system by pipetting through a filling opening of the counting chamber, wherein the one or more counting chambers are held in position on the automated system by a holding device and wherein after filling, the one or more counting chambers are transferred to a counting position for counting the biological compartments, wherein transfer to the counting position occurs by axially moving the holding device and thereby the held one or more counting chambers.

13. The method according to claim 11, wherein the counting step comprises the following consecutive steps
(aa) filling one or more counting chambers with biological compartments of the plurality of biological compartments, wherein the one or more counting chambers are mounted to a holding device,
optionally wherein (aa) comprises transferring the biological compartments in an automated manner into the one or more counting chambers provided at a filling position of an automated system by pipetting through a filling opening of a counting chamber using a pipetting module, wherein the one or more counting chambers are held in position on the automated system by the holding device;
(bb) moving the holding device axially;
(cc) imaging biological compartments present in the one or more counting chambers using an imaging device; and
(dd) optionally automatically counting the biological compartments and calculating the number of the plurality of biological compartments,
optionally wherein in step (bb) a pipetting module engages with the holding device, followed by axial movement from the filling position of step (aa) to a position for imaging in step (cc).

14. The method according to claim 12 or 13, wherein a pipetting module is used for filling and for axially moving the holding device, wherein preferably the holding device comprises engagement means with which a part of the pipetting module, optionally a pipette tip, is capable of engaging.

15. The method according to any one of claims 11 to 14, having one or more of the following characteristics:
(i) wherein the holding device for holding the one or more counting chambers is engaged with a sliding device which provides guidance for the axial movement and wherein the holding device is axially movable in relation to the sliding device;
(ii) wherein at the counting or imaging position, the counting chambers are in a position that is accessible for the optical path of an imaging device; and/or
(iii) wherein a pipetting module is used for axially moving the holding device comprising the one or more held counting chambers from a filling position to a counting/imaging position, and wherein for engaging with the holding device, the pipetting module performs a vertical movement towards engaging means provided by the holding device and wherein after axially moving the holding device from the filing position to the counting/imaging position, the pipetting module performs an axial movement in the opposite direction to bring the holding device back into the initial position.

16. The method according to one or more of claims 1 to 15, in particular claims 11 to 15, comprising adjusting the concentration of biological compartments prior to step (a), optionally wherein adjusting the concentration fulfills one or more of the following characteristics:
(i) adjustment is performed to lower the concentration of biological compartments by dilution so that the concentration of biological compartments is within an acceptable range, wherein the acceptable range is preferably predetermined;
(ii) adjustment occurs after performing a cell counting step as defined in any one of claims 11 to 15;
(iii) adjustment of the concentration is performed in an automated manner;
(iv) adjustment of the concentration is performed in a dynamic manner by an automated system using the results of the cell counting step as defined in any one of claims 11 to 15, wherein preferably, the automated system compares the concentration determined as a result of a cell counting step with a predetermined reference value or reference range, wherein if the determined concentration is higher than the reference value of reference range, the automated system adds the required volume of a dilution solution to the plurality of biological compartments to achieve that the plurality of biological compartments are provided in a concentration that is within the predetermined reference value or reference range;
(v) wherein adjusting comprises adding a dilution solution to the plurality of biological compartments, wherein the dilution solution is preferably an aqueous solution, wherein the dilution solution optionally corresponds to the liquid surrounding the plurality of biological compartments; and/or
(vi) wherein the plurality of cells are concentrated using a filter module as defined in any one of claims 1 (i), 2, 3 or 24.

17. The method according to claim 16, said method comprising:
(a) separating a plurality of biological compartments into a number (n) of aliquots, wherein n is at least 2;
(b) contacting barcode labels comprising a barcode sequence with each of the n aliquots,
- wherein the barcode sequence of the barcode labels contacted with a given aliquot is the same, and
- wherein a different barcode sequence is used for different aliquots, preferably for each of the n different aliquots;
(c) combining the n aliquots into a pool; and
(d) repeating steps (a), (b) and (c) using the combined pool,
wherein the number (n) of aliquots provided in step (a) are chosen using the results of the cell counting step performed as defined in any one of claims 11 to 15,
optionally wherein
the number (n) of aliquots are determined by an automated system using the results of the cell counting step, wherein preferably, the automated system compares the concentration determined as a result of a cell counting step with a predetermined reference value or reference range, wherein if the determined concentration is lower than the reference value or reference range, the automated system separates the plurality of biological compartments into fewer aliquots compared to as when the determined concentration meets the reference value of reference range.

18. The method according to one or more of claims 1 to 17, having one or more of the following characteristics:
(i) it further comprises performing a ligation reaction, preferably for ligating at least two of the barcode labels that are bound to the target molecules, wherein preferably the ligation is performed within the plurality of cells;
(ii) it further comprises fixing and/or permeabilizing the plurality of biological compartments prior to step (a) and preferably prior to reverse transcription, if a reverse transcription is performed;
(iii) it comprises performing one or more mixing steps, wherein mixing comprises using an automated mixing mechanism, wherein the automated mixing mechanism comprises the following substeps:
(i) a mixing element, preferably comprising a pipette tip, is contacted with the sample to be mixed, such as with the plurality of biological compartments which are preferably provided in form of a suspension, and performs an ellipsoidal movement; and
(ii) a portion of the sample is transferred from position A to position B within the same containment using the mixing element;
wherein substeps (i) and (ii) can be performed in any order and wherein preferably, substeps (i) and (ii) are repeated one or more times and wherein preferably, position A and B are chosen differently during repetition;
(iv) the biological compartments are selected from cells or cell nuclei;
(v) the target molecules are selected from at least one of RNA, cDNA, DNA, protein, peptide, and antigen, preferably selected from nucleic acids, more preferably selected from RNA or cDNA; and/or
(vi) the method comprises contacting the plurality of biological compartments with at least one adhesion reducing compound, wherein contacting with the adhesion reducing compound preferably fulfills one or more of the following characteristics:
(i) wherein at least one adhesion reducing compound is in contact with the plurality of biological compartments during washing and/or fluid exchange;
(ii) wherein at least one adhesion reducing compound is present when the plurality of biological compartments are present in a solution;
(iii) wherein at least one adhesion reducing compound is included in one or more reaction or processing solutions that are contacted with the biological compartments;
(iv) wherein containments for receiving biological compartments and/or consumables used for transferring biological compartments are contacted, preferably coated, with at least one adhesion reducing compound prior to contact with the plurality of biological compartments; and/or
(v) wherein the at least one adhesion reducing compound has one or more of the following characteristics:
(aa) the adhesion reducing compound is a detergent, preferably a non-ionic detergent;
(bb) the adhesion reducing compound is a detergent selected from the group of polyoxyethylene alkylphenyl ether, polyoxyethylenepolyoxypropylene block copolymers and polyoxyethylene fatty alcohol ether, optionally selected from polyoxamers, such as poloxamer 407 (Pluronic F127) and polyoxyethylene alkylphenyl ethers, such as Triton X100; and/or
(cc) the at least one adhesion reducing compound, which preferably is a non-ionic detergent is provided at a final concentration of at least 5mg/L, at least 1 0mg/L, at least 15mg/L or at least 20 mg/L.

19. An automated system suitable for use in a method according to any one of claims 1 to 18, the system comprising
- a pipetting module;
and
- one or more filter modules for exchanging a liquid surrounding a plurality of biological compartments or for separating a liquid from a plurality of biological compartments, preferably one or more filter modules as defined in claim 3 or 24;
and/or
- one or more sieving modules for removing objects, such as aggregates from a plurality of biological compartments, preferably one or more sieving modules as defined in claim 21 or 22;
the automated system optionally further comprising
- optionally a counting module, preferably a counting module as defined in claim 23;
- optionally a device capable of generating a pressure differential, preferably a syringe pump;
- optionally, an imaging device, preferably a microscope;
- optionally, a cooling module; and
- optionally, a heating module.

20. The automated system according to claim 19, wherein the automated system has one or more of the following characteristics:
(a) the automated system comprises at least one filter module, which comprises
- a feed portion, an effluent portion and a filter element, preferably a membrane, wherein the filter element is provided between the feed portion and the effluent portion;
- wherein the feed portion or the effluent portion is configured to be connected to a device capable of generating a pressure differential; and
- wherein the feed portion is configured such that liquid and/or liquid comprising a plurality of biological compartments can be fed into the feed portion,
and wherein the automated system furthermore has one or more of the following characteristics:
(i) the filter module is connected to a device capable of generating a pressure differential, wherein preferably the effluent portion of the filter module is connected to the device;
(ii) the filter module is connected to a syringe pump, wherein preferably the effluent portion of the filter module is connected to the syringe pump;
(iii) the pressure differential applied to the filter module is controlled in an automated manner; and/or
(iv) the automated system comprises a control unit which is adapted to apply a pressure differential, optionally wherein the control unit has one or more of the following characteristics:
(aa) it is in functional connection with the device capable of generating a pressure differential;
(bb) it is part of the device capable of generating a pressure differential;
(cc) it controls the feed of a liquid, preferably comprising biological compartments, to the filter module;
(dd) it is configured to allow a liquid in the feed portion before and after filtering; and/or
(ee) the control unit and/or the device capable of generating a pressure differential are/is in functional connection with a pressure sensor;
(b) the automated system comprises at least one sieving module as defined in claim 21 or 22 and furthermore has one or more of the following characteristics:
(i) the automated system is configured so that a pipetting module transfers a fluid to the inlet of the sieving module and wherein a pipetting module can retrieve the sieved fluid without removing the sieving module;
(ii) the comprised sieving module directs the sieved fluid to a position that is lateral to the sieving module, wherein preferably the sieved fluid is directed to a lateral containment, preferably a well of a well-plate that is located adjacent to a well that comprises the sieving module; and/or
(iii) the automated system comprises two or more sieving modules as defined in any one of claims 21 or 22;
(c) the automated system comprises a counting module comprising a holding device configured to hold one or more counting chambers and a sliding device configured to axially direct the holding device, wherein the holding device is engaged with the sliding device, and wherein the system has one or more of the following characteristics:
(i) it comprises an imaging device, preferably a microscope;
(ii) the holding device of the counting module comprises means configured for engaging with a part of a pipetting module, such as preferably an attached pipetting tip;
(iii) the automated system is configured so that a pipetting module can engage with the holding device of the counting module and wherein the holding device is moved axially by a pipetting module upon engagement; and/or
(iv) it comprises two or more counting modules as defined in claim 23;
(d) wherein the system comprises a heating module, such as a cycling module, wherein the heating module comprises
- a temperature controlled platform configured for holding containments, wherein the containments are preferably provided as wells of a well plate; and
- a sealing cover configured so that it can be applied onto the containments for sealing the containments;
wherein the sealing cover is a flexible mat, preferably configured to seal the containments when contacting the containments, and wherein the system is configured to apply the sealing cover onto the containments by axially and preferably by vertically moving the sealing cover, preferably by using a pipetting module;
wherein optionally, the automated system comprising the heating module has one or more of the following characteristics:
(i) the heating module is controlled in an automated manner, wherein in particular the temperature and the sealing of the containments by a sealing cover is controlled in an automated manner;
(ii) the heating module is automatically lockable/closable; and/or
(iii) the heating module further comprises components to perform axial and optionally vertical movements, wherein the components may have one or more of the following features:
(aa) one component corresponds to a device for performing an axial movement, preferably comprising a holding device and a sliding device, preferably wherein the axial movement is performed using the pipetting module which optionally comprises a pipetting tip; and/or
(bb) one component corresponds to a device for performing a vertical movement, preferably wherein a device for performing a vertical movement comprises a mechanical or electromechanical actuation, preferably a lifting magnet;
(e) the automated system is controlled by an algorithm for automating a method; and/or
(f) the comprised modules have an interface with the processing unit, wherein the interface is preferably an electronic interface.

21. A sieving module for removing aggregates from a plurality of biological compartments in a method according to any one of claims 1 to 18, the sieving module comprising:
- an inlet and a lateral outlet of a fluid path,
- and a sieve, wherein the sieve is provided in the fluid path;
- wherein the inlet and the lateral outlet are configured such that a fluid can pass the inlet and the lateral outlet; and
- wherein the lateral outlet is directed such that the fluid which passed the sieve is laterally directed with regard to the inlet.

22. The sieving module according to claim 21, wherein the sieving module is a sieving module as defined in claim 5 or 6.

23. A counting module for use in a method as defined in any one of claims 11 to 18, comprising
- a holding device configured to hold one or more counting chambers; and
- a sliding device configured to axially direct the holding device,
wherein the holding device is engaged with the sliding device configured to axially direct the holding device, and
wherein the holding device comprises means configured for engaging with a part of a pipetting module, preferably an attached pipetting tip, and
wherein the holding device is capable of being moved axially by a pipetting module upon engagement.

24. A filter module for use in a method according to any one of claims 1 to 18, the filter module comprising:
- a feed portion, an effluent portion and a filter element, wherein the filter element is provided between the feed portion and the effluent portion;
- wherein the feed portion or the effluent portion is configured to be connected to a device capable of generating a pressure differential;
- wherein the feed portion is configured such that liquid can be fed into the feed portion; and
wherein the filter module comprises an elongated body comprising a feed portion provided as top part, and an effluent portion provided as bottom part, configured to be connected by a frictional fit, form fit and/or adhesive bonding and wherein the filter element is secured between the connected feed portion and the effluent portion, optionally wherein securing includes one or more sealing means or filter element holders; and
wherein the filter element is a surface filter provided by a membrane, the membrane preferably comprising or consisting of a film or foil having pores or holes, more preferably the membrane being a track etched membrane.
